# EUROPEAN PATENT SPECIFICATION

(11) **EP 1 915 344 B1**
(45) Date of publication and mention of the grant of the patent: **01.07.2009**
(21) Application number: 06778054.4
(22) Date of filing: 28.07.2006
(51) Int. Cl.: C07D 213/78, A01N 43/40

(54) **CYANOPYRIDINE PESTICIDES**
CYANOPYRIDINPESTIZIDE
PESTICIDES DE TYPE CYANOPYRIDINE

(30) Priority: 29.07.2005 US 703753 P
(43) Date of publication of application: 30.04.2008
(73) Proprietor: BASF SE, 67056 Ludwigshafen (DE)
(72) Inventor: KAISER, Florian, 68167, Mannheim (DE); VON DEYN, Wolfgang, 67435 Neustadt (DE); POHLMAN, Matthias, 69115 Heidelberg (DE); BASTIAANS, Henricus, Maria, Martinus, 61250 Usingen (DE); ANSPAUGH, Douglas, D., Apex, NC 27502 (US); CULBERTSON, Deborah, L., Fuquay Varina, NC 27526 (US); COTTER, Henry, Van Tuyl, Raleigh, NC 27613 (US)
(74) Representative: Reitstötter - Kinzebach
(86) International application number: PCT/EP2006/064788
(87) International publication number: WO 2007/014913

(56) References cited:
- EP-A- 0 033 984
- WO-A-03/043990
- WO-A-20/05035486
- BORISOV, A. V. ET AL: "Cycloaddition of 3-cyano-4,6-dimethyl-2-pyridinesulfenyl chloride to norbornene" CHEMISTRY OF HETEROCYCLIC COMPOUNDS (NEW YORK, NY, UNITED STATES)(TRANSLATION OF KHIMIYA GETEROTSIKLICHESKIKH SOEDINENII) , 38(9), 1146-1147 CODEN: CHCCAL; ISSN: 0009-3122, 2002, XP002399533
- GEWALD, K. ET AL: "3-Aminoisothiazolo[5,4-b]pyridines" JOURNAL FUER PRAKTISCHE CHEMIE (LEIPZIG) , 317(6), 959-64 CODEN: JPCEAO; ISSN: 0021-8383, 1975, XP002399534
- AHMED, GAMAL A.: "Some reactions of 3-cyano-4,6-dimethyl-2(1H)-pyridinethione. Part 2" JOURNAL OF THE INDIAN CHEMICAL SOCIETY , 73(2-3), 141-143 CODEN: JICSAH; ISSN: 0019-4522, 1996, XP009072532

## Description

The present invention relates to cyanopyridine compounds and to the agriculturally useful salts thereof and to compositions comprising such compounds. The invention also relates to the use of the cyanopyridine compounds, of their salts or of compositions comprising them for combating animal pests.

Animal pests destroy growing and harvested crops and attack wooden dwelling and commercial structures, causing large economic loss to the food supply and to property. While a large number of pesticidal agents are known, due to the ability of target pests to develop resistance to said agents, there is an ongoing need for new agents for combating animal pests. In particular, animal pests such as insects and acaridae are difficult to be effectively controlled.

K. Gewald et al. Journal f. prakt. Chemie, vol. 317 (6), pages 959-64, 1975, describe 3-cyano-6-methylpyridine-2-sulfenylamine compounds which carry hydrogen, or a methyl or ethoxycarbonyl group in the 4-positon. These compounds are reactive intermediates in the preparation of 3-aminoisothiazolo[5,4-b]pyridines.

Gamal A. Ahmed, J. Indian Chem. Soc., 73 (2-3), pages 141-143, 1996, describes benzoisothiozolone derivatives and their antiviral and antibacterial activity. Example 4 describes 3-cyano-4,6-dimethylpyridine-2-sulfeoylamine as reactive intermediate. Example 5 relates to 3-cyano-4,6-dimethylpyridine-2-sulfonamide which is used to study the influence of the sulfonamide function on the antimicrobial activity. Example 7 discloses 3-cyano-4,6-dimethylpyridine-2-sulfonylchloride as the expected oxidation product of the corresponding thione with chlorine

WO 03/043990 describes pesticidal active pyridine-3-sulfonyl compounds which carry an optionally substituted alkyl, alkenyl, alkynyl or phenyl group or nitro group in the 4-positon.

EP 33984 discloses 2-cyanobenzene sulfonamides having aphicidal activity. Their activity, however, is not satisfactory. Similar compounds are described in WO 2005/035486.

It is therefore an object of the present invention to provide compounds having a good pesticidal activity, especially against difficult to control insects and acaridae.

It has been found that these objects are solved by cyanopyridine derivatives of the general formula I: 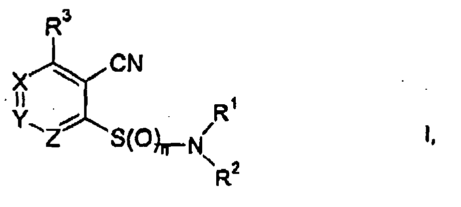 where
- n: is 0, 1 or 2;
- X: is N, N-O or C-R⁴;
- Y: is N, N-O or C-R⁵;
- Z: is N, N-O or C-R⁶;
with the proviso that one of the variables X, Y and Z is N or NO and the other two variables are optionally substituted carbon atoms;
- R¹, R²: are, independently of one another, selected from the group consisting of hydrogen, C(=O)-R⁷, C₁-C₁₀₋alkyl, C₂-C₆-alkenyl, C₂-C₁₀-alkinyl, C₁-C₁₀-alkoxy or C₃-C₁₀-cycloalkyl, wherein the five last-mentioned radicals may be unsubstituted, partially or fully halogenated and/or may carry 1, 2 or 3 radicals, independently of one another each selected from the group consisting of cyano, nitro, amino, C₁-C₁₀-alkoxy, C₁-C₁₀-alkylthio, C₁-C₁₀-alkylsulfinyl, C₁-C₁₀alkylsulfonyl, C₁-C₁₀-haloalkoxy, C₁-C₁₀-haloalkylthio,C₁-C₁₀-alkoxycarbonyl, (C₁-C₁₀-alkyl)amino, di-(C₁-C₁₀-alkyl)amino, C₃-C₁₀-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy;
or
- R¹ and R²: together with the adjacent nitrogen form a 3 to 10-membered ring, optionally substituted by 1, 2 or 3 radicals selected from C₁-C₅-alkyl and halogen, wherein the ring may contain, in addition to the nitrogen and carbon ring members, 1, 2 or 3 heteroatoms as ring members selected from the group consisting of nitrogen, oxygen, sulfur, a group SO, SO₂ or N-R⁸;
- R³: is hydrogen, nitro, cyano, azido, amino, halogen, sulfonylamino, sulfenylamino, sulfinylamino, C(=O)R⁹,
C₁-C₆-alkyl, C₂-C₆-alkenyl, C₂-C₆-alkinyl, C₃-C₈-cycloalkyl, C₁-C₆-alkoxy, C₁-C₆-alkylthio, (C₁-C₆-alkyl)amino, di(C₁-C₆-alkyl)amino, C₁-C₆-alkylsulfinyl, or C₁-C₆-alkylsulfonyl, wherein the ten last-mentioned radicals may be unsubstituted, partially or fully halogenated and/or may carry 1, 2 or 3 radicals, selected from the group consisting of cyano, nitro, amino, C₁-C₄-alkoxy, C₁-C₄-alkylthio, C₁-C₄-alkylsulfinyl, C₁-C₄-alkylsulfonyl, C₁-C₄-haloalkoxy, C₁-C₄-haloalkylthio, (C₁-C₄-alkoxy)carbonyl, (C₁-C₄-alkyl)amino, di(C₁-C₄-alkyl)amino, C₃-C₈-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy;
- R⁴, R⁵ and R⁶: are independently of one another selected from the group consisting of hydrogen, halogen, cyano, azido, nitro, C₁-C₆-alkyl, C₃-C₈-cydoalkyl, C₁-C₄-haloalkyl, C₁-C₄-alkoxy, C₁-C₄-alkylthio, C₁-C₄-alkylsulfinyl, C₁-C₄-alkylsulfonyl, C₁-C₄-haloalkoxy, C₁-C₄-haloalkylthio, C₁-C₆-alkenyl, C₂-C₆-alkinyl, (C₁-C₄-alkoxy)carbonyl, amino, (C₁-C₄-alkyl)amino, di(C₁-C₄-alkyl)amino, aminocarbonyl, (C₁-C₄-alkyl)aminocarbonyl, di(C₁-C₄-alkyl)aminocarbonyl, hydroxysulfonyl, sulfonylamino, sulfenylamino, sulfinylamino and C(=O)-R¹⁰;
- R⁷: is C₁-C₆-alkyl, C₁-haloalkyl, aryl, aryl-C₁-C₄-alkyl,
3- to 7-membered heteroaryl or heteroaryl-C₁-C₄-alkyl, wherein the heteroaryl ring contains as ring members 1, 2 or 3 heteroatoms, selected from the group consisting of nitrogen, oxygen, sulfur, a group SO, SO₂ or N-R¹¹, wherein R¹¹ is hydrogen or C₁-C₄-alkyl;
3- to 7-membered heterocyclyl or heterocyclyl-C₁-C₄-alkyl, wherein the heterocyclic ring contains as ring members 1, 2 or 3 heteroatoms, selected from the group consisting of nitrogen, oxygen, sulfur, a group SO, SO₂ or N-R¹², wherein R¹² is hydrogen or C₁-C₄-alkyl;
and wherein the carbon atoms of the heterocyclic rings may by unsubstituted or substituted by 1 or 2 C₁-C₄-alkyl groups;
- R⁸: is hydrogen or C₁-C₄-alkyl;
- R⁹ and R¹⁰,: independently of one another, are hydrogen, hydroxy, C₁-C₆-alkoxy, amino, C₁-C₄-alkyl, aryl, aryl-C₁-C₄-alkyl,
3- to 7-membered heteroaryl or heteroaryl-C₁-C₄-alkyl, wherein the heteroaryl ring contains as ring members 1, 2 or 3 heteroatoms, selected from the group consisting of nitrogen, oxygen, sulfur, a group SO, SO₂ or N-R¹³, wherein R¹³ is hydrogen or C₁-C₄-alkyl;
3- to 7-membered heterocyclyl or heterocyclyl C₁-C₄-alkyl, wherein the heterocyclic ring contains 1, 2 or 3 heteroatoms, selected from the group consisting of nitrogen, oxygen, sulfur, a group SO, SO₂ or N-R¹⁴, wherein R¹⁴ is hydrogen or C₁-C₄-alkyl;
and wherein the carbon atoms of the heterocyclic rings may by unsubstituted or substituted by 1 or 2 C₁-C₄-alkyl groups;
and/or the agriculturally acceptable salts thereof.

The compounds of the formula I and their agriculturally acceptable salts are highly active against animal pest, i.e. harmful arthropodes and nematodes, especially against difficult to control insects and acaridae.

Accordingly, the present invention relates to cyanopyridine compounds of the general formula I and to their agriculturally useful salts, except for
the compounds of formula I, where n is 0; X is CR⁴, Y is CR⁵; Z is N; R¹, R² and R⁴ are each hydrogen; R⁵ is CH₃; and R³ is hydrogen, CH₃ or C(=O)OC₂H₅; and
the compound of formula I, where n is 2; X is CR⁴; Y is CR⁵; Z is N; R¹, R² and R⁴ are each hydrogen: and R³ and R⁵ each are CH₃.

Moreover, the present invention relates to:
- agricultural compositions comprising such an amount of at least one cyanopyridine derivative of the formula I and/or at least one agriculturally useful salt of I and at least one inert liquid and/or solid agronomically acceptable carrier that it has a pesticidal action and, if desired, at least one surfactant;
- the use of compounds I and/or their salts for combating animal pests; and
- a method of combating animal pests which comprises contacting the animal pests, their habit, breeding ground, food supply, plant, seed, soil, area, material or environment in which the animal pests are growing or may grow, or the materials, plants, seeds, soils, surfaces or spaces to be protected from animal attack or infestation with a pesticidally effective amount of at least one compound of the formula I and/or at least one agriculturally acceptable salt thereof, as defined herein wherein a method of treating humans is excluded and to
- a method for protecting crops from attack or infestation by animal pests, which comprises contacting a crop with a pesticidally effective amount of at least one compound of the formula I and/or at least one salt thereof.

In the substituents R¹ to R¹⁴ the compounds of the general formula I may have one or more centers of chirality, in which case they are present as mixtures of enantiomers or diastereomers. The present invention provides both the pure enantiomes or diastereomers or mixtures thereof. The compounds of the general formula I may also exist in the form of different tautomers if R⁴, R⁵ or R⁶ are amino. The invention comprises the single tautomers, if seperable, as well as the tautomer mixtures.

Salts of the compounds of the formula I are preferably agriculturally acceptable salts. They can be formed in a customary method, e.g. by reacting the compound with an acid of the anion In question if the compound of formula I has a basic functionality or by reacting an acidic compound of formula I with a suitable base.

Suitable agriculturally useful salts are especially the salts of those cations or the acid addition salts of those acids whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethylammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formula I (and likewise of the formulae Ia, Ib and Ic) with an acid of the corresponding anion, preferably of hydrochloric acid, hydrobromic acid, sulfuric acid, phosphoric acid or nitric acid.

The organic moieties mentioned in the above definitions of the variables are - like the term halogen - collective terms for individual listings of the individual group members. The prefix Cₙ-Cₘ indicates in each case the possible number of carbon atoms in the group.

"Halogen" will be taken to mean fluoro, chloro, bromo and iodo.

The term "partially or fully halogenated" will be taken to mean that 1 or more, e.g. 1, 2, 3, 4 or 5 or all of the hydrogen atoms of a given radical have been replaced by a halogen atom, in particular by fluorine or chlorine.

The term "Cₙ-Cₘ-alkyl" as used herein (and also in Cₙ-Cₘ-alkylamino, di-Cₙ-Cₘ-alkylamino, Cₙ-Cₘ-alkylaminocarbonyl, di-(Cₙ-Cₘ-alkylamino)carbonyl, Cₙ-Cₘ-alkylthio, Cₙ-Cₘ-alkylsulfinyl and Cₙ-Cₘ-alkylsulfonyl) refers to a branched or unbranched saturated hydrocarbon group having n to m, e.g. 1 to 10 carbon atoms, preferably 1 to 6 carbon atoms, for example methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "Cₙ-Cₘ-haloalkyl" as used herein (and also in Cₙ-Cₘ-haloalkylsulfinyl and Cₙ-Cₘ-haloalkylsulfonyl) refers to a straight-chain or branched alkyl group having n to m carbon atoms, e.g. 1 to 10 in particular 1 to 6 carbon atoms (as mentioned above), where some or all of the hydrogen atoms in these groups may be replaced by halogen atoms as mentioned above, for example C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like. The term C₁-C₁₀-haloalkyl in particular comprises C₁-C₂-fluoroalkyl, which is synonym with methyl or ethyl, wherein 1, 2, 3, 4 or 5 hydrogen atoms are substituted by fluorine atoms, such as fluoromethyl, difluoromethyl, trifluoromethyl, 1-fluoroethyl, 2-fluoroethyl, 2,2-difluoroethyl, 2,2,2-trifluoroethyl and pentafluoromethyl.

Similarly, "Cₙ-Cₘ-alkoxy" and "Cₙ-Cₘ-alkylthio" (or Cₙ-Cₘ-alkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group. Examples include C₁-C₄-alkoxy such as methoxy, ethoxy, propoxy, isopropoxy, butoxy, sec-butoxy, isobutoxy and tert-butoxy, further C₁-C₄-alkylthio such as methylthio, ethylthio, propylthio, isopropylthio, and n-butylthio.

Accordingly, the terms "Cₙ-Cₘ-haloalkoxy" and "Cₙ-Cₘ-haloalkylthio" (or Cₙ-Cₘ-haloalkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group, where some or all of the hydrogen atoms in these groups may be replaced by halogen atoms as mentioned above, for example C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy, further C₁-C₂-haloalkylthio, such as chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like. Similarly the terms C₁-C₂-fluoroalkoxy and C₁-C₂-fluoroalkylthio refer to C₁-C₂-fluoroalkyl which is bound to the remainder of the molecule via an oxygen atom or a sulfur atom, respectively.

The term "C₂-Cₘ-alkenyl" as used herein intends a branched or unbranched unsaturated hydrocarbon group having 2 to m, e.g. 2 to 10 or 2 to 6 carbon atoms and a double bond in any position, such as ethenyl, 1-propenyl, 2-propenyl, 1-methyl-ethenyl, 1-butenyl, 2-butenyl, 3-butenyl, 1-methyl-l-propenyl, 2-methyl-1-propenyl,1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-l-propenyl and 1-ethyl-2-methyl-2-propenyl.

The term "C₂-Cₘ-alkynyl" as used herein refers to a branched or unbranched unsaturated hydrocarbon group having 2 to m, e.g. 2 to 10 or 2 to 6 carbon atoms and containing at least one triple bond, such as ethynyl, propynyl, 1-butynyl, 2-butynyl, and the like.

The term C₁-C₄-alkoxy-C₁-C₄-alkyl as used herein refers to alkyl having 1 to 4 carbon atoms, wherein one hydrogen atom of the alkyl radical is replaced by a C₁-C₄-alkoxy group.

The term C₁-C₄-alkylthio-C₁-C₄-alkyl as used herein refers to alkyl having 1 to 4 carbon atoms, wherein one hydrogen atom of the alkyl radical is replaced by a C₁-C₄-alkylthio group.

The term "C₃-Cₘ-cycloalkyl" as used herein refers to a monocyclic 3- to m-membered saturated cycloaliphatic radicals, e.g. cyclopropyl, cyclobutyl, cyclopentyl, cyclohexyl, cycloheptyl, cyclooctyl and cyclodecyl.

The term aryl as used herein refers to an aromatic hydrocarbon radical such as naphthyl or in particular phenyl.

The term aryl-C₁-C₄-alkyl as used herein refers to an aromatic hydrocarbon radical, which is bound to the remainder of the molecule via a C₁-C₄-alkylene group, examples comprise benzyl, 1-phenylethyl or 2-phenylethyl.

The term "3- to 7-membered heterocyclyl as used herein (and also in heterocyclyl-C₁-C₄-alkyl) refers to a saturated or partially unsaturated non-aromatic heterocyclic radical havin 3 to 7 ring members, wherein 1, 2 or 3 ring members are heteroatoms selected from O, N and S or heteroatom groups, selected from S=O, S(O)₂ or N-R with R being H or alkyl. Examples for non-aromatic rings include azetidiyl, pyrrolidinyl, pyrazolinyl, imidazolinyl, pyrrolinyl, pyrazolinyl, imidazolinyl, tetrahydrofuranyl, dihydrofuranyl, 1,3-dioxolanyl, dioxolenyl, thiolanyl, dihydrothienyl, oxazolidinyl, isoxazolidinyl, oxazolinyl, isoxazolinyl, thiazolinyl, isothiazolinyl, thiazolidinyl, isothiazolidinyl, oxathiolanyl, piperidinyl, piperazinyl, pyranyl, dihydropyranyl, tetrahydropyranyl, dioxanyl, thiopyranyl, dihydrothiopyranyl, tetrahydrothiopyranyl, morpholinyl, thiazinyl and the like.

The term "3- to 7-membered heteroaryl as used herein (and also in heteroaryl-C₁-C₄-alkyl) refers to an aromatic heterocyclic radical havin 3 to 7 ring members, wherein 1, 2 or 3 ring members are heteroatoms selected from O, N and S or heteroatom groups, selected from S=O, S(O)₂ or N-R with R being H or alkyl. Examples for monocyclic 3-to 7-membered heteroaromatic rings include triazinyl, pyrazinyl, pyrimidyl, pyridazinyl, pyridyl, thienyl, furyl, pyrrolyl, pyrazolyl, imidazolyl, triazolyl, tetrazolyl, thiazolyl, oxazolyl, thiadiazolyl, oxadiazolyl, isothiazolyl and isoxazolyl.

The terms heterocyclyl-C₁-C₄-alkyl and heteroaryl-C₁-C₄-alkyl as used herein refer to an aromatic or non-aromatic heterocyclic radical, which is bound to the remainder of the molecule via a C₁-C₄-alkylene group.

With respect to the use according to the invention of the compounds of formula I, particular preference is given to the following meanings of the substituents and variables, in each case on their own or in combination:
R¹ is preferably selected from the group consisting of hydrogen, C₁-C₆-alkyl, C₁-C₆-haloalkyl, C₂-C₆-alkenyl, C₂-C₆-haloalkenyl, C₂-C₆-alkinyl, C₂-C₆-haloalkinyl, C₃-C₆-cycloalkyl, C₁-C₄-alkoxy-C₁-C₄-alkyl and C₁-C₄-alkylthio-C₁-C₄-alkyl. In particular R¹ is H or a radical having 1 to 6 and more preferably 1 to 4 carbon atoms. More preferably R¹ is selected from the group consisting of hydrogen, C₁-C₄-alkyl, C₁-C₄-haloalkyl, C₂-C₄-alkinyl, cyclopropyl, C₁-C₃-alkoxy-C₁-C₃-alkyl, in particular 2-methoxyethyl, and C₁-C₃-alkylthio-C₁-C₃-alkyl, in particular 2-methylthioethyl. More particularly preferably R¹ is selected from hydrogen, C₁-C₄-alkyl, in particular methyl, ethyl, or 1-methylethyl, and C₂-C₄-alkinyl, in particular prop-3-yn-1-yl.
R² is preferably selected from the group consisting of hydrogen, C₁-C₆-alkyl, C₁-C₆-haloalkyl, C₂-C₆-alkenyl, C₂-C₆-haloalkenyl, C₂-C₆-alkinyl, C₂-C₆-haloalkinyl, C₃-C₆-cycloalkyl, C₁-C₄-alkoxy-C₁-C₄-alkyl and C₁-C₄-alkylthio-C₁-C₄-alkyl. However R² may also form a radical C(=O)-R⁷, wherein R⁷ is as defined herein, in particular R⁷ being C₁-C₄-alkyl or C₁-haloalkyl. More preferably R² is selected from the group consisting of hydrogen, C₁-C₄-alkyl, in particular methyl, ethyl, or 1-methylethyl, C₂-C₄-alkinyl, in particular prop-3-yn-1-yl, and C(=O)-R^{7a}, wherein R^{7a} is selected from C₁-C₄-alkyl, in particular methyl ethyl, 1-methylethyl or 2-methylpropan-2-yl, and C₁-haloalkyl, in particular trifluoromethyl or trichloromethyl.
R³ is preferably selected from halogen, C₁-C₄-alkyl, C₁-C₄-haloalkyl, C₁-C₄-alkoxy or C₁-C₄-haloalkoxy. More preferably R³ is selected from C₁-C₂-alkyl, C₁-haloalkyl, C₁-C₂-alkoxy or C₁-haloalkoxy. More particularly preferably R³ is selected from, methyl, trifluoromethyl, trichloromethyl, methoxy, trifluoromethoxy, difluoromethoxy, fluoromethoxy or chlorodifluoromethoxy. In another embodiment of the invention R³ is halogen in particular chlorine.
R⁴, R⁵, and R⁶ are preferably selected, independently from each other, from H, C₁-C₄-alkyl, C₁-C₄-haloalkyl, C₁-C₄-alkoxy, C₁-C₄-haloalkoxy or halogen. More preferably R⁴, R⁵, and R⁶ are selected, independently from each other, from hydrogen, halogen, C₁-C₄-alkyl, or C₁-C₄-haloalkyl, especially from hydrogen, halogen, C₁-C₂-alkyl, or C₁-haloalkyl, most preferably, from H, Cl, Br, I**,** CH₃ or CF₃. In a preferred embodiment of the invention one of the radicals R⁴, R⁵ or R⁶ being present, is different from hydrogen, while the other one being present is hydrogen, or both of the radicals R⁴, R⁵ or R⁶ being present are hydrogen.

In formula I the variable n is preferably 2. These compounds are also referred to as compounds la:

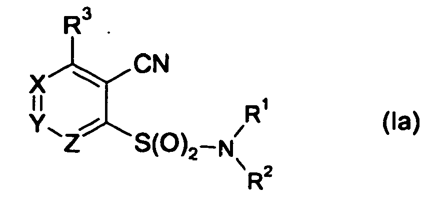

Compounds I, where n is 0 are also referred to as compounds Ib, while compounds I, where n is 1 are also referred to as compounds Ic,

A preferred embodiment of the invention relates to 4-cyanopyridine compounds of the formula I, i.e. compounds of the formula I wherein X is C-R⁴, Y is N or NO and Z is C-R⁶. In this embodiment, preference is given to those compounds, wherein X is C-R⁴, Y is N, Z is C-R⁶ and n is 2. In this embodiment preference is given to compounds of the formula I, wherein either R⁴ or R⁶ is hydrogen and the other radical R⁴ or R⁶ is selected from halogen, C₁-C₄-alkyl, or C₁-C₄-haloalkyl, in particular form halogen, C₁-C₂-alkyl, or C₁-haloalkyl, most preferably, from Cl, Br, I, CH₃ or CF₃. Likewise preferred are compounds of this embodiment, wherein both R⁴ and R⁶ are hydrogen.

Another embodiment of the invention relates to 3-cyanopyridine compounds of the formula I, i.e. compounds of the formula I wherein X is C-R⁴, Y is C-R⁵ and Z is N or NO. In this embodiment, preference is given to those compounds, wherein X is C-R⁴, Y is C-R⁵ and Z is N and n is 2. In this embodiment preference is given to compounds of the formula I, wherein either R⁴ or R⁵ is hydrogen and the other radical R⁴ or R⁵ is selected from halogen, C₁-C₄-alkyl, or C₁-C₄-haloalkyl, in particular form halogen, C₁-C₂-alkyl, or C₁-haloalkyl, most preferably, from Cl, Br, I, CH₃ or CF₃. Likewise preferred are compounds of this embodiment, wherein both R⁴ and R⁵ are hydrogen.

Examples of preferred compounds of the formula la are given in the following tables A1 to A 11268.

| | |
|---|---|
| Tabelle A1: | Compounds of the formulae Ia, Ib and Ic, wherein each of X and Z are CH, Y is N, R³ is CH₃ and R¹ and R² are as defined in one row of table A; |
| Tabelle A2: | Compounds of the formulae Ia, Ib and Ic, wherein each of X and Z are CH, Y is N, R³ is C₂H₅ and R¹ and R² are as defined in one row of table A; |
| Tabelle A3: | Compounds of the formulae Ia, Ib and Ic, wherein each of X and Z are CH, Y is N, R³ is CF₃ and R¹ and R² are as defined in one row of table A; |
| Tabelle A4: | Compounds of the formulae Ia, Ib and Ic, wherein each of X and Z are CH, Y is N, R³ is CCl₃ and R¹ and R² are as defined in one row of table A; |
| Tabelle A5: | Compounds of the formulae Ia, Ib and Ic, wherein each of X and Z are CH, Y is N, R³ is F and R¹ and R² are as defined in one row of table A; |
| Tabelle A6: | Compounds of the formulae Ia, Ib and Ic, wherein each of X and Z are CH, Y is N, R³ is Cl and R¹ and R² are as defined in one row of table A; |
| Tabelle A7: | Compounds of the formulae Ia, Ib and Ic, wherein each of X and Z are CH, Y is N, R³ is Br and R¹ and R² are as defined in one row of table A; |
| Tabelle A8: | Compounds of the formulae Ia, Ib and Ic, wherein each of X and Z are CH, Y is N, R³ is I and R¹ and R² are as defined in one row of table A; |
| Tabelle A9: | Compounds of the formulae Ia, Ib and Ic, wherein each of X and Z are CH, Y is N, R³ is OCH₃ and R¹ and R² are as defined in one row of table A; |
| Tabelle A10: | Compounds of the formulae Ia, Ib and Ic, wherein each of X and Z are CH, Y is N, R³ is OCF₃ and R¹ and R² are as defined in one row of table A; |
| Tabelle A11: | Compounds of the formulae Ia, Ib and Ic, wherein each of X and Z are CH, Y is N, R³ is OCCl₃ and R¹ and R² are as defined in one row of table A; |
| Tabelle A12: | Compounds of the formulae Ia, Ib and Ic, wherein each of X and Z are CH, Y is N, R³ is OCHF₂ and R¹ and R² are as defined in one row of table A; |
| Tabelle A13: | Compounds of the formulae Ia, Ib and Ic, wherein each of X and Z are CH, Y is N, R³ is OCClF₂ and R¹ and R² are as defined in one row of table A; |
| Tabelle A14: | Compounds of the formulae Ia, Ib and Ic, wherein each of X and Z are CH, Y is N, R³ is OCH₂F and R¹ and R² are as defined in one row of table A; |
| Tabelle A15: | Compounds of the formulae Ia, Ib and Ic, wherein each of X and Z are CH, Y is N, R³ is OCHCl₂ and R¹ and R² are as defined in one row of table A; |
| Tabelle A16: | Compounds of the formulae Ia, Ib and Ic, wherein each of X and Z are CH, Y is N, R³ is OCH₂Cl and R¹ and R² are as defined in one row of table A; |
| Tabelle A17: | Compounds of the formulae Ia, Ib and Ic, wherein each of X and Z are CH, Y is N, R³ is OCH₂-OCH₃ and R¹ and R² are as defined in one row of table A; |
| Tabelle A18: | Compounds of the formulae Ia, Ib and Ic, wherein each of X and Z are CH, Y is N, R³ is OCH₂CH₃ and R¹ and R² are as defined in one row of table A; |
| Tabelle A19: | Compounds of the formulae Ia, Ib and Ic, wherein X is CH, Y is N, Z is C-Cl, R³ is CH₃ and R¹ and R² are as defined in one row of table A; |
| Tabelle A20: | Compounds of the formulae la, Ib and Ic, wherein X is CH, Y is N, Z is C-Cl, R³ is C₂H₅ and R¹ and R² are as defined in one row of table A; |
| Tabelle A21: | Compounds of the formulae Ia, Ib and Ic, wherein X is CH, Y is N, Z is C-Cl, R³ is CF₃ and R¹ and R² are as defined in one row of table A; |
| Tabelle A22: | Compounds of the formulae Ia, Ib and Ic, wherein X is CH, Y is N, Z is . C-Cl, R³ is CCl₃ and R¹ and R² are as defined in one row of table A; |
| Tabelle A23: | Compounds of the formulae Ia, Ib and Ic, wherein X is CH, Y is N, Z is C-Cl, R³ is F and R¹ and R² are as defined in one row of table A; |
| Tabelle A24: | Compounds of the formulae Ia, Ib and Ic, wherein X is CH, Y is N, Z is C-Cl, R³ is Cl and R¹ and R² are as defined in one row of table A; |
| Tabelle A25: | Compounds of the formulae Ia, Ib and Ic, wherein X is CH, Y is N, Z is C-Cl, R³ is Br and R¹ and R² are as defined in one row of table A; |
| Tabelle A26: | Compounds of the formulae Ia, Ib and Ic, wherein X is CH, Y is N, Z is C-Cl, R³ is I and R¹ and R² are as defined in one row of table A; |
| Tabelle A27: | Compounds of the formulae Ia, Ib and Ic, wherein X is CH, Y is N, Z is C-Cl. R³ is OCH₃ and R¹ and R² are as defined in one row of table A; |
| Tabelle A28: | Compounds of the formulae Ia, Ib and Ic, wherein X is CH, Y is N, Z is C-Cl, R³ is OCF₃ and R¹ and R² are as defined in one row of table A; |
| Tabelle A29: | Compounds of the formulae Ia, Ib and Ic, wherein X is CH, Y is N, Z is C-Cl, R³ is OCCl₃ and R¹ and R² are as defined in one row of table A; |
| Tabelle A30: | Compounds of the formulae Ia, Ib and Ic, wherein X is CH, Y is N, Z is C-Cl, R³ is OCHF₂ and R¹ and R² are as defined in one row of table A; |
| Tabelle A31: | Compounds of the formulae Ia, Ib and Ic, wherein X is CH, Y is N, Z is C-Cl, R³ is OCClF₂ and R¹ and R² are as defined in one row of table A; |
| Tabelle A32: | Compounds of the formulae Ia, Ib and Ic, wherein X is CH, Y is N, Z is C-Cl, R³ is OCH₂F and R¹ and R² are as defined in one row of table A; |
| Tabelle A33: | Compounds of the formulae Ia, Ib and Ic, wherein X is CH, Y is N, Z is C-Cl, R³ is OCHCl₂ and R¹ and R² are as defined in one row of table A; |
| Tabelle A34: | Compounds of the formulae Ia, Ib and Ic, wherein X is CH, Y is N, Z is C-Cl, R³ is OCH₂Cl and R¹ and R² are as defined in one row of table A; |
| Tabelle A35: | Compounds of the formulae Ia, Ib and Ic, wherein X is CH, Y is N, Z is C-Cl, R³ is OCH₂-OCH₃ and R¹ and R² are as defined in one row of table A; |
| Tabelle A36: | Compounds of the formulae Ia, Ib and Ic, wherein X is CH, Y is N, Z is C-Cl, R³ is OCH₂CH₃ and R¹ and R² are as defined in one row of table A; |
| Tabelle A37: | compounds of the formulae Ia, Ib and Ic, wherein X is CH, Y is N, Z is C-F, R³ is CH₃ and R¹ and R² are as defined in one row of table A; |
| Tabelle A38: | Compounds of the formulae Ia, Ib and Ic, wherein X is CH, Y is N, Z is C-F, R³ is C₂H₅ and R¹ and R² are as defined in one row of table A; |
| Tabelle A39: | Compounds of the formulae Ia, Ib and Ic, wherein X is CH, Y is N, Z is C-F, R³ is CF₃ and R¹ and R² are as defined in one row of table A; |
| Tabelle A40: | Compounds of the formulae Ia, Ib and Ic, wherein X is CH, Y is N, Z is C-F, R³ is CCl₃ and R¹ and R² are as defined in one row of table A; |
| Tabelle A41: | Compounds of the formulae Ia, Ib and Ic, wherein X is CH, Y is N, Z is C-F, R³ is F and R¹ and R² are as defined in one row of table A; |
| Tabelle A42: | Compounds of the formulae Ia, Ib and Ic, wherein X is CH, Y is N, Z is C-F, R³ is Cl and R¹ and R² are as defined in one row of table A; |
| Tabelle A43: | Compounds of the formulae Ia, Ib and Ic, wherein X is CH, Y is N, Z is C-F, R³ is Br and R¹ and R² are as defined in one row of table A; |
| Tabelle A44: | Compounds of the formulae Ia, Ib and Ic, wherein X is CH, Y is N, Z is C-F, R³ is I and R¹ and R² are as defined in one row of table A; |
| Tabelle A45: | Compounds of the formulae Ia, Ib and Ic, wherein X is CH, Y is N, Z is C-F, R³ is OCH₃ and R¹ and R² are as defined in one row of table A; |
| Tabelle A46: | Compounds of the formulae Ia, Ib and Ic, wherein X is CH, Y is N, Z is C-F, R³ is OCF₃ and R¹ and R² are as defined in one row of table A; |
| Tabelle A47: | Compounds of the formulae Ia, Ib and Ic, wherein X is CH, Y is N, Z is C-F, R³ is OCCl₃ and R¹ and R² are as defined in one row of table A; |
| Tabelle A48: | Compounds of the formulae Ia, Ib and Ic, wherein X is CH, Y is N, Z is . C-F, R³ is OCHF₂ and R¹ and R² are as defined in one row of table A; |
| Tabelle A49: | Compounds of the formulae Ia, Ib and Ic, wherein X is CH, Y is N, Z is C-F, R³ is OCClF₂ and R¹ and R² are as defined in one row of table A; |
| Tabelle A50: | Compounds of the formulae Ia, Ib and Ic, wherein X is CH, Y is N; Z is C-F, R³ is OCH₂F and R¹ and R² are as defined in one row of table A; |
| Tabelle A51: | Compounds of the formulae Ia, Ib and Ic, wherein X is CH, Y is N, Z is C-F, R³ is OCHCl₂ and R¹ and R² are as defined in one row of table A; |
| Tabelle A52: | Compounds of the formulae Ia, Ib and Ic, wherein X is CH, Y is N, Z is C-F, R³ is OCH₂Cl and R¹ and R² are as defined in one row of table A; |
| Tabelle A53: | Compounds of the formulae Ia, Ib and Ic, wherein X is CH, Y is N, Z is C-F, R³ is OCH₂-OCH₃ and R¹ and R² are as defined in one row of table A; |
| Tabelle A54: | Compounds of the formulae Ia, Ib and Ic, wherein X is CH, Y is N, Z is C-F, R³ is OCH₂CH₃ and R¹ and R² are as defined in one row of table A; |
| Tabelle A55: | Compounds of the formulae Ia, Ib and Ic, wherein X is CH, Y is N, Z is C-Br, R³ is CH₃ and R¹ and R² are as defined in one row of table A; |
| Tabelle A56: | Compounds of the formulae Ia, Ib and Ic, wherein X is CH, Y is N, Z is C-Br, R³ is C₂H₅ and R¹ and R² are as defined in one row of table A; |
| Tabelle A57: | Compounds of the formulae la, Ib and Ic, wherein X is CH, Y is N, Z is C-Br, R³ is CF₃ and R¹ and R² are as defined in one row of table A; |
| Tabelle A58: | Compounds of the formulae Ia, Ib and Ic, wherein X is CH, Y is N, Z is C-Br, R³ is CCl₃ and R¹ and R² are as defined in one row of table A; |
| Tabelle A59: | Compounds of the formulae Ia, Ib and Ic, wherein X is CH, Y is N, Z is C-Br, R³ is F and R¹ and R² are as defined in one row of table A; |
| Tabelle A60: | Compounds of the formulae Ia, Ib and Ic, wherein X is CH, Y is N, Z is C-Br, R³ is Cl and R¹ and R² are as defined in one row of table A; |
| Tabelle A61: | Compounds of the formulae Ia, Ib and Ic, wherein X is CH, Y is N, Z is C-Br, R³ is Br and R¹ and R² are as defined in one row of table A; |
| Tabelle A62: | Compounds of the formulae Ia, Ib and Ic, wherein X is CH, Y is N, Z is C-Br, R³ is I and R¹ and R² are as defined in one row of table A; |
| Tabelle A63: | Compounds of the formulae Ia, Ib and Ic, wherein X is CH, Y is N, Z is C-Br, R³ is OCH₃ and R¹ and R² are as defined in one row of table A; |
| Tabelle A64: | Compounds of the formulae Ia, Ib and Ic, wherein X is CH, Y is N, Z is C-Br, R³ is OCF₃ and R¹ and R² are as defined in one row of table A; |
| Tabelle A65: | Compounds of the formulae Ia, Ib and Ic, wherein X is CH, Y is N, Z is C-Br, R³ is OCCl₃ and R¹ and R² are as defined in one row of table A; |
| Tabelle A66: | Compounds of the formulae Ia, Ib and Ic, wherein X is CH, Y is N, Z is C-Br, R³ is OCHF₂ and R¹ and R² are as defined in one row of table A; |
| Tabelle A67: | Compounds of the formulae Ia, Ib and Ic, wherein X is CH, Y is N, Z is C-Br, R³ is OCClF₂ and R¹ and R² are as defined in one row of table A; |
| Tabelle A68: | Compounds of the formulae Ia, Ib and Ic, wherein X is CH, Y is N, Z is C-Br, R³ is OCH₂F and R¹ and R² are as defined in one row of table A; |
| Tabelle A69: | Compounds of the formulae Ia, Ib and Ic, wherein X is CH, Y is N, Z is C-Br, R³ is OCHCl₂ and R¹ and R² are as defined in one row of table A; |
| Tabelle A70: | Compounds of the formulae Ia, Ib and Ic, wherein X is CH, Y is N, Z is C-Br, R³ is OCH₂Cl and R¹ and R² are as defined in one row of table A; |
| Tabelle A71: | Compounds of the formulae Ia, Ib and Ic, wherein X is CH, Y is N, Z is C-Br, R³ is OCH₂-OCH₃ and R¹ and R² are as defined in one row of table A; |
| Tabelle A72: | Compounds of the formulae Ia, Ib and Ic, wherein X is CH, Y is N, Z is C-Br, R³ is OCH₂CH₃ and R¹ and R² are as defined in one row of table. A; |
| Tabelle A73: | Compounds of the formulae Ia, Ib and Ic, wherein X is CH, Y is N, Z is C-I, R³ is CH₃ and R¹ and R² are as defined in one row of table A; |
| Tabelle A74: | Compounds of the formulae Ia, Ib and Ic, wherein X is CH, Y is N, Z is C-I, R³ is C₂H₅ and R¹ and R² are as defined in one row of table A; |
| Tabelle A75: | Compounds of the formulae Ia, Ib and Ic, wherein X is CH, Y is N, Z is C-I, R³ is CF₃ and R¹ and R² are as defined in one row of table A; |
| Tabelle A76: | Compounds of the formulae Ia, Ib and Ic, wherein X is CH, Y is N, Z is C-I, R³ is CCl₃ and R¹ and R² are as defined in one row of table A; |
| Tabelle A77: | Compounds of the formulae Ia, Ib and Ic, wherein X is CH, Y is N, Z is C-I, R³ is F and R¹ and R² are as defined in one row of table A; |
| Tabelle A78: | Compounds of the formulae Ia, Ib and Ic, wherein X is CH, Y is N, Z is C-I, R³ is Cl and R¹ and R² are as defined in one row of table A; |
| Tabelle A79: | Compounds of the formulae Ia, Ib and Ic, wherein X is CH, Y is N, Z is C-I, R³ is Br and R¹ and R² are as defined in one row of table A; |
| Tabelle A80: | Compounds of the formulae Ia, Ib and Ic, wherein X is CH, Y is N, Z is C-I, R³ is I and R¹ and R² are as defined in one row of table A; |
| Tabelle A81: | Compounds of the formulae Ia, Ib and Ic, wherein X is CH, Y is N, Z is C-I, R³ is OCH₃ and R¹ and R² are as defined in one row of table A; |
| Tabelle A82: | Compounds of the formulae Ia, Ib and Ic, wherein X is CH, Y is N, Z is C-I, R³ is OCF₃ and R¹ and R² are as defined in one row of table A; . |
| Tabelle A83: | Compounds of the formulae Ia, Ib and Ic, wherein X is CH, Y is N, Z is C-I, R³ is OCCl₃ and R¹ and R² are as defined in one row of table A; |
| Tabelle A84: | Compounds of the formulae Ia, Ib and Ic, wherein X is CH, Y is N, Z is C-I, R³ is OCHF₂ and R¹ and R² are as defined in one row of table A; |
| Tabelle A85: | Compounds of the formulae Ia, Ib and Ic, wherein X is CH, Y is N, Z is C-I, R³ is OCClF₂ and R¹ and R² are as defined in one row of table A; |
| Tabelle A86: | Compounds of the formulae Ia, Ib and Ic, wherein X is CH, Y is N, Z is C-I, R³ is OCH₂F and R¹ and R² are as defined in one row of table A; |
| Tabelle A87: | Compounds of the formulae Ia, Ib and Ic, wherein X is CH, Y is N, Z is C-I, R³ is OCHCl₂ and R¹ and R² are as defined in one row of table A; |
| Tabelle A88: | Compounds of the formulae la, Ib and Ic, wherein X is CH, Y is N, Z is C-I, R³ is OCH₂Cl and R¹ and R² are as defined in one row of table A; |
| Tabelle A89: | Compounds of the formulae Ia, Ib and Ic, wherein X is CH, Y is N, Z is C-I, R³ is OCH₂-OCH₃ and R¹ and R² are as defined in one row of table A; |
| Tabelle A90: | Compounds of the formulae Ia, Ib and Ic, wherein X is CH, Y is N, Z is C-I, R³ is OCH₂CH₃ and R¹ and R² are as defined in one row of table A; |
| Tabelle A91: | Compounds of the formulae Ia, Ib and Ic, wherein X is CH, Y is N, Z is C-NH₂, R³ is CH₃ and R¹ and R² are as defined in one row of table A; |
| Tabelle A92: | Compounds of the formulae Ia, Ib and Ic, wherein X is CH, Y is N, Z is C-NH₂, R³ is C₂H₅ and R¹ and R² are as defined in one row of table A; |
| Tabelle A93: | Compounds of the formulae Ia, Ib and Ic, wherein X is CH, Y is N, Z is C-NH₂, R³ is CF₃ and R¹ and R² are as defined in one row of table A; |
| Tabelle A94: | Compounds of the formulae Ia, Ib and Ic, wherein X is CH, Y is N, Z is C-NH₂, R³ is CCl₃ and R¹ and R² are as defined in one row of table A; |
| Tabelle A95: | Compounds of the formulae Ia, Ib and Ic, wherein X is CH, Y is N, Z is C-NH₂, R³ is F and R¹ and R² are as defined in one row of table A; |
| Tabelle A96: | Compounds of the formulae Ia, Ib and Ic, wherein X is CH, Y is N, Z is C-NH₂, R³ is Cl and R¹ and R² are as defined in one row of table A; |
| Tabelle A97: | Compounds of the formulae Ia, Ib and Ic, wherein X is CH, Y is N, Z is . C-NH₂, R³ is Br and R¹ and R² are as defined in one row of table A; |
| Tabelle A98: | Compounds of the formulae Ia, Ib and Ic, wherein X is CH, Y is N, Z is C-NH₂, R³ is I and R¹ and R² are as defined in one row of table A; |
| Tabelle A99: | Compounds of the formulae Ia, Ib and Ic, wherein X is CH, Y is N, Z is C-NH₂, R³ is OCH₃ and R¹ and R² are as defined in one row of table A; |
| Tabelle A100: | Compounds of the formulae Ia, Ib and Ic, wherein X is CH, Y is N, Z is C-NH₂, R³ is OCF₃ and R¹ and R² are as defined in one row of table A; |
| Tabelle A101: | Compounds of the formulae Ia, Ib and Ic, wherein X is CH, Y is N, Z is C-NH₂, R³ is OCCl₃ and R¹ and R² are as defined in one row of table A; |
| Tabelle A102: | Compounds of the formulae Ia, Ib and Ic, wherein X is CH, Y is N, Z is C-NH₂, R³ is OCHF₂ and R¹ and R² are as defined in one row of table A; |
| Tabelle A103: | Compounds of the formulae Ia, Ib and Ic, wherein X is CH, Y is N, Z is C-NH₂, R³ is OCClF₂ and R¹ and R² are as defined in one row of table A; |
| Tabelle A104: | Compounds of the formulae Ia, Ib and Ic, wherein X is CH, Y is N, Z is C-NH₂, R³ is OCH₂F and R¹ and R² are as defined in one row of table A; |
| Tabelle A105: | Compounds of the formulae Ia, Ib and Ic, wherein X is CH, Y is N, Z is C-NH₂, R³ is OCHCl₂ and R¹ and R² are as defined in one row of table A; |
| Tabelle A106: | Compounds of the formulae Ia, Ib and Ic, wherein X is CH, Y is N, Z is C-NH₂, R³ is OCH₂Cl and R¹ and R² are as defined in one row of table A; |
| Tabelle A107: | Compounds of the formulae Ia, Ib and Ic, wherein X is CH, Y is N, Z is C-NH₂, R³ is OCH₂-OCH₃ and R¹ and R² are as defined in one row of table A; |
| Tabelle A108: | Compounds of the formulae la, Ib and Ic, wherein X is CH, Y is N, Z is C-NH₂, R³ is OCH₂CH₃ and R¹ and R² are as defined in one row of table A; |
| Tabelle A109: | Compounds of the formulae Ia, Ib and Ic, wherein X is CH, Y is N, Z is C-N(CH₃)₂, R³ is CH₃ and R¹ and R² are as defined in one row of table A; |
| Tabelle A110: | Compounds of the formulae Ia, Ib and Ic, wherein X is CH, Y is N, Z is C-N(CH₃)₂, R³ is C₂H₅ and R¹ and R² are as defined in one row of table A; |
| Tabelle A111: | Compounds of the formulae Ia, Ib and Ic, wherein X is CH, Y is N, Z is C-N(CH₃)₂, R³ is CF₃ and R¹ and R² are as defined in one row of table A; |
| Tabelle A112: | Compounds of the formulae Ia, Ib and Ic, wherein X is CH, Y is N, Z is C-N(CH₃)₂, R³ is CCl₃ and R¹ and R² are as defined in one row of table A; |
| Tabelle A113: | Compounds of the formulae Ia, Ib and Ic, wherein X is CH, Y is N, Z is C-N(CH₃)₂, R³ is F and R¹ and R² are as defined in one row of table A; |
| Tabelle A114: | Compounds of the formulae Ia, Ib and Ic, wherein X is CH, Y is N, Z is C-N(CH₃)₂, R³ is Cl and R¹ and R² are as defined in one row of table A; |
| Tabelle A115: | Compounds of the formulae la, Ib and Ic, wherein X is CH, Y is N, Z is C-N(CH₃)₂, R³ is Br and R¹ and R² are as defined in one row of table A; |
| Tabelle A116: | Compounds of the formulae Ia, Ib and Ic, wherein X is CH, Y is N, Z is C-N(CH₃)₂, R³ is I and R¹ and R² are as defined in one row of table A; |
| Tabelle A117: | Compounds of the formulae la, Ib and Ic, wherein X is CH, Y is N, Z is C-N(CH₃)₂, R³ is OCH₃ and R¹ and R² are as defined in one row of table A; |
| Tabelle A118: | Compounds of the formulae Ia, Ib and Ic, wherein X is CH, Y is N, Z is C-N(CH₃)₂, R³ is OCF₃ and R¹ and R² are as defined in one row of table A; |
| Tabelle A119: | Compounds of the formulae Ia, Ib and Ic, wherein X is CH, Y is N, Z is C-N(CH₃)₂, R³ is OCCl₃ and R¹ and R² are as defined in one row of table A; |
| Tabelle A120: | Compounds of the formulae la, Ib and Ic, wherein X is CH, Y is N, Z is C-N(CH₃)₂, R³ is OCHF₂ and R¹ and R² are as defined in one row of table A; |
| Tabelle A121: | Compounds of the formulae Ia, Ib and Ic, wherein X is CH, Y is N, Z is C-N(CH₃)₂, R³ is OCClF₂ and R¹ and R² are as defined in one row of table A; |
| Tabelle A122: | Compounds of the formulae la, Ib and Ic, wherein X is CH, Y is N, Z is C-N(CH₃)₂, R³ is OCH₂F and R¹ and R² are as defined in one row of table A; |
| Tabelle A123: | Compounds of the formulae Ia, Ib and Ic, wherein X is CH, Y is N, Z is C-N(CH₃)₂, R³ is OCHCl₂ and R¹ and R² are as defined in one row of table A; |
| Tabelle A124: | Compounds of the formulae Ia, Ib and Ic, wherein X is CH, Y is N, Z is C-N(CH₃)₂I, R³ is OCH₂Cl and R¹ and R² are as defined in one row of table A; |
| Tabelle A125: | Compounds of the formulae Ia, Ib and Ic, wherein X is CH, Y is N, Z is C-N(CH₃)₂, R³ is OCH₂-OCH₃ and R¹ and R² are as defined in one row of table A; |
| Tabelle A126: | Compounds of the formulae Ia, Ib and Ic, wherein X is CH, Y is N, Z is C-N(CH₃)₂, R³ is OCH₂CH₃ and R¹ and R² are as defined in one row of table A; |
| Tabelle A127: | Compounds of the formulae Ia, Ib and Ic, wherein X is CH, Y is N, Z is C-CH₃, R³ is CH₃ and R¹ and R² are as defined in one row of table A; |
| Tabelle A128: | Compounds of the formulae Ia, Ib and Ic, wherein X is CH, Y is N, Z is C-CH₃, R³ is C₂H₅ and R¹ and R² are as defined in one row of table A; |
| Tabelle A129: | Compounds of the formulae Ia, Ib and Ic, wherein X is CH, Y is N, Z is C-CH₃, R³ is CF₃ and R¹ and R² are as defined in one row of table A; |
| Tabelle A130: | Compounds of the formulae Ia, Ib and Ic, wherein X is CH, Y is N, Z is C-CH₃, R³ is CCl₃ and R¹ and R² are as defined in one row of table A; |
| Tabelle A131: | Compounds of the formulae Ia, Ib and Ic, wherein X is CH, Y is N, Z is C-CH₃, R³ is F and R¹ and R² are as defined in one row of table A; |
| Tabelle A132: | Compounds of the formulae Ia, Ib and Ic, wherein X is CH, Y is N, Z is C-CH₃, R³ is Cl and R¹ and R² are as defined in one row of table A; |
| Tabelle A133: | Compounds of the formulae Ia, Ib and Ic, wherein X is CH, Y is N, Z is C-CH₃, R³ is Br and R¹ and R² are as defined in one row of table A; |
| Tabelle A134: | Compounds of the formulae Ia, Ib and Ic, wherein X is CH, Y is N, Z is C-CH₃, R³ is I and R¹ and R² are as defined in one row of table A; |
| Tabelle A135: | Compounds of the formulae Ia, Ib and Ic, wherein X is CH, Y is N, Z is C-CH₃, R³ is OCH₃ and R¹ and R² are as defined in one row of table A; |
| Tabelle A136: | Compounds of the formulae Ia, Ib and Ic, wherein X is CH, Y is N, Z is C-CH₃, R³ is OCF₃ and R¹ and R² are as defined in one row of table A; |
| Tabelle A137: | Compounds of the formulae Ia, Ib and Ic, wherein X is CH, Y is N, Z is C-CH3, R³ is OCCl₃ and R¹ and R² are as defined in one row of table A; |
| Tabelle A138: | Compounds of the formulae Ia, Ib and Ic, wherein X is CH, Y is N, Z is C-CH₃, R³ is OCHF₂ and R¹ and R² are as defined in one row of table A; |
| Tabelle A139: | Compounds of the formulae Ia, Ib and Ic, wherein X is CH, Y is N, Z is C-CH₃, R³ is OCClF₂ and R¹ and R² are as defined in one row of table A; |
| Tabelle A140: | Compounds of the formulae Ia, Ib and Ic, wherein X is CH, Y is N, Z is C-CH₃, R³ is OCH₂F and R¹ and R² are as defined in one row of table A; |
| Tabelle A141: | Compounds of the formulae Ia, Ib and Ic, wherein X is CH, Y is N, Z is C-CH₃, R³ is OCHCl₂ and R¹ and R² are as defined in one row of table A; , |
| Tabelle A142: | Compounds of the formulae la, Ib and Ic, wherein X is CH, Y is N, Z is C-CH₃, R³ is OCH₂Cl and R¹ and R² are as defined in one row of table A; |
| Tabelle A143: | Compounds of the formulae la, lb and lc, wherein X is CH, Y is N, Z is C-CH₃, R³ is OCH₂-OCH₃ and R¹ and R² are as defined in one row of table A; |
| Tabelle A144: | Compounds of the formulae Ia, Ib and Ic, wherein X is CH, Y is N, Z is C-CH₃, R³ is OCH₂CH₃ and R¹ and R² are as defined in one row of table A; |
| Tabelle A145: | Compounds of the formulae Ia, Ib and Ic, wherein X is CH, Y is N, Z is C-CF₃, R³ is CH₃ and R¹ and R² are as defined in one row of table A; |
| Tabelle A146: | Compounds of the formulae Ia, Ib and Ic, wherein X is CH, Y is N, Z is C-CF₃, R³ is C₂H₅ and R¹ and R² are as defined in one row of table A; |
| Tabelle A147: | Compounds of the formulae Ia, Ib and Ic, wherein X is CH, Y is N, Z is C-CF₃, R³ is CF₃ and R¹ and R² are as defined in one row of table A; |
| Tabelle A148: | Compounds of the formulae Ia, Ib and Ic, wherein X is CH, Y is N, Z is C-CF₃, R³ is CCl₃ and R¹ and R² are as defined in one row of table A; |
| Tabelle A149: | Compounds of the formulae Ia, Ib and Ic, wherein X is CH, Y is N, Z is C-CF₃, R³ is F and R¹ and R² are as defined in one row of table A; |
| Tabelle A150: | Compounds of the formulae Ia, Ib and Ic, wherein X is CH, Y is N, Z is C-CF₃, R³ is Cl and R¹ and R² are as defined in one row of table A; |
| Tabelle A151: | Compounds of the formulae Ia, Ib and Ic, wherein X is CH, Y is N, Z is C-CF₃, R³ is Br and R¹ and R² are as defined in one row of table A; |
| Tabelle A152: | Compounds of the formulae Ia, Ib and Ic, wherein X is CH, Y is N, Z is C-CF₃, R³ is I and R¹ and R² are as defined in one row of table A; |
| Tabelle A153: | Compounds of the formulae Ia, Ib and Ic, wherein X is CH, Y is N, Z is C-CF₃, R³ is OCH₃ and R¹ and R² are as defined in one row of table A; |
| Tabelle A154: | Compounds of the formulae Ia, Ib and Ic, wherein X is CH, Y is N, Z is C-CF₃, R³ is OCF₃ and R¹ and R² are as defined in one row of table A; |
| Tabelle A155: | Compounds of the formulae Ia, Ib and Ic, wherein X is CH, Y is N, Z is C-CF₃, R³ is OCCl₃ and R¹ and R² are as defined in one row of table A; |
| Tabelle A156: | Compounds of the formulae Ia, Ib and Ic, wherein X is CH, Y is N, Z is C-CF₃, R³ is OCHF₂ and R¹ and R² are as defined in one row of table A; |
| Tabelle A157: | Compounds of the formulae Ia, Ib and Ic, wherein X is CH, Y is N, Z is C-CF₃, R³ is OCClF₂ and R¹ and R² are as defined in one row of table A; |
| Tabelle A158: | Compounds of the formulae Ia, Ib and Ic, wherein X is CH, Y is N, Z is C-CF₃, R³ is OCH₂F and R¹ and R² are as defined in one row of table A; |
| Tabelle A159: | Compounds of the formulae Ia, Ib and Ic, wherein X is CH, Y is N, Z is C-CF₃, R³ is OCHCl₂ and R¹ and R² are as defined in one row of table A; |
| Tabelle A160: | Compounds of the formulae Ia, Ib and Ic, wherein X is CH, Y is N, Z is C-CF₃, R³ is OCH₂Cl and R¹ and R² are as defined in one row of table A; |
| Tabelle A161: | Compounds of the formulae Ia, Ib and Ic, wherein X is CH, Y is N, Z is C-CF₃, R³ is OCH₂-OCH₃ and R¹ and R² are as defined in one row of table A; |
| Tabelle A162: | Compounds of the formulae Ia, Ib and Ic, wherein X is CH, Y is N, Z is C-CF₃, R³ is OCH₂CH₃ and R¹ and R² are as defined in one row of table A; |
| Tabelle A163: | Compounds of the formulae Ia, Ib and Ic, wherein X is CH, Y is N, Z is C-OCH₃, R³ is CH₃ and R¹ and R² are as defined in one row of table A; |
| Tabelle A164: | Compounds of the formulae Ia, Ib and Ic, wherein X is CH, Y is N, Z is C-OCH₃, R³ is C₂H₅ and R¹ and R² are as defined in one row of table A; |
| Tabelle A165: | Compounds of the formulae Ia, Ib and Ic, wherein X is CH, Y is N, Z is C-OCH₃, R³ is CF₃ and R¹ and R² are as defined in one row of table A; |
| Tabelle A166: | Compounds of the formulae Ia, Ib and Ic, wherein X is CH, Y is N, Z is C-OCH₃, R³ is CCl₃ and R¹ and R² are as defined in one row of table A; |
| Tabelle A167: | Compounds of the formulae Ia, Ib and Ic, wherein X is CH, Y is N, Z is C-OCH₃, R³ is F and R¹ and R² are as defined in one row of table A; |
| Tabelle A168: | Compounds of the formulae Ia, Ib and Ic, wherein X is CH, Y is N, Z is C-OCH₃, R³ is Cl and R¹ and R² are as defined in one row of table A; |
| Tabelle A169: | Compounds of the formulae Ia, Ib and Ic, wherein X is CH, Y is N, Z is C-OCH₃, R³ is Br and R¹ and R² are as defined in one row of table A; |
| Tabelle A170: | Compounds of the formulae Ia, Ib and Ic, wherein X is CH, Y is N, Z is C-OCH₃, R³ is I and R¹ and R² are as defined in one row of table A; |
| Tabelle A171: | Compounds of the formulae Ia, Ib and Ic, wherein X is CH, Y is N, Z is C-OCH₃, R³ is OCH₃ and R¹ and R² are as defined in one row of table A; |
| Tabelle A172: | Compounds of the formulae Ia, Ib and Ic, wherein X is CH, Y is N, Z is C-OCH₃, R³ is OCF₃ and R¹ and R² are as defined in one row of table A; |
| Tabelle A173: | Compounds of the formulae Ia, Ib and Ic, wherein X is CH, Y is N, Z is C-OCH₃, R³ is OCCl₃ and R¹ and R² are as defined in one row of table A; |
| Tabelle A174: | Compounds of the formulae Ia, Ib and Ic, wherein X is CH, Y is N, Z is C-OCH₃, R³ is OCHF₂ and R¹ and R² are as defined in one row of table A; |
| Tabelle A175: | Compounds of the formulae Ia, Ib and Ic, wherein X is CH, Y is N, Z is C-OCH₃, R³ is OCClF₂ and R¹ and R² are as defined in one row of table A; |
| Tabelle A176: | Compounds of the formulae Ia, Ib and Ic, wherein X is CH, Y is N, Z is C-OCH₃, R³ is OCH₂F and R¹ and R² are as defined in one row of table A; |
| Tabelle A177: | Compounds of the formulae Ia, Ib and Ic, wherein X is CH, Y is N, Z is C-OCH₃, R³ is OCHCl₂ and R¹ and R² are as defined in one row of table A; |
| Tabelle A178: | Compounds of the formulae Ia, Ib and Ic, wherein X is CH, Y is N, Z is C-OCH₃, R³ is OCH₂Cl and R¹ and R² are as defined in one row of table A; |
| Tabelle A179: | Compounds of the formulae Ia, Ib and Ic, wherein X is CH, Y is N, Z is C-OCH₃, R³ is OCH₂-OCH₃ and R¹ and R² are as defined in one row of table A; |
| Tabelle A180: | Compounds of the formulae Ia, Ib and Ic, wherein X is CH, Y is N, Z is C-OCH₃, R³ is OCH₂CH₃ and R¹ and R² are as defined in one row of table A; |
| Tabelle A181: | Compounds of the formulae Ia, Ib and Ic, wherein X is CH, Y is N, Z is C-CCl₃, R³ is CH₃ and R¹ and R² are as defined in one row of table A; |
| Tabelle A182: | Compounds of the formulae Ia, Ib and Ic, wherein X is CH, Y is N, Z is C-CCl₃, R³ is C₂H₅ and R¹ and R² are as defined in one row of table A; |
| Tabelle A183: | Compounds of the formulae Ia, Ib and Ic, wherein X is CH, Y is N, Z is C-CCl₃, R³ is CF₃ and R¹ and R² are as defined in one row of table A; |
| Tabelle A184: | Compounds of the formulae Ia, Ib and Ic, wherein X is CH, Y is N, Z is C-CCl₃, R³ is CCl₃ and R¹ and R² are as defined in one row of table A; |
| Tabelle A185: | Compounds of the formulae Ia, Ib and Ic, wherein X is CH, Y is N, Z is C-CCl₃, R³ is F and R¹ and R² are as defined in one row of table A; |
| Tabelle A186: | Compounds of the formulae Ia, Ib and Ic, wherein X is CH, Y is N, Z is C-CCl₃; R³ is Cl and R¹ and R² are as defined in one row of table A; |
| Tabelle A187: | Compounds of the formulae Ia, Ib and Ic, wherein X is CH, Y is N, Z is C-CCl₃, R³ is Br and R¹ and R² are as defined in one row of table A; |
| Tabelle A188: | Compounds of the formulae Ia, Ib and Ic, wherein X is CH, Y is N, Z is C-CCl₃, R³ is I and R¹ and R² are as defined in one row of table A; |
| Tabelle A189: | Compounds of the formulae Ia, Ib and Ic, wherein X is CH, Y is N, Z is C-CCl₃, R³ is OCH₃ and R¹ and R² are as defined in one row of table A; |
| Tabelle A190: | Compounds of the formulae Ia, Ib and Ic, wherein X is CH, Y is N, Z is C-CCl₃, R³ is OCF₃ and R¹ and R² are as defined in one row of table A; |
| Tabelle A191: | Compounds of the formulae Ia, Ib and Ic, wherein X is CH, Y is N, Z is C-CCl₃, R³ is OCCl₃ and R¹ and R² are as defined in one row of table A; |
| Tabelle A192: | Compounds of the formulae Ia, Ib and Ic, wherein X is CH, Y is N, Z is C-CCl₃, R³ is OCHF₂ and R¹ and R² are as defined in one row of table A; |
| Tabelle A193: | Compounds of the formulae Ia, Ib and Ic, wherein X is CH, Y is N, Z is C-CCl₃, R³ is OCClF₂ and R¹ and R² are as defined in one row of table A; |
| Tabelle A194: | Compounds of the formulae Ia, Ib and Ic, wherein X is CH, Y is N, Z is C-CCl₃, R³ is OCH₂F and R¹ and R² are as defined in one row of table A; |
| Tabelle A195: | Compounds of the formulae Ia, Ib and Ic, wherein X is CH, Y is N, Z is C-CCl₃, R³ is OCHCl₂ and R¹ and R² are as defined in one row of table A; |
| Tabelle A196: | Compounds of the formulae Ia, Ib and Ic, wherein X is CH, Y is N, Z is C-CCl₃, R³ is OCH₂Cl and R¹ and R² are as defined in one row of table A; |
| Tabelle A197: | Compounds of the formulae Ia, Ib and Ic, wherein X is CH, Y is N, Z is C-CCl₃, R³ is OCH₂-OCH₃ and R¹ and R² are as defined in one row of table A; |
| Tabelle A198: | Compounds of the formulae Ia, Ib and Ic, wherein X is CH, Y is N, Z is C-CCl₃, R³ is OCH₂CH₃ and R¹ and R² are as defined in one row of table A; |
| Tabelle A199: | Compounds of the formulae Ia, Ib and Ic, wherein X is CH, Y is N, Z is C-C₂H₅, R³ is CH₃ and R¹ and R² are as defined in one row of table A; |
| Tabelle A200: | Compounds of the formulae Ia, Ib and Ic, wherein X is CH, Y is N, Z is C-C₂H₅, R³ is C₂H₅ and R¹ and R² are as defined in one row of table A; |
| Tabelle A201: | Compounds of the formulae Ia, Ib and Ic, wherein X is CH, Y is N, Z is C-C₂H₅, R³ is CF₃ and R¹ and R² are as defined in one row of table A; |
| Tabelle A202: | Compounds of the formulae Ia, Ib and Ic, wherein X is CH, Y is N, Z is C-C₂H₅, R³ is CCl₃ and R¹ and R² are as defined in one row of table A; |
| Tabelle A203: | Compounds of the formulae Ia, Ib and Ic, wherein X is CH, Y is N, Z is C-C₂H₅, R³ is F and R¹ and R² are as defined in one row of table A; |
| Tabelle A204: | Compounds of the formulae Ia, Ib and Ic, wherein X is CH, Y is N, Z is C-C₂H₅, R³ is Cl and R¹ and R² are as defined in one row of table A; |
| Tabelle A205: | Compounds of the formulae Ia, Ib and Ic, wherein X is CH, Y is N, Z is C-C₂H₅, R³ is Br and R¹ and R² are as defined in one row of table A; |
| Tabelle A206: | Compounds of the formulae Ia, Ib and Ic, wherein X is CH, Y is N, Z is C-C₂H₅, R³ is I and R¹ and R² are as defined in one row of table A; |
| Tabelle A207: | Compounds of the formulae Ia, Ib and Ic, wherein X is CH, Y is N, Z is C-C₂H₅, R³ is OCH₃ and R¹ and R² are as defined in one row of table A; |
| Tabelle A208: | Compounds of the formulae Ia, Ib and Ic, wherein X is CH, Y is N, Z is C-C₂H₅, R³ is OCF₃ and R¹ and R² are as defined in one row of table A; |
| Tabelle A209: | Compounds of the formulae Ia, Ib and Ic, wherein X is CH, Y is N, Z is C-C₂H₅, R³ is OCCl₃ and R¹ and R² are as defined in one row of table A; |
| Tabelle A210: | Compounds of the formulae Ia, Ib and Ic, wherein X is CH, Y is N, Z is C-C₂H₅, R³ is OCHF₂ and R¹ and R² are as defined in one row of table A; |
| Tabelle A211: | Compounds of the formulae Ia, Ib and Ic, wherein X. is CH, Y is N, Z is C-C₂H₅, R³ is OCClF₂ and R¹ and R² are as defined in one row of table A; |
| Tabelle A212: | Compounds of the formulae Ia, Ib and Ic, wherein X is CH, Y is N, Z is C-C₂H₅, R³ is OCH₂F and R¹ and R² are as defined in one row of table A; |
| Tabelle A213: | Compounds of the formulae Ia, Ib and Ic, wherein X is CH, Y is N, Z is C-C₂H₅, R³ is OCHCl₂ and R¹ and R² are as defined in one row of table A; |
| Tabelle A214: | Compounds of the formulae Ia, Ib and Ic, wherein X is CH, Y is N, Z is C-C₂H₅, R³ is OCH₂Cl and R¹ and R² are as defined in one row of table A; |
| Tabelle A215: | Compounds of the formulae Ia, Ib and Ic, wherein X is CH, Y is N, Z is C-C₂H₅, R³ is OCH₂-OCH₃ and R¹ and R² are as defined in one row of table A; |
| Tabelle A216: | Compounds of the formulae Ia, Ib and Ic, wherein X is CH, Y is N, Z is C-C₂H₅, R³ is OCH₂CH₃ and R¹ and R² are as defined in one row of table A; |
| Tabelle A217: | Compounds of the formulae Ia, Ib and Ic, wherein X is CH, Y is N, Z is C-OC₂H₅, R³ is CH₃ and R¹ and R² are as defined in one row of table A; |
| Tabelle A218: | Compounds of the formulae Ia, Ib and Ic, wherein X is CH, Y is N, Z is C-OC₂H₅. R³ is C₂H₅ and R¹ and R² are as defined in one row of table A; |
| Tabelle A219: | Compounds of the formulae Ia, Ib and Ic, wherein X is CH, Y is N, Z is C-OC₂H₅, R³ is CF₃ and R¹ and R² are as defined in one row of table A; |
| Tabelle A220: | Compounds of the formulae Ia, Ib and Ic, wherein X is CH, Y is N, Z is C-OC₂H₅, R³ is CCl₃ and R¹ and R² are as defined in one row of table A; |
| Tabelle A221: | Compounds of the formulae Ia, Ib and Ic, wherein X is CH, Y is N, Z is C-OC₂H₅, R³ is F and R¹ and R² are as defined in one row of table A; |
| Tabelle A222: | Compounds of the formulae Ia, Ib and Ic, wherein X is CH, Y is N, Z is C-OC₂H₅, R³ is Cl and R¹ and R² are as defined in one row of table A; |
| Tabelle A223: | Compounds of the formulae Ia, Ib and Ic, wherein X is CH, Y is N, Z is C-OC₂H₅, R³ is Br and R¹ and R² are as defined in one row of table A; |
| Tabelle A224: | Compounds of the formulae Ia, Ib and Ic, wherein X is CH, Y is N, Z is C-OC₂H₅, R³ is I and R¹ and R² are as defined in one row of table A; |
| Tabelle A225: | Compounds of the formulae Ia, Ib and Ic, wherein X is CH, Y is N, Z is GOC₂H₅, R³ is OCH₃ and R¹ and R² are as defined in one row of table A; |
| Tabelle A226: | Compounds of the formulae Ia, Ib and Ic, wherein X is CH, Y is N, Z is C-OC₂H₅, R³ is OCF₃ and R¹ and R² are as defined in one row of table A; |
| Tabelle A227: | Compounds of the formulae Ia, Ib and Ic, wherein X is CH, Y is N, Z is C-OC₂H₅, R³ is OCCl₃ and R¹ and R² are as defined in one row of table A; |
| Tabelle A228: | Compounds of the formulae Ia, Ib and Ic, wherein X is CH, Y is N, Z is C-OC₂H₅, R³ is OCHF₂ and R¹ and R² are as defined in one row of table As; |
| Tabelle A229: | Compounds of the formulae Ia, Ib and Ic, wherein X is CH, Y is N, Z is C-OC₂H₅, R³ is OCClF₂ and R¹ and R² are as defined in one row of table A; |
| Tabelle A230: | Compounds of the formulae Ia, Ib and Ic, wherein X is CH, Y is N, Z is C-OC₂H₅, R³ is OCH₂F and R¹ and R² are as defined in one row of table A; |
| Tabelle A231: | Compounds of the formulae Ia, Ib and Ic, wherein X is CH, Y is N, Z is C-OC₂H₅, R³ is OCHCl₂ and R¹ and R² are as defined in one row of table A; |
| Tabelle A232: | Compounds of the formulae Ia, Ib and Ic, wherein X is CH, Y is N, Z is C-OC₂H₅, R³ is OCH₂Cl and R¹ and R² are as defined in one row of table A; |
| Tabelle A233: | Compounds of the formulae Ia, Ib and Ic, wherein X is CH, Y is N, Z is C-OC₂H₅, R³ is OCH₂-OCH₃ and R¹ and R² are as defined in one row of table A; |
| Tabelle A234: | Compounds of the formulae Ia, Ib and Ic, wherein X is CH, Y is N, Z is OC-C₂H₅, R³ is OCH₂CH₃ and R¹ and R² are as defined in one row of table A; |
| Tabelle A235: | Compounds of the formulae Ia, Ib and Ic, wherein Z is CH, Y is N, X is C-Cl, R³ is CH₃ and R¹ and R² are as defined in one row of table A; |
| Tabelle A236: | Compounds of the formulae Ia, Ib and Ic, wherein Z is CH, Y is N, X is C-Cl, R³ is C₂H₅ and R¹ and R² are as defined in one row of table A; |
| Tabelle A237: | Compounds of the formulae Ia, Ib and Ic, wherein Z is CH, Y is N, X is C-Cl, R³ is CF₃ and R¹ and R² are as defined in one row of table A; |
| Tabelle A238: | Compounds of the formulae Ia, Ib and Ic, wherein Z is CH, Y is N, X is C-Cl, R³ is CCl₃ and R¹ and R² are as defined in one row of table A; |
| Tabelle A239: | Compounds of the formulae Ia, Ib and Ic, wherein Z is CH, Y is N, X is C-Cl, R³ is F and R¹ and R² are as defined in one row of table A; |
| Tabelle A240: | Compounds of the formulae Ia, Ib and Ic, wherein Z is CH, Y is N, X is C-Cl, R³ is Cl and R¹ and R² are as defined in one row of table A; |
| Tabelle A241: | Compounds of the formulae Ia, Ib and Ic, wherein Z is CH, Y is N, X is C-Cl, R³ is Br and R¹ and R² are as defined in one row of table A; |
| Tabelle A242: | Compounds of the formulae Ia, Ib and Ic, wherein Z is CH, Y is N, X is C-Cl, R³ is I and R¹ and R² are as defined in one row of table A; |
| Tabelle A243: | Compounds of the formulae Ia, Ib and Ic, wherein Z is CH, Y is N, X is C-Cl, R³ is OCH₃ and R¹ and R² are as defined in one row of table A; |
| Tabelle A244: | Compounds of the formulae Ia, Ib and Ic, wherein Z is CH, Y is N, X is C-Cl, R³ is OCF₃ and R¹ and R² are as defined in one row of table A; |
| Tabelle A245: | Compounds of the formulae Ia, Ib and Ic, wherein Z is CH, Y is N, X is C-Cl, R³ is OCCl₃ and R¹ and R² are as defined in one row of table A; |
| Tabelle A246: | Compounds of the formulae Ia, Ib and Ic, wherein Z is CH, Y is N, X is C-Cl, R³ is OCHF₂ and R¹ and R² are as defined in one row of table A; |
| Tabelle A247: | Compounds of the formulae Ia, Ib and Ic, wherein Z is CH, Y is N, X is C-Cl, R³ is OCClF₂ and R¹ and R² are as defined in one row of table A; |
| Tabelle A248: | Compounds of the formulae Ia, Ib and Ic, wherein Z is CH, Y is N, X is C-Cl, R³ is OCH₂F and R¹ and R² are as defined in one row of table A; |
| Tabelle A249: | Compounds of the formulae Ia, Ib and Ic, wherein Z is CH, Y is N, X is C-Cl, R³ is OCHCl₂ and R¹ and R² are as defined in one row of table A; |
| Tabelle A250: | Compounds of the formulae Ia, Ib and Ic, wherein Z is CH, Y is N, X is C-Cl, R³ is OCH₂Cl and R¹ and R² are as defined in one row of table A; |
| Tabelle A251: | Compounds of the formulae Ia, Ib and Ic, wherein Z is CH, Y is N, X is C-Cl, R³ is OCH₂-OCH₃ and R¹ and R² are as defined in one row of table A; |
| Tabelle A252: | Compounds of the formulae Ia, Ib and Ic, wherein Z is CH, Y is N, X is C-Cl, R³ is OCH₂CH₃ and R¹ and R² are as defined in one row of table A; |
| Tabelle A253: | Compounds of the formulae Ia, Ib and Ic, wherein Z is CH, Y is N, X is C-F, R³ is CH₃ and R¹ and R² are as defined in one row of table A; |
| Tabelle A254: | Compounds of the formulae Ia, Ib and Ic, wherein Z is CH, Y is N, X is C-F, R³ is C₂H₅ and R¹ and R² are as defined in one row of table A; |
| Tabelle A255: | Compounds of the formulae Ia, Ib and Ic, wherein Z is CH, Y is N, X is C-F, R³ is CF₃ and R¹ and R² are as defined in one row of table A; |
| Tabelle A256: | Compounds of the formulae Ia, Ib and Ic, wherein Z is CH, Y is N, X is C-F, R³ is CCl₃ and R¹ and R² are as defined in one row of table A; |
| Tabelle A257: | Compounds of the formulae Ia, Ib and Ic, wherein Z is CH, Y is N, X is C-F, R³ is F and R¹ and R² are as defined in one row of table A; |
| Tabelle A258: | Compounds of the formulae Ia, Ib and Ic, wherein Z is CH, Y is N, X is C-F, R³ is Cl and R¹ and R² are as defined in one row of table A; |
| Tabelle A259: | Compounds of the formulae Ia, Ib and Ic, wherein Z is CH, Y is N, X is C-F, R³ is Br and R¹ and R² are as defined in one row of table A; |
| Tabelle A260: | Compounds of the formulae Ia, Ib and Ic, wherein Z is CH, Y is N, X is C-F, R³ is and R¹ and R² are as defined in one row of table A; |
| Tabelle A261: | Compounds of the formulae Ia, Ib and Ic, wherein Z is CH, Y is N, X is C-F, R³ is OCH₃ and R¹ and R² are as defined in one row of table A; |
| Tabelle A262: | Compounds of the formulae Ia, Ib and Ic, wherein Z is CH, Y is N, X is C-F, R³ is OCF₃ and R¹ and R² are as defined in one row of table A; |
| Tabelle A263: | Compounds of the formulae Ia, Ib and Ic, wherein Z is CH, Y is N, X is C-F, R³ is OCCl₃ and R¹ and R² are as defined in one row of table A; |
| Tabelle A264: | Compounds of the formulae Ia, Ib and Ic, wherein Z is CH, Y is N, X is C-F, R³ is OCHF₂ and R¹ and R² are as defined in one row of table A; |
| Tabelle A265: | Compounds of the formulae Ia, Ib and Ic, wherein Z is CH, Y is N, X is C-F, R³ is OCCIF₂ and R¹ and R² are as defined in one row of table A; |
| Tabelle A266: | Compounds of the formulae Ia, Ib and Ic, wherein Z is CH, Y is N, X is C-F, R³ is OCH₂F and R¹ and R² are as defined in one row of table A; |
| Tabelle A267: | Compounds of the formulae Ia, Ib and Ic, wherein Z is CH, Y is N, X is C-F, R³ is OCHCl₂ and R¹ and R² are as defined in one row of table A; |
| Tabelle A268: | Compounds of the formulae Ia, Ib and Ic, wherein Z is CH, Y is N, X is C-F, R³ is OCH₂Cl and R¹ and R² are as defined in.one row of table A; |
| Tabelle A269: | Compounds of the formulae Ia, Ib and Ic, wherein Z is CH, Y is N, X is C-F, R³ is OCH₂OCH₃ and R¹ and R² are as defined in one row of table A; |
| Tabelle A270: | Compounds of the formulae Ia, Ib and Ic, wherein Z is CH, Y is N, X is C-F, R³ is OCH₂CH₃ and R¹ and R² are as defined in one row of table A; |
| Tabelle A271: | Compounds of the formulae Ia, Ib and Ic, wherein Z is CH, Y is N, X is C-Br, R³ is CH₃ and R¹ and R² are as defined in one row of table A; |
| Tabelle A272: | Compounds of the formulae Ia, Ib and Ic, wherein Z is CH, Y is N, X is . C-Br, R³ is C₂H₅ and R¹ and R² are as defined in one row of table A; |
| Tabelle A273: | Compounds of the formulae Ia, Ib and Ic, wherein Z is CH, Y is N, X is C-Br, R³ is CF₃ and R¹ and R² are as defined in one row of table A; |
| Tabelle A274: | Compounds of the formulae Ia, Ib and Ic, wherein Z is CH, Y is N, X is C-Br, R³ is CCl₃ and R¹ and R² are as defined in one row of table A; |
| Tabelle A275: | Compounds of the formulae Ia, Ib and Ic, wherein Z is CH, Y is N, X is C-Br, R³ is F and R¹ and R² are as defined in one row of table A; |
| Tabelle A276: | Compounds of the formulae Ia, Ib and Ic, wherein Z is CH, Y is N, X is C-Br, R³ is Cl and R¹ and R² are as defined in one row of table A; |
| Tabelle A277: | Compounds of the formulae Ia, Ib and Ic, wherein Z is CH, Y is N, X is C-Br, R³ is Br and R¹ and R² are as defined in one row of table A; |
| Tabelle A278: | Compounds of the formulae Ia, Ib and Ic, wherein Z is CH, Y is N, X is |
| | C-Br, R³ is I and R¹ and R² are as defined in one row of table A; |
| Tabelle A279: | Compounds of the formulae Ia, Ib and Ic, wherein Z is CH, Y is N, X is C-Br, R³ is OCH₃ and R¹ and R² are as defined in one row of table A; |
| Tabelle A280: | Compounds of the formulae Ia, Ib and Ic, wherein Z is CH, Y is N, X is C-Br, R³ is OCF₃ and R¹ and R² are as defined in one row of table A; |
| Tabelle A281: | Compounds of the formulae Ia, Ib and Ic, wherein Z is CH, Y is N, X is C-Br, R³ is OCCl₃ and R¹ and R² are as defined in one row of table A; |
| Tabelle A282: | Compounds of the formulae Ia, Ib and Ic, wherein Z is CH, Y is N, X is C-Br, R³ is OCHF₂ and R¹ and R² are as defined in one row of table A; |
| Tabelle A283: | Compounds of the formulae Ia, Ib and Ic, wherein Z is CH, Y is N, X is C-Br, R³ is OCClF₂ and R¹ and R² are as defined in one row of table A; |
| Tabelle A284: | Compounds of the formulae Ia, Ib and Ic, wherein Z is CH, Y is N, X is C-Br, R³ is OCH₂F and R¹ and R² are as defined in one row of table A; |
| Tabelle A285: | Compounds of the formulae Ia, Ib and Ic, wherein Z is CH, Y is N, X is C-Br, R³ is OCHCl₂ and R¹ and R² are as defined in one row of table A; |
| Tabelle A286: | Compounds of the formulae Ia, Ib and Ic, wherein Z is CH, Y is N, X is C-Br, R³ is OCH₂Cl and R¹ and R² are as defined in one row of table A; |
| Tabelle A287: | Compounds of the formulae Ia, Ib and Ic, wherein Z is CH, Y is N, X is C-Br, R³ is OCH₂-OCH₃ and R¹ and R² are as defined in one row of table A; |
| Tabelle A288: | Compounds of the formulae Ia, Ib and Ic, wherein Z is CH, Y is N, X is C-Br, R³ is OCH₂CH₃ and R¹ and R² are as defined in one row of table A; |
| Tabelle A289: | Compounds of the formulae Ia, Ib and Ic, wherein Z is CH, Y is N, X is C-I, R³ is CH₃ and R¹ and R² are as defined in one row of table A; |
| Tabelle A290: | Compounds of the formulae Ia, Ib and Ic, wherein Z is CH, Y is N, X is C-I, R³ is C₂H₅ and R¹ and R² are as defined in one row of table A; |
| Tabelle A291: | Compounds of the formulae Ia, Ib and Ic, wherein Z is CH, Y is N, X is C-I, R³ is CF₃ and R¹ and R² are as defined in one row of table A; |
| Tabelle A292: | Compounds of the formulae Ia, Ib and Ic, wherein Z is CH, Y is N, X is C-I, R³ is CCl₃ and R¹ and R² are as defined in one row of table A; |
| Tabelle A293: | Compounds of the formulae Ia, Ib and Ic, wherein Z is CH, Y is N, X is C-I, R³ is F and R¹ and R² are as defined in one row of table A; |
| Tabelle A294: | Compounds of the formulae Ia, Ib and Ic, wherein Z is CH, Y is N, X is C-I, R³ is Cl and R¹ and R² are as defined in one row of table A; |
| Tabelle A295: | Compounds of the formulae Ia, Ib and Ic**,** wherein Z is CH, Y is N, X is C-I, R³ is Br and R¹ and R² are as defined in one row of table A; |
| Tabelle A296: | Compounds of the formulae Ia, Ib and Ic, wherein Z is CH, Y is N, X is C-I, R³ is I and R¹ and R² are as defined in one row of table A; |
| Tabelle A297: | Compounds of the formulae Ia, Ib and Ic, wherein Z is CH, Y is N, X is C-I, R³ is OCH₃ and R¹ and R² are as defined in one row of table A; |
| Tabelle A298: | Compounds of the formulae Ia, Ib and Ic, wherein Z is CH, Y is N, X is C-I, R³ is OCF₃ and R¹ and R² are as defined in one row of table A; |
| Tabelle A299: | Compounds of the formulae Ia, Ib and Ic, wherein Z is CH, Y is N, X is C-I, R³ is OCCl₃ and R¹ and R² are as defined in one row of table A; |
| Tabelle A300: | Compounds of the formulae Ia, Ib and Ic, wherein Z is CH, Y is N, X is C-I, R³ is OCHF₂ and R¹ and R² are as defined in one row of table A; |
| Tabelle A301: | Compounds of the formulae Ia, Ib and Ic, wherein Z is CH, Y is N, X is C-I, R³ is OCClF₂ and R¹ and R² are as defined in one row of table A; |
| Tabelle A302: | Compounds of the formulae Ia, Ib and Ic, wherein Z is CH, Y is N, X is C-I, R³ is OCH₂F and R¹ and R² are as defined in one row of table A; |
| Tabelle A303: | Compounds of the formulae Ia, Ib and Ic, wherein Z is CH, Y is N, X is C-I, R³ is OCHCl₂ and R¹ and R² are as defined in one row of table A; |
| Tabelle A304: | Compounds of the formulae Ia, Ib and Ic, wherein Z is CH, Y is N, X is C-I, R³ is OCH₂Cl and R¹ and R² are as defined in one row of table A; |
| Tabelle A305: | Compounds of the formulae Ia, Ib and Ic, wherein Z is CH, Y is N, X is C-I, R³ is OCH₂-OCH₃ and R¹ and R² are as defined in one row of table A; |
| Tabelle A306: | Compounds of the formulae Ia, Ib and Ic, wherein Z is CH, Y is N, X is C-I, R³ is OCH₂CH₃ and R¹ and R² are as defined in one row of table A; |
| Tabelle A307: | Compounds of the formulae Ia, Ib and Ic, wherein Z is CH, Y is N, X is C-NH₂, R³ is CH₃ and R¹ and R² are as defined in one row of table A; |
| Tabelle A308: | Compounds of the formulae Ia, Ib and Ic, wherein Z is CH, Y is N, X is C-NH₂, R³ is C₂H₅ and R¹ and R² are as defined in one row of table A; |
| Tabelle A309: | Compounds of the formulae Ia, Ib and Ic, wherein Z is CH, Y is N, X is C-NH₂, R³ is CF₃ and R¹ and R² are as defined in one row of table A; |
| Tabelle A310: | Compounds of the formulae Ia, Ib and Ic, wherein Z is CH, Y is N, X is C-NH₂, R³ is CCl₃ and R¹ and R² are as defined in one row of table A; |
| Tabelle A311: | Compounds of the formulae Ia, Ib and Ic, wherein Z is CH, Y is N, X is C-NH₂, R³ is F and R¹ and R² are as defined in one row of table A; |
| Tabelle A312: | Compounds of the formulae Ia, Ib and Ic, wherein Z is CH, Y is N, X is C-NH₂, R³ is Cl and R¹ and R² are as defined in one row of table A; |
| Tabelle A313: | Compounds of the formulae Ia, Ib and Ic, wherein Z is CH, Y is N, X is C-NH₂, R³ is Br and R¹ and R² are as defined in one row of table A; |
| Tabelle A314: | Compounds of the formulae Ia, Ib and Ic, wherein Z is CH, Y is N, X is C-NH₂, R³ is I and R¹ and R² are as defined in one row of table A; |
| Tabelle A315: | Compounds of the formulae Ia, Ib and Ic, wherein Z is CH, Y is N; X is C-NH₂, R³ is OCH₃ and R¹ and R² are as defined in one row of table A; |
| Tabelle A316: | Compounds of the formulae Ia, Ib and Ic, wherein Z is CH, Y is N, X is C-NH₂, R³ is OCF₃ and R¹ and R² are as defined in one row of table A; |
| Tabelle A317: | Compounds of the formulae Ia, Ib and Ic, wherein Z is CH, Y is N, X is C-NH₂, R³ is OCCl₃ and R¹ and R² are as defined in one row of table A; |
| Tabelle A318: | Compounds of the formulae Ia, Ib and Ic, wherein Z is CH, Y is N, X is C-NH₂, R³ is OCHF₂ and R¹ and R² are as defined in one row of table A; |
| Tabelle A319: | Compounds of the formulae Ia, Ib and Ic, wherein Z is CH, Y is N, X is C-NH₂, R³ is OCClF₂ and R¹ and R² are as defined in one row of table A; |
| Tabelle A320: | Compounds of the formulae Ia, Ib and Ic, wherein Z is CH, Y is N, X is C-NH₂, R³ is OCH₂F and R¹ and R² are as defined in one row of table A; |
| Tabelle A321: | Compounds of the formulae Ia, Ib and Ic, wherein Z is CH, Y is N, X is C-NH₂, R³ is OCHCl₂ and R¹ and R² are as defined in one row of table A; |
| Tabelle A322: | Compounds of the formulae Ia, Ib and Ic, wherein Z is CH, Y is N, X is C-NH₂, R³ is OCH₂Cl and R¹ and R² are as defined in one row of table A; |
| Tabelle A323: | Compounds of the formulae Ia, Ib and Ic, wherein Z is CH, Y is N, X is C-NH₂, R³ is OCH₂-OCH₃ and R¹ and R² are as defined in one row of table A; |
| Tabelle A324: | Compounds of the formulae Ia, Ib and Ic, wherein Z is CH, Y is N, X is C-NH₂, R³ is OCH₂CH₃ and R¹ and R² are as defined in one row of table A; |
| Tabelle A325: | Compounds of the formulae Ia, Ib and Ic, wherein Z is CH, Y is N, X is C-N(CH₃)₂, R³ is CH₃ and R¹ and R² are as defined in one row of table A; |
| Tabelle A326: | Compounds of the formulae Ia, Ib and Ic, wherein Z is CH, Y is N, X is C-N(CH₃)₂, R³ is C₂H₅ and R¹ and R² are as defined in one row of table A; |
| Tabelle A327: | Compounds of the formulae Ia, Ib and Ic, wherein Z is CH, Y is N, X is C-N(CH₃)₂, R³ is CF₃ and R¹ and R² are as defined in one row of table A; |
| Tabelle A328: | Compounds of the formulae Ia, Ib and Ic, wherein Z is CH, Y is N, X is C-N(CH₃)₂, R³ is CCl₃ and R¹ and R² are as defined in one row of table A; |
| Tabelle A329: | Compounds of the formulae Ia, Ib and Ic, wherein Z is CH, Y is N, X is C-N(CH₃)₂, R³ is F and R¹ and R² are as defined in one row of table A; |
| Tabelle A330: | Compounds of the formulae Ia, Ib and Ic, wherein Z is CH, Y is N, X is C-N(CH₃)₂, R³ is Cl and R¹ and R² are as defined in one row of table A; |
| Tabelle A331: | Compounds of the formulae Ia, Ib and Ic, wherein Z is CH, Y is N, X is C-N(CH₃)₂, R³ is Br and R¹ and R² are as defined in one row of table A; |
| Tabelle A332: | Compounds of the formulae Ia, Ib and Ic, wherein Z is CH, Y is N, X is C-N(CH₃)₂, R³ is I and R¹ and R² are as defined in one row of table A; |
| Tabelle A333: | Compounds of the formulae Ia, Ib and Ic, wherein Z is CH, Y is N, X is C-N(CH₃)₂, R³ is OCH₃ and R¹ and R² are as defined in one row of table A; |
| Tabelle A334: | Compounds of the formulae Ia, Ib and Ic, wherein Z is CH, Y is N, X is C-N(CH₃)₂, R³ is OCF₃ and R¹ and R² are as defined in one row of table A; |
| Tabelle A335: | Compounds of the formulae Ia, Ib and Ic, wherein Z is CH, Y is N, X is C-N(CH₃)₂, R³ is OCCl₃ and R¹ and R² are as defined in one row of table A; |
| Tabelle A336: | Compounds of the formulae Ia, Ib and Ic, wherein Z is CH, Y is N, X is C-N(CH₃)₂, R³ is OCHF₂ and R¹ and R² are as defined in one row of table A; |
| Tabelle A337: | Compounds of the formulae Ia, Ib and Ic, wherein Z is CH, Y is N, X is C-N(CH₃)₂, R³ is OCClF₂ and R¹ and R² are as defined in one row of table A; |
| Tabelle A338: | Compounds of the formulae Ia, Ib and Ic, wherein Z is CH, Y is N, X is C-N(CH₃)₂, R³ is OCH₂F and R¹ and R² are as defined in one row of table A; |
| Tabelle A339: | Compounds of the formulae Ia, Ib and Ic, wherein Z is CH, Y is N, X is C-N(CH₃)₂, R³ is OCHCl₂ and R¹ and R² are as defined in one row of table A; |
| Tabelle A340: | Compounds of the formulae Ia, Ib and Ic, wherein Z is CH, Y is N, X is . C-N(CH₃)₂I, R³ is OCH₂Cl and R¹ and R² are as defined in one row of table A; |
| Tabelle A341: | Compounds of the formulae Ia, Ib and Ic, wherein Z is CH, Y is N, X is C-N(CH₃)₂, R³ is OCH₂-OCH₃ and R¹ and R² are as defined in one row of table A; |
| Tabelle A342: | Compounds of the formulae Ia, Ib and Ic, wherein Z is CH, Y is N, X is C-N(CH₃)₂, R³ is OCH₂CH₃ and R¹ and R² are as defined in one row of table A; |
| Tabelle A343: | Compounds of the formulae Ia, Ib and Ic, wherein Z is CH, Y is N, X is C-CH₃, R³ is CH₃ and R¹ and R² are as defined in one row of table A; |
| Tabelle A344: | Compounds of the formulae Ia, Ib and Ic, wherein Z is CH, Y is N, X is C-CH₃, R³ is C₂H₅ and R¹ and R² are as defined in one row of table A; |
| Tabelle A345: | Compounds of the formulae Ia, Ib and Ic, wherein Z is CH, Y is N, X is C-CH₃, R³ is CF₃ and R¹ and R² are as defined in one row of table A; |
| Tabelle A346: | Compounds of the formulae Ia, Ib and Ic, wherein Z is CH, Y is N, X is C-CH₃, R³ is CCl₃ and R¹ and R² are as defined in one row of table A; |
| Tabelle A347: | Compounds of the formulae Ia, Ib and Ic, wherein Z is CH, Y is N, X is C-CH₃, R³ is F and R¹ and R² are as defined in one row of table A; |
| Tabelle A348: | Compounds of the formulae Ia, Ib and Ic, wherein Z is CH, Y is N, X is C-CH₃, R³ is Cl and R¹ and R² are as defined in one row of table A; |
| Tabelle A349: | Compounds of the formulae Ia, Ib and Ic, wherein Z is CH, Y is N, X is C-CH₃, R³ is Br and R¹ and R² are as defined in one row of table A; |
| Tabelle A350: | Compounds of the formulae Ia, Ib and Ic, wherein Z is CH, Y is N, X is C-CH₃, R³ is I and R¹ and R² are as defined in one row of table A; |
| Tabelle A351: | Compounds of the formulae Ia, Ib and Ic, wherein Z is CH, Y is N, X is C-CH₃, R³ is OCH₃ and R¹ and R² are as defined in one row of table A; |
| Tabelle A352: | Compounds of the formulae Ia, Ib and Ic, wherein Z is CH, Y is N, X is C-CH₃, R³ is OCF₃ and R¹ and R² are as defined in one row of table A; |
| Tabelle A353: | Compounds of the formulae Ia, Ib and Ic, wherein Z is CH, Y is N, X is C-CH₃, R³ is OCCl₃ and R¹ and R² are as defined in one row of table A; |
| Tabelle A354: | Compounds of the formulae Ia, Ib and Ic, wherein Z is CH, Y is N, X is C-CH₃, R³ is OCHF₂ and R¹ and R² are as defined in one row of table A; |
| Tabelle A355: | Compounds of the formulae Ia, Ib and Ic, wherein Z is CH, Y is N, X is C-CH₃, R³ is OCClF₂ and R¹ and R² are as defined in one row of table A; |
| Tabelle A356: | Compounds of the formulae Ia, Ib and Ic, wherein Z is CH, Y is N, X is C-CH₃, R³ is OCH₂F and R¹ and R² are as defined in one row of table A; |
| Tabelle A357: | Compounds of the formulae Ia, Ib and Ic, wherein Z is CH, Y is N, X is C-CH₃, R³ is OCHCl₂ and R¹ and R² are as defined in one row of table A; |
| Tabelle A358: | Compounds of the formulae Ia, Ib and Ic, wherein Z is CH, Y is N, X is C-CH₃, R³ is OCH₂Cl and R¹ and R² are as defined in one row of table A; |
| Tabelle A359: | Compounds of the formulae Ia, Ib and Ic, wherein Z is CH, Y is N, X is C-CH₃, R³ is OCH₂-OCH₃ and R¹ and R² are as defined in one row of table A; |
| Tabelle A360: | Compounds of the formulae Ia, Ib and Ic, wherein Z is CH, Y is N, X is C-CH₃, R³ is OCH₂CH₃ and R¹ and R² are as defined in one row of table A; |
| Tabelle A361: | Compounds of the formulae Ia, Ib and Ic, wherein Z is CH, Y is N, X is C-CF₃, R³ is CH₃ and R¹ and R² are as defined in one row of table A; |
| Tabelle A362: | Compounds of the formulae Ia, Ib and Ic, wherein Z is CH, Y is N, X is C-CF₃, R³ is C₂H₅ and R¹ and R² are as defined in one row of table A; |
| Tabelle A363: | Compounds of the formulae Ia, Ib and Ic, wherein Z is CH, Y is N, X is C-CF₃, R³ is CF₃ and R¹ and R² are as defined intone row of table A; |
| Tabelle A364: | Compounds of the formulae Ia, Ib and Ic, wherein Z is CH, Y is N, X is C-CF₃, R³ is CCl₃ and R¹ and R² are as defined in one row of table A; |
| Tabelle A365: | Compounds of the formulae Ia, Ib and Ic, wherein Z is CH, Y is N, X is C-CF₃, R³ is F and R¹ and R² are as defined in one row of table A; |
| Tabelle A366: | Compounds of the formulae Ia, Ib and Ic, wherein Z is CH, Y is N, X is C-CF₃, R³ is Cl and R¹ and R² are as defined in one row of table A; |
| Tabelle A367: | Compounds of the formulae Ia, Ib and Ic, wherein Z is CH, Y is N, X is C-CF₃, R³ is Br and R¹ and R² are as defined in one row of table A; |
| Tabelle A368: | Compounds of the formulae Ia, Ib and Ic, wherein Z is CH, Y is N, X is C-CF₃, R³ is I and R¹ and R² are as defined in one row of table A; |
| Tabelle A369: | Compounds of the formulae Ia, Ib and Ic, wherein Z is CH, Y is N, X is C-CF₃, R³ is OCH₃ and R¹ and R² are as defined in one row of table A; |
| Tabelle A370: | Compounds of the formulae Ia, Ib and Ic, wherein Z is CH, Y is N, X is C-CF₃, R³ is OCF₃ and R¹ and R² are as defined in one row of table A; |
| Tabelle A371: | Compounds of the formulae Ia, Ib and Ic, wherein Z is CH, Y is N, X is C-CF₃, R³ is OCCl₃ and R¹ and R² are as defined in one row of table A; |
| Tabelle A372: | Compounds of the formulae Ia, Ib and Ic, wherein Z is CH, Y is N, X is C-CF₃, R³ is OCHF₂ and R¹ and R² are as defined in one row of table A; |
| Tabelle A373: | Compounds of the formulae Ia, Ib and Ic, wherein Z is CH, Y is N, X is C-CF₃, R³ is OCClF₂ and R¹ and R² are as defined in one row of table A;. |
| Tabelle A374: | Compounds of the formulae Ia, Ib and Ic, wherein Z is CH, Y is N, X is C-CF₃, _{.}R³ is OCH₂F and R¹ and R² are as defined in one row of table A; |
| Tabelle A375: | Compounds of the formulae Ia, Ib and Ic, wherein Z is CH, Y is N, X is C-CF₃, R³ is OCHCl₂ and R¹ and R² are as defined in one row of table A; |
| Tabelle A376: | Compounds of the formulae Ia, Ib and Ic, wherein Z is CH, Y is N, X is C-CF₃, R³ is OCH₂Cl and R¹ and R² are as defined in one row of table A; |
| Tabelle A377: | Compounds of the formulae Ia, Ib and Ic, wherein Z is CH, Y is N, X is C-CF₃, R³ is OCH₂-OCH₃ and R¹ and R² are as defined in one row of table A; |
| Tabelle A378: | Compounds of the formulae Ia, Ib and Ic, wherein Z is CH, Y is N, X is C-CF₃, R³ is OCH₂CH₃ and R¹ and R² are as defined in one row of table A; |
| Tabelle A379: | Compounds of the formulae Ia, Ib and Ic, wherein Z is CH, Y is N, X is C-OCH₃, R³ is CH₃ and R and R² are as defined in one row of table A; |
| Tabelle A380: | Compounds of the formulae Ia, Ib and Ic, wherein Z is CH, Y is N, X is C-OCH₃, R³ is C₂H₅ and R¹ and R² are as defined in one row of table A; |
| Tabelle A381: | Compounds of the formulae Ia, Ib and Ic, wherein Z is CH, Y is N, X is C-OCH₃, R³ is CF₃ and R¹ and R² are as defined in one row of table A; |
| Tabelle A382: | Compounds of the formulae Ia, Ib and Ic, wherein Z is CH, Y is N, X is C-OCH₃, R³ is CCl₃ and R¹ and R² are as defined in one row of table A; |
| Tabelle A383: | Compounds of the formulae Ia, Ib and Ic, wherein Z is CH, Y is N, X is C-OCH₃, R³ is F and R¹ and R² are as defined in one row of table A; |
| Tabelle A384: | Compounds of the formulae Ia, Ib and Ic, wherein Z is CH, Y is N, X is C-OCH₃, R³ is Cl and R¹ and R² are as defined in one row of table A; |
| Tabelle A385: | Compounds of the formulae Ia, Ib and Ic, wherein Z is CH, Y is N, X is C-OCH₃, R³ is Br and R¹ and R² are as defined in one row of table A; |
| Tabelle A386: | Compounds of the formulae Ia, Ib and Ic, wherein Z is CH, Y is N, X is C-OCH₃, R³ is I and R¹ and R² are as defined in one row of table A; |
| Tabelle A387: | Compounds of the formulae Ia, Ib and Ic, wherein Z is CH, Y is N, X is C-OCH₃, R³ is OCH₃ and R¹ and R² are as defined in one row of table A; |
| Tabelle A388: | Compounds of the formulae Ia, Ib and Ic, wherein Z is CH, Y is N, X is C-OCH₃, R³ is OCF₃ and R¹ and R² are as defined in one row of table A; |
| Tabelle A389: | Compounds of the formulae Ia, Ib and Ic, wherein Z is CH, Y is N, X is C-OCH₃, R³ is OCCl₃ and R¹ and R² are as defined in one row of table A; |
| Tabelle A390: | Compounds of the formulae Ia, Ib and Ic, wherein Z is CH, Y is N, X is C-OCH₃, R³ is OCHF₂ and R¹ and R² are as defined in one row of table A; |
| Tabelle A391: | Compounds of the formulae Ia, Ib and Ic, wherein Z is CH, Y is N, X is C-OCH₃, R³ is OCClF₂ and R¹ and R² are as defined in one row of table A; |
| Tabelle A392: | Compounds of the formulae Ia, Ib and Ic, wherein Z is CH, Y is N, X is C-OCH₃, R³ is OCH₂F and R¹ and R² are as defined in one row of table A; |
| Tabelle A393: | Compounds of the formulae Ia, Ib and Ic, wherein Z is CH, Y is N, X is C-OCH₃, R³ is OCHCl₂ and R¹ and R² are as defined in one row of table A; |
| Tabelle A394: | Compounds of the formulae Ia, Ib and Ic, wherein Z is CH, Y is N, X is C-OCH₃, R³ is OCH₂Cl and R¹ and R² are as defined in one row of table A; |
| Tabelle A395: | Compounds of the formulae Ia, Ib and Ic, wherein Z is CH, Y is N, X is C-OCH₃, R³ is OCH₂-OCH₃ and R¹ and R² are as defined in one row of table A; |
| Tabelle A396: | Compounds of the formulae Ia, Ib and Ic, wherein Z is CH, Y is N, X is C-OCH₃, R³ is OCH₂CH₃ and R¹ and R² are as defined in one row of table A; |
| Tabelle A397: | Compounds of thee formulae Ia, Ib and Ic, wherein Z is CH, Y is N, X is C-CCl₃, R³ is CH₃ and R¹ and R² are as defined in one row of table A; |
| Tabelle A398: | Compounds of the formulae Ia, Ib and Ic, wherein Z is CH, Y is N, X is C-CCl₃, R³ is C₂H₅ and R¹ and R² are as defined in one row of table A; |
| Tabelle A399: | Compounds of the formulae Ia, Ib and Ic, wherein Z is CH, Y is N, X is C-CCl₃, R³ is CF₃ and R¹ and R² are as defined in one row of table A; |
| Tabelle A400: | Compounds of the formulae Ia, Ib and Ic, wherein Z is CH, Y is N, X is C-CCl₃, R³ is CCl₃ and R¹ and R² are as defined in one row of table A; |
| Tabelle A401: | Compounds of the formulae Ia, Ib and Ic, wherein Z is CH, Y is N, X is C-CCl₃, R³ is F and R¹ and R² are as defined in one row of table A; |
| Tabelle A402: | Compounds of the formulae Ia, Ib and Ic, wherein Z is CH, Y is N, X is C-CCl₃, R³ is Cl and R¹ and R² are as defined in one row of table A; |
| Tabelle A403: | Compounds of the formulae Ia, Ib and lc, wherein Z is CH, Y is N, X is C-CCl₃, R³ is Br and R¹ and R² are as defined in one row of table A; |
| Tabelle A404: | Compounds of the formulae Ia, Ib and Ic, wherein Z is CH, Y is N, X is C-CCl₃, R³ is I and R¹ and R² are as defined in one row of table A; |
| Tabelle A405: | Compounds of the formulae Ia, Ib and Ic, wherein Z is CH, Y is N, X is C-CCl₃, R³ is OCH₃ and R¹ and R² are as defined in one row of table A; |
| Tabelle A406: | Compounds of the formulae Ia, Ib and Ic, wherein Z is CH, Y is N, X is C-CCl₃, R³ is OCF₃ and R¹ and R² are as defined in one row of table A; |
| Tabelle A407: | Compounds of the formulae Ia, Ib and Ic, wherein Z is CH, Y is N, X is C-CCl₃, R³ is OCCl₃ and R¹ and R² are as defined in one row of table A; |
| Tabelle A408: | Compounds of the formulae Ia, Ib and Ic, wherein Z is CH, Y is N, X is C-CCl₃, R³ is OCHF₂ and R¹ and R² are as defined in one row of table A; |
| Tabelle A409: | Compounds of the formulae Ia, Ib and Ic, wherein Z is CH, Y is N, X is C-CCl₃, R³ is OCClF₂ and R¹ and R² are as defined in one row of table A; |
| Tabelle A410: | Compounds of the formulae Ia, Ib and Ic, wherein Z is CH, Y is N, X is C-CCl₃, R³ is OCH₂F and R¹ and R² are as defined in one row of table A; |
| Tabelle A411: | Compounds of the formulae Ia, Ib and Ic, wherein Z is CH, Y is N, X is C-CCl₃, R³ is OCHCl₂ and R¹ and R² are as defined in one row of table A; |
| Tabelle A412: | Compounds of the formulae Ia, Ib and Ic, wherein Z is CH, Y is N, X is C-CCl₃, R³ is OCH₂Cl and R¹ and R² are as defined in one row of table A; |
| Tabelle A413: | Compounds of the formulae Ia, Ib and Ic, wherein Z is CH, Y is N, X is C-CCl₃, R³ is OCH₂-OCH₃ and R¹ and R² are as defined in one row of table A; |
| Tabelle A414: | Compounds of the formulae Ia, Ib and Ic, wherein Z is CH, Y is N, X is C-CCl₃, R³ is OCH₂CH₃ and R¹ and R² are as defined in one row of table A; |
| Tabelle A415: | Compounds of the formulae Ia, Ib and Ic, wherein Z is CH, Y is N, X is C-C₂H₅, R³ is CH₃ and R¹ and R² are as defined in one row of table A; |
| Tabelle A416: | Compounds of the formulae Ia, Ib and Ic, wherein Z is CH, Y is N, X is C-C₂H₅, R³ is C₂H₅ and R¹ and R² are as defined in one row of table A; |
| Tabelle A417: | Compounds of the formulae Ia, Ib and Ic, wherein Z is CH, Y is N, X is C-C₂H₅, R³ is CF₃ and R¹ and R² are as defined in one row of table A; |
| Tabelle A418: | Compounds of the formulae Ia, Ib and Ic, wherein Z is CH, Y is N, X is C-C₂H₅, R³ is CCl₃ and R¹ and R² are as defined in one row of table A; |
| Tabelle A419: | Compounds of the formulae Ia, Ib and Ic, wherein Z is CH, Y is N, X is C-C₂H₅, R³ is F and R¹ and R² are as defined in one row of table A; |
| Tabelle A420: | Compounds of the formulae Ia, Ib and Ic, wherein Z is CH, Y is N, X is C-C₂H₅, R³ is Cl and R¹ and R² are as defined in one row of table A; |
| Tabelle A421: | Compounds of the formulae Ia, Ib and Ic, wherein Z is CH, Y is N, X is C-C₂H₅, R³ is Br and R¹ and R² are as defined in one row of table A; |
| Tabelle A422: | Compounds of the formulae Ia, Ib and Ic, wherein Z is CH, Y is N, X is C-C₂H₅, R³ is I and R¹ and R² are as defined in one row of table A; |
| Tabelle A423: | Compounds of the formulae Ia, Ib and Ic, wherein Z is CH, Y is N, X is C-C₂H₅, R³ is OCH₃ and R¹ and R² are as defined in one row of table A; |
| Tabelle A424: | Compounds of the formulae Ia, Ib and Ic, wherein Z is CH, Y is N, X is C-C₂H₅, R³ is OCF₃ and R¹ and R² are as defined in one row of table A; |
| Tabelle A425: | Compounds of the formulae Ia, Ib and Ic, wherein Z is CH, Y is N, X is C-C₂H₅, R³ is OCCl₃ and R¹ and R² are as defined in one row of table A; |
| Tabelle A426: | Compounds of the formulae Ia, Ib and Ic, wherein Z is CH, Y is N, X is C-C₂H₅, R³ is OCHF₂ and R¹ and R² are as defined in one row of table A; |
| Tabelle A427: | Compounds of the formulae Ia, Ib and Ic, wherein Z is CH, Y is N, X is C-C₂H₅, R³ is OCClF₂ and R¹ and R² are as defined in one row of table A; |
| Tabelle A428: | Compounds of the formulae Ia, Ib and Ic, wherein Z is CH, Y is N, X is C-C₂H₅, R³ is OCH₂F and R¹ and R² are as defined in one row of table A; |
| Tabelle A429: | Compounds of the formulae Ia, Ib and Ic, wherein Z is CH, Y is N, X is C-C₂H₅, R³ is OCHCl₂ and R¹ and R² are as defined in one row of table A; |
| Tabelle A430: | Compounds of the formulae Ia, Ib and Ic, wherein Z is CH, Y is N, X is C-C₂H₅, R³ is OCH₂Cl and R¹ and R² are as defined in one row of table A; |
| Tabelle A431: | Compounds of the formulae Ia, Ib and Ic, wherein Z is CH, Y is N, X is C-C₂H₅, R³ is OCH₂-OCH₃ and R¹ and R² are as defined in one row of table A; |
| Tabelle A432: | Compounds of the formulae Ia, Ib and Ic, wherein Z is CH, Y is N, X is C-C₂H₅, R³ is OCH₂CH₃ and R¹ and R² are as defined in one row of table A; |
| Tabelle A433: | Compounds of the formulae Ia, Ib and Ic, wherein Z is CH, Y is N, X is C-OC₂H₅, R³ is CH₃ and R¹ and R² are as defined in one row of table A; |
| Tabelle A434: | Compounds of the formulae Ia, Ib and Ic, wherein Z is CH, Y is N, X is C-OC₂H₅, R³ is C₂H₅ and R¹ and R² are as defined in one row of table A; |
| Tabelle A435: | Compounds of the formulae Ia, Ib and Ic, wherein Z is CH, Y is N, X is C-OC₂H₅, R³ is CF₃ and R¹ and R² are as defined in one row of table A; |
| Tabelle A436: | Compounds of the formulae Ia, Ib and Ic, wherein Z is CH, Y is N, X is C-OC₂H₅, R³ is CCl₃ and R¹ and R² are as defined in one row of table A; |
| Tabelle A437: | Compounds of the formulae Ia, Ib and Ic, wherein Z is CH, Y is N, X is C-OC₂H₅, R³ is F and R¹ and R² are as defined in one row of table A; |
| Tabelle A438: | Compounds of the formulae Ia, Ib and Ic, wherein Z is CH, Y is N, X is C-OC₂H₅, R³ is Cl and R¹ and R² are as defined in one row of table A; |
| Tabelle A439: | Compounds of the formulae Ia, Ib and Ic, wherein Z is CH, Y is N, X is C-OC₂H₅, R³ is Br and R¹ and R² are as defined in one row of table A; |
| Tabelle A440: | Compounds of the formulae la, lb and lc, wherein Z is CH, Y is N, X is C-OC₂H₅, R³ is I and R¹ and R² are as defined in one row of table A; |
| Tabelle A441: | Compounds of the formulae Ia, Ib and Ic, wherein Z is CH, Y is N, X is C-OC₂H₅, R³ is OCH₃ and R¹ and R² are as defined in one row of table A; |
| Tabelle A442: | Compounds of the formulae Ia, Ib and Ic, wherein Z is CH, Y is N, X is C-OC₂H₅, R³ is OCF₃ and R¹ and R² are as defined in one row of table A; |
| Tabelle A443: | Compounds of the formulae Ia, Ib and Ic, wherein Z is CH, Y is N, X is. C-OC₂H₅, R³ is OCCl₃ and R¹ and R² are as defined in one row of table A; |
| Tabelle A444: | Compounds of the formulae Ia, Ib and Ic, wherein Z is CH, Y is N, X is C-OC₂H₅, R³ is OCHF₂ and R¹ and R² are as defined in one row of table A; |
| Tabelle A445: | Compounds of the formulae Ia, Ib and Ic, wherein Z is CH, Y is N, X is C-OC₂H₅, R³ is OCClF₂ and R¹ and R² are as defined in one row of table A; |
| Tabelle A446: | Compounds of the formulae Ia, Ib and Ic, wherein Z is CH, Y is N, X is C-OC₂H₅, R³ is OCH₂F and R¹ and R² are as defined in one row of table A; |
| Tabelle A447: | Compounds of the formulae Ia, Ib and Ic, wherein Z is CH, Y is N, X is C-OC₂H₅, R³ is OCHCl₂ and R¹ and R² are as defined in one row of table A; |
| Tabelle A448: | Compounds of the formulae Ia, Ib and Ic, wherein Z is CH, Y is N, X is C-OC₂H₅, R³ is OCH₂Cl and R¹ and R² are as defined in one row of table A; |
| Tabelle A449: | Compounds of the formulae Ia, Ib and Ic, wherein Z is CH, Y is N, X is C-OC₂H₅, R³ is OCH₂-OCH₃ and R¹ and R² are as defined in one row of table A; |
| Tabelle A450: | Compounds of the formulae Ia, Ib and Ic, wherein Z is CH, Y is N, X is OC-C₂H₅, R³ is OCH₂CH₃ and R¹ and R² are as defined in one row of table A; |
| Tables 451 to 666: | Compounds of the formulae Ia, Ib and Ic, wherein X is C-Cl, R¹ and R² are as defined in one row of table A and wherein Y, Z and R³ correspond to those given in tables 19 to 234. |
| Tables 667 to 882: | Compounds of the formulae la, lb and lc, wherein X is C-Br, R¹ and R² are as defined in one row of table A and wherein Y, Z and R³ correspond to those given in tables 19 to 234. |
| Tables 883 to 1098: | Compounds of the formulae la, lb and lc, wherein X is C-F, R¹ and R² are as defined in one row of table A and wherein Y, Z and R³ correspond to those given in tables 19 to 234. |
| Tables 1099 to 1314: | Compounds of the formulae Ia, Ib and Ic, wherein X is C-I, R¹ and R² are as defined in one row of table A and wherein Y, Z and R³ correspond to those given in tables 19 to 234. |
| Tables 1315 to 1530: | Compounds of the formulae Ia, Ib and Ic, wherein X is C-NH₂, R¹ and R² are as defined in one row of table A and wherein Y, Z and R³ correspond to those given in tables 19 to 234. |
| Tables 1531 to 1746: | Compounds of the formulae Ia, Ib and Ic, wherein X is C-N(CH₃)₂, R¹ and R² are as defined in one row of table A and wherein Y, Z and R³ correspond to those given in tables 19 to 234. |
| Tables 1747 to 1962: | Compounds of the formulae Ia, Ib and Ic, wherein X is C-CH₃, R¹ and R² are as defined in one row of table A and wherein Y, Z and R³ correspond to those given in tables 19 to 234. |
| Tables 1963 to 2178: | Compounds of the formulae la, lb and lc, wherein X is C-C₂H₅, R¹ and R² are as defined in one row of table A and wherein Y, Z and R³ correspond to those given in tables 19 to 234. |
| Tables 2179 to 2394: | Compounds of the formulae la, lb and lc, wherein X is C-OCH₃, R¹ and R² are as,defined in one row of table A and wherein Y, Z and R³ correspond to those given in tables 19 to 234. |
| Tables 2395 to 2610: | Compounds of the formulae Ia, Ib and Ic, wherein X is C-OC₂H₅, R¹ and R² are as defined in one row of table A and wherein Y, Z and R³ correspond to those given in tables 19 to 234. |
| Tables 2611 to 2826: | Compounds of the formulae Ia, Ib and Ic, wherein X is C-CF₃, R¹ and R² are as defined in one row of table A and wherein Y, Z and R³ correspond to those given in tables 19 to 234. |
| Tables 2827 to 3042: | Compounds of the formulae Ia, Ib and Ic, wherein X is C-CCl₃, R¹ and R² are as defined in one row of table A and wherein Y, Z and R³ correspond to those given in tables 19 to 234. |
| Tables 3043 to 3258: | Compounds of the formulae Ia, Ib and Ic, wherein Z is C-Cl, R¹ and R² are as defined in one row of table A and wherein X, Y and R³ correspond to those given in tables 235 to 450. |
| Tables 3259 to 3474: | Compounds of the formulae Ia, Ib and Ic, wherein Z is Br, R¹ and R² are as defined in one row of table A and wherein X, Y and R³ correspond to those given in tables 235 to 450. |
| Tables 3475 to 3690: | Compounds of the formulae Ia, Ib and Ic, wherein Z is C-F, R¹ and R² are as defined in one row of table A and wherein X, Y and R³ correspond to those given in tables 235 to 450. |
| Tables 3691 to 3906: | Compounds of the formulae Ia, Ib and Ic, wherein Z is C-I, R¹ and R² are as defined in one row of table A and wherein X, Y and R³ correspond to those given in tables 235 to 450. |
| Tables 3907 to 4122: | Compounds of the formulae Ia, Ib and Ic, wherein Z is C-NH₂, R¹ and R² are as defined in one row of table A and wherein X, Y and R³ correspond to those given in tables 235 to 450. |
| Tables 4123 to 4338: | Compounds of the formulae Ia, Ib and Ic, wherein Z is C-N(CH₃)₂, R¹ and R² are as defined in one row of table A and wherein X, Y and R³ correspond to those given in tables 235 to 450. |
| Tables 4339 to 4554: | Compounds of the formulae Ia, Ib and Ic, wherein Z is C-CH₃, R¹ and R² are as defined in one row of table A and wherein X, Y and R³ correspond to those given in tables 235 to 450. |
| Tables 4555 to 4770: | Compounds of the formulae Ia, Ib and Ic, wherein Z is C-C₂H₅, R¹ and R² are as defined in one row of table A and wherein X, Y and R³ correspond to those given in tables 235 to 450. |
| Tables 4771 to 4986: | Compounds of the formulae Ia, Ib and Ic, wherein Z is C-OCH₃, R¹ and R² are as defined in one row of table A and wherein X, Y and R³ correspond to those given in tables 235 to 450. |
| Tables 4987 to 5202: | Compounds of the formulae Ia, Ib and Ic, wherein Z is C-OC₂H₅, R¹ and R² are as defined in one row of table A and wherein X, Y and R³ correspond to those given in tables 235 to 450. |
| Tables 5203 to 5418: | Compounds of the formulae Ia, Ib and Ic, wherein Z is C-CF₃, R¹ and R² are as defined in one row of table A and wherein X, Y and R³ correspond to those given in tables 235 to 450. |
| Tables 5419 to 5634: | Compounds of the formulae Ia, Ib and Ic, wherein Z is C-CCl₃, R¹ and R² are as defined in one row of table A and wherein X, Y and R³ correspond to those given in tables 235 to 450. |
| Tables 5635 to 11268: | Compounds of the formulae Ia, Ib and Ic, wherein Y is N-O, R¹ and R² are as defined in one row of table A and wherein X, Z and R³ correspond to those given in tables 1 to 5634. |

**Table A:**

| | R¹ | R² |
|---|---|---|
| 1. | H | H |
| 2. | CH₃ | H |
| 3. | CH₃CH₂- | H |
| 4. | (CH₃)₂CH- | H |
| 5. | CH₃CH₂CH₂- | H |
| 6. | n-C₄H₉ | H |
| 7. | (CH₃)₃C- | H |
| 8. | (CH₃)₂CH-CH₂- | H |
| 9. | n-C₅H₁₁ | H |
| 10. | (CH₃)₂CH-CH₂-CH₂- | H |
| 11. | (C₂H₅)₂-CH- | H |
| 12. | (CH₃)₃C-CH₂- | H |
| 13. | (CH₃)₃C-CH₂CH₂- | H |
| 14. | C₂H₅CH(CH₃)-CH₂- | H |
| 15. | CH₃-CH₂-C(CH₃)₂- | H |
| 16. | (CH₃)₂CH-CH(CH₃)- | H |
| 17. | (CH₃)₃C-CH(CH₃)- | H |
| 18. | (CH₃)₂CH-CH₂-CH(CH₃)- | H |
| 19. | CH₃-CH₂-C(CH₃)(C₂H₅)- | H |
| 20. | CH₃-CH₂CH₂-C(CH₃)₂- | H |
| 21. | C₂H₅-CH₂-CH(CH₃)₋CH₂- | H |
| 22. | cyclopropyl | H |
| 23. | cyclopropyl-CH₂- | H |
| 24. | cyclopropyl-CH(CH₃)- | H |
| 25. | cyclobutyl | H |
| 26. | cyclopentyl | H |
| 27. | cyclohexyl | H |
| 28. | HC≡C-CH₂₋ | H |
| 29. | HC≡C-CH(CH₃)- | H |
| 30. | HC≡C-C(CH₃)₂- | H |
| 31. | HC≡C-C(CH₃)(C₂H₅)- | H |
| 32. | HC≡C-C(CH₃)(C₃H₇)- | H |
| 33. | CH₂=CH-CH₂- | H |
| 34. | H₂C=CH-CH(CH₃)- | H |
| 35. | H₂C=CH-C(CH₃)₂- | H |
| 36. | H₂C=CH-C(C₂H₅)(CH₃)- | H |
| 37. | C₆H₅-CH₂- | H |
| 38. | 4-(CH₃)₃C-C₆H₄-CH₂- | H |
| 39. | C₆H₅-CH₂- | H |
| 40. | 4-(CH₃)₃C-C₆H₄-CH₂- | H |
| 41. | 4-Cl-C₆H₄-CH₂- | H |
| 42. | 3-(CH₃O)-C₆H₄-CH₂- | H |
| 43. | 4-(CH₃O)-C₆H₄-CH₂- | H |
| 44. | 2-(CH₃O)-C₆H₄-CH₂- | H |
| 45. | 3-Cl-C₆H₄-CH₂- | H |
| 46. | 2-Cl-C₆H₄-CH₂- | H |
| 47. | 4-(F₃C)-C₆H₄-CH₂- | H |
| 48. | NC-CH₂- | H |
| 49. | NC-CH₂-CH₂- | H |
| 50. | NC-CH₂-CH(CH₃)- | H |
| 51. | NC-CH₂-C(CH₃)₂- | H |
| 52. | NC-CH₂-CH₂-CH₂- | H |
| 53. | FH₂C-CH₂₋ | H |
| 54. | CIH₂C-CH₂- | H |
| 55. | BrH₂C-CH₂- | H |
| 56. | FH₂C-CH(CH₃)- | H |
| 57. | CIH₂C-CH(CH₃)- | H |
| 58. | BrH₂C-CH(CH₃)- CH₃ | H |
| 59. | F₂HC-CH₂- | H |
| 60. | F₃C-CH₂- | H |
| 61. | FH₂C-CH₂-CH₂- | H |
| 62. | ClH₂C-CH₂-CH₂- | H |
| 63. | BrH₂C-CH₂-CH₂- | H |
| 64. | F₂HC-CH₂-CHᵣ | H |
| 65. | F₃C-CH₂-CH₂**-** | H |
| 66. | CH₃-O-CH₂-CH₂- | H |
| 67. | CH₃-S-CH₂-CH₂- | H |
| 68. | CH₃-SO₂-CH₂-CH₂- | H |
| 69. | C₂H₅-O-CH₂-CH₂- | H |
| 70. | (CH₃)₂CH-O-CH₂-CH₂- | H |
| 71. | C₂H₅-S-CH₂-CH₂- | H |
| 72. | C₂H₅-SO₂CH₂-CH₂- | H |
| 73. | (CH₃)₂N-CH₂-CH₂- | H |
| 74. | (C₂H₅)₂N-CH₂-CH₂- | H |
| 75. | [(CH₃)₂CH]₂N-CH₂-CH₂- | H |
| 76. | CH₃-O-CH₂-CH(CH₃)- | H |
| 77. | CH₃-S-CH₂-CH(CH₃)- | H |
| 78. | CH₃-SO₂-CH₂-CH(CH₃)- | H |
| 79. | C₂H₅-O-CH₂-CH(CH₃)- | H |
| 80. | C₂H₅-S-CH₂-CH(CH₃)- | H |
| 81. | C₂H₅-SO₂-CH₂-CH(CH₃)- | H |
| 82. | (CH₃)₂N-CH₂-CH(CH₃)- | H |
| 83. | (C₂H₅)₂N-CH₂-CH(CH₃)- | H |
| 84. | [(CH₃)₂CH]₂N-CH₂-CH(CH₃)- | H |
| 85. | CH₃-O-CH(CH₃)-CH₂- | H |
| 86. | CH₃-S-CH(CH₃)-CH₂- | H |
| 87. | CH₃-SO₂-CH(CH₃)-CH₂- | H |
| 88. | C₂H₅-O-CH(CH₃₎-CH₂- | H |
| 89. | C₂H₅-S-CH(CH₃)-CH₂- | H |
| 90. | C₂H₅-SO₂-CH(CH₃)-CH₂- | H |
| 91. | (CH₃)₂N-CH(CH₃)-CH₂- | H |
| 92. | (C₂H₅)₂N-CH(CH₃)-CH₂- | H |
| 93. | [(CH₃)₂CH]₂N-CH(CH₃)-CH₂- | H |
| 94. | CH3-O-CH₂-CH₂-CH₂- | H |
| 95. | CH₃-S-CH₂-CH₂-CH₂- | H |
| 96. | CH₃-SO₂-CH₂-CH₂-CH₂- | H |
| 97. | C₂H₅-O-CH₂-CH₂-CH₂- | H |
| 98. | C₂H₅-S-CH₂CH₂-CH₂- | H |
| 99. | C₂H₅-SO₂-CH₂-CH₂-CH₂- | H |
| 100. | (CH₃)₂N-CH₂-CH₂-CH₂- | H |
| 101. | (C₂H₅)₂N-CH₂-CH₂-CH₂- | H |
| 102. | CH₃-O-CH₂-C(CH₃)₂- | H |
| 103. | CH₃-S-CH₂₋C(CH₃)- | H |
| 104. | CH₃-SO₂-CH₂-C(CH₃)₂- | H |
| 105. | C₂H₅-O-CH₂-C(CH₃)₂- | H |
| 106. | C₂H₅-S-CH₂-C(CH₃)₂- | H |
| 107. | C₂H₅-SO₂-CH₂-C(CH₃)₂- | H |
| 108. | (CH₃)₂N-CH₂-C(CH₃)₂- | H |
| 109. | (C₂H₅)₂N-CH₂-C(CH₃)₂- | H |
| 110. | [(CH₃)₂CH]₂N-CH₂₋C(CH₃)₂- | H |
| 111. | CI-CH₂-C≡C-CH₂- | H |
| 112. | CH₃-O-C(O)-CH₂ | H |
| 113. | C₂H₅-O-C(O)-CH₂ | H |
| 114. | CH₃-O-C(O)-CH(CH₃)- | H |
| 115. | C₂H₅-O-C(O)-CH(CH₃)- | H |
| 116. | (CH₃O)₂CH-CH₂- | H |
| 117. | (C₂H₅O)₂CH-CH₂- | H |
| 118. | C(=O)CH₃ | H |
| 119. | C(=O)CH₂-CH₃ | H |
| 120. | C(=O)CF₃ | . H |
| 121. | C(=O)CCl₃ | H |
| 122. | C(=O)CH₂-CH₂-CH₃ | H |
| 123. | C(=O)C-(CH₃)₃ | H |
| 124. | C(=O)CH₂-C₆H₅ | H |
| 125. | C(=O)CH₂-CH₂-CH₃ | H |
| 126. | H | CH₃ |
| 127. | CH₃ | CH₃ |
| 128. | CH₃CH₂- | CH₃ |
| 129. | (CH₃)₂CH- | CH₃ |
| 130. | CH₃CH₂CH₂- | CH₃ |
| 131. | n-C₄H₉ | CH₃ |
| 132. | (CH₃)₃C- | CH₃ |
| 133. | (CH₃)₂CH-CH₂- | CH₃ |
| 134. | n-C₅H₁₁ | CH₃ |
| 135. | (CH₃)₂CH-CH₂-CH₂- | CH₃ |
| 136. | (C₂H₅)₂-CH- | CH₃ |
| 137. | (CH₃)₃C-CH₂- | CH₃ |
| 138. | (CH₃)₃C-CH₂-CH₂- | CH₃ |
| 139. | C₂H₅CH(CH₃)-CH₂- | CH₃ |
| 140. | CH₃-CH₂-C(CH₃)₂- | CH₃ |
| 141. | (CH₃)₂CH-CH(CH₃)- | CH₃ |
| 142. | (CH₃)₃C-CH(CH₃)- | CH₃ |
| 143. | (CH₃)₂CH-CH₂-CH(CH₃)- | CH₃ |
| 144. | CH₃-CH₂-C(CH₃)(C₂H₅)- | CH₃ |
| 145. | CH₃-CH₂-CH₂-C(CH₃)₂- | CH₃ |
| 146. | C₂H₅-CH₂-CH(CH₃)-CH₂ | CH₃ |
| 147. | cyclopropyl | CH₃ |
| 148. | cyclopropyl-CH₂- | CH₃ |
| 149. | cyclopropyl-CH(CH₃)- | CH₃ |
| 150. | cyclobutyl | CH₃ |
| 151. | cyclopentyl | CH₃ |
| 152. | cyclohexyl | CH₃ |
| 153. | HC≡C-CH₂- | CH₃ |
| 154. | HC≡C-CH(CH₃)- | CH₃ |
| 155. | HC≡C-C(CH₃)₂- | CH₃ |
| 156. | HC≡C-C(CH₃)(C₂H₅)- | CH₃ |
| 157. | HC≡C-C(CH₃)(C₃H₇)- | CH₃ |
| 158. | CH₂=CH-CH₂- | CH₃ |
| 159. | H₂C=CH-CH(CH₃)- | CH₃ |
| 160. | H₂C=CH-C(CH₃)₂- | CH₃ |
| 161. | H₂C=CH-C(C₂H₅)(CH₃)- | CH₃ |
| 162. | C₆H₅-CH₂- | CH₃ |
| 163. | 4-(CH₃)₃C-C₆H₄-CH₂- | CH₃ |
| 164. | C₆H₅-CH₂- | CH₃ |
| 165. | 4-(CH₃)₃C-C₆H₄-CH₂- | CH₃ |
| 166. | 4-Cl-C₆H₄-CH₂- | CH₃ |
| 167. | 3-(CH₃O)-C₆H₄-CH₂- | CH₃ |
| 168. | 4-(CH₃O)-C₆H₄-CH₂- | CH₃ |
| 169. | 2-(CH₃O)-C₆H₄-CH₂- | CH₃ |
| 170. | 3-Cl-C₆H₄-CH₂- | CH₃ |
| 171. | 2-Cl-C₆H₄-CH₂- | CH₃ |
| 172. | 4-(F₃C)-C₆H₄-CH₂- | CH₃ |
| 173. | NC-CH₂- | CH₃ |
| 174. | NC-CH₂-CH₂- | CH₃ |
| 175. | NC-CH₂-CH(CH₃)- | CH₃ |
| 176. | NC-CH₂-C(CH₃)₂- | CH₃ |
| 177. | NC-CH₂-CH₂-CH₂- | CH₃ |
| 178. | FH₂C-CH₂- | CH₃ |
| 179. | ClH₂C-CH₂- | CH₃ |
| 180. | BrH₂C-CH₂- | CH₃ |
| 181. | FH₂C-CH(CH₃)- | CH₃ |
| 182. | ClH₂C-CH(CH₃)- | CH₃ |
| 183. | BrH₂C-CH(CH₃)- CH₃ | CH₃ |
| 184. | F₂HC-CH₂- | CH₃ |
| 185. | F₃C-CH₂- | CH₃ |
| 186. | FH₂C-CH₂-CH₂- | CH₃ |
| 187. | ClH₂C-CH₂-CH₂- | CH₃ |
| 188. | BrH₂C-CH₂-CH₂- | CH₃ |
| 189. | F₂HC-CH₂-CH₂- | CH₃ |
| 190. | F₃C-CH₂-CH₂- | CH₃ |
| 191. | CH₃-O-CH₂-CH₂- | CH₃ |
| 192. | CH₃-S-CH₂-CH₂- | CH₃ |
| 193. | CH₃-SO₂-CH₂-CH₂- | CH₃ |
| 194. | C₂H₅-O-CH₂-CH₂- | CH₃ |
| 195. | (CH₃)₂CH-O-CH₂-CH₂- | CH₃ |
| 196. | C₂H₅-S-CH₂-CH₂- | CH₃ |
| 197. | C₂H₅-SO₂-CH₂-CH₂- | CH₃ |
| 198. | (CH₃)₂N-CH₂-CH₂- | CH₃ |
| 199. | (C₂H₅)₂N-CH₂-CH₂- | CH₃ |
| 200. | [(CH₃)₂CH]₂N-CH₂-CH₂- | CH₃ |
| 201. | CH₃-O-CH₂-CH(CH₃)- | CH₃ |
| 202. | CH₃-S-CH₂-CH(CH₃)- | CH₃ |
| 203. | CH₃-SO₂-CH₂-CH(CH₃)- | CH₃ |
| 204. | C₂H₅-O-CH₂-CH(CH₃)- | CH₃ |
| 205. | C₂H₅-S-CH₂-CH(CH₃)- | CH₃ |
| 206. | C₂H₅-SO₂-CH₂-CH(CH₃)- | CH₃ |
| 207. | (CH₃)₂N-CH₂-CH(CH₃)- | CH₃ |
| 208. | (C₂H₅)₂N-CH₂-CH(CH₃)- | CH₃ |
| 209. | [(CH₃)₂CH]₂N-CH₂-CH(CH₃)- | CH₃ |
| 210. | CH₃-O-CH(CH₃)-CH₂- | CH₃ |
| 211. | CH₃-S-CH(CH₃)-CH₂- | CH₃ |
| 212. | CH₃-SO₂-CH(CH₃)-CH₂- | CH₃ |
| 213. | C₂H₅-O-CH(CH₃)-CH₂- | CH₃ |
| 214. | C₂H₅-S-CH(CH₃)-CH₂- | CH₃ |
| 215. | C₂H₅-SO₂-CH(CH₃)-CH₂- | CH₃ |
| 216. | (CH₃)₂N-CH(CH₃)-CH₂- | CH₃ |
| 217. | (C₂H₅)₂N-CH(CH₃)-CH₂- | CH₃ |
| 218. | [(CH₃)₂CH]₂N-CH(CH₃)-CH₂- | CH₃ |
| 219. | CH₃-O-CH₂-CH₂-CH₂- | CH₃ |
| 220. | CH₃-S-CH₂-CH₂-CH₂- | CH₃ |
| 221. | CH₃-SO₂-CH₂-CH₂-CH₂- | CH₃ |
| 222. | C₂H₅-O-CH₂-CH₂-CH₂- | CH₃ |
| 223. | C₂H₅-S-CH₂-CH₂-CH₂- | CH₃ |
| 224. | C₂H₅-SO₂-CH₂-CH₂-CH₂- | CH₃ |
| 225. | (CH₃)₂N-CH₂-CH₂-CH₂- | CH₃ |
| 226. | (C₂H₅)₂N-CH₂CH₂CH₂- | CH₃ |
| 227. | CH₃-O-CH₂-C(CH₃)₂- | CH₃ |
| 228. | CH₃-S-CH₂-C(CH₃)₂- | CH₃ |
| 229. | CH₃-SO₂-CH₂-C(CH₃)₂- | CH₃ |
| 230. | C₂H₅-O-CH₂-C(CH₃)₂- | CH₃ |
| 231. | C₂H₅-S-CH₂-C(CH₃)₂- | CH₃ |
| 232. | C₂H₅-SO₂-CH₂-C(CH₃)₂- | CH₃ |
| 233. | (CH₃)₂N-CH₂-C(CH₃)₂- | CH₃ |
| 234. | (C₂H₅)₂N-CH₂-C(CH₃)₂- | CH₃ |
| 235. | [(CH₃)₂CH]₂N-CH₂-C(CH₃)₂- | CH₃ |
| 236. | Cl-CH₂-C≡C-CH₂- | CH₃ |
| 237. | CH₃-O-C(O)-CH₂ | CH₃ |
| 238. | C₂H₅-O-C(O)-CH₂ | CH₃ |
| 239. | CH₃-O-C(O)-CH(CH₃)- | CH₃ |
| 240. | C₂H₅-O-C(O)-CH(CH₃)- | CH₃ |
| 241. | (CH₃O)₂CH-CH₂- | CH₃ |
| 242. | (C₂H₅O)₂CH-CH₂- | CH₃ |
| 243. | C(=O)CH₃ | CH₃ |
| 244. | C(=O)CH₂-CH₃ | CH₃ |
| 245. | C(=O)CF₃ | CH₃ |
| 246. | C(=O)CCl₃ | CH₃ |
| 247. | C(=O)CH₂-CH₂-CH₃ | CH₃ |
| 248. | C(=O)C-(CH₃)₃ | CH₃ |
| 249. | C(=O)CH₂-C₆H₅ | CH₃ |
| 250. | C(=O)CH₂-CH₂-CH₃ | CH₃ |
| 251. | H | C₂H₅ |
| 252. | CH₃ | C₂H₅ |
| 253. | CH₃CH₂₋ | C₂H₅ |
| 254. | (CH₃)₂CH- | C₂H₅ |
| 255. | CH₃CH₂CH₂- | C₂H₅ |
| 256. | n-C₄H₉ | C₂H₅ |
| 257. | (CH₃)₃C- | C₂H₅ |
| 258. | (CH₃)₂CH-CH₂- | C₂H₅ |
| 259. | n-C₅H₁₁ | C₂H₅ |
| 260. | (CH₃)₂CH-CH₂-CH₂- | C₂H₅ |
| 261. | (C₂H₅)₂-CH- | C₂H₅ |
| 262. | (CH₃)₃C-CH₂- | C₂H₅ |
| 263. | (CH₃)₃C-CH₂-CH₂- | C₂H₅ |
| 264. | C₂H₅CH(CH₃)-CH₂- | C₂H₅ |
| 265. | CH₃-CH₂-C(CH₃)₂- | C₂H₅ |
| 266. | (CH₃)₂CH-CH(CH₃)- | C₂H₅ |
| 267. | (CH₃)₃C-CH(CH₃)- | C₂H₅ |
| 268. | (CH₃)₂CH-CH₂-CH(CH₃)- | C₂H₅ |
| 269. | CH₃-CH₂-C(CH₃)(C₂H₅)- | C₂H₅ |
| 270. | CH₃-CH₂-CH₂-C(CH₃)₂- | C₂H₅ |
| 271. | C₂H₅-CH₂-CH(CH₃)-CH₂- | C₂H₅ |
| 272. | cyclopropyl | C₂H₅ |
| 273. | cyclopropyl-CH₂- | C₂H₅ |
| 274. | cyclopropyl-CH(CH₃)- | C₂H₅ |
| 275. | cyclobutyl | C₂H₅ |
| 276. | cyclopentyl | C₂H₅ |
| 277. | cyclohexyl | C₂H₅ |
| 278. | HC≡C-CH₂- | C₂H₅ |
| 279. | HC≡C-CH(CH₃)- | C₂H₅ |
| 280. | HC≡C-C(CH₃)₂- | C₂H₅ |
| 281. | HC≡C-C(CH₃)(C₂H₅)- | C₂H₅ |
| 282. | HC≡C-C(CH₃)(C₃H₇)- | C₂H₅ |
| 283. | CH₂=CH-CH₂- | C₂H₅ |
| 284. | H₂C=CH-CH(CH₃)- | C₂H₅ |
| 285. | H₂C=CH-C(CH₃)₂- | C₂H₅ |
| 286. | H₂C=CH-C(C₂H₅)(CH₃)- | C₂H₅ |
| 287. | C₆H₅-CH₂- | C₂H₅ |
| 288. | 4-(CH₃)₃C-C₆H₄-CH₂- | C₂H₅ |
| 289. | C₆H₅-CH₂- | C₂H₅ |
| 290. | 4-(CH₃)₃C-C₆H₄-CH₂₋ | C₂H₅ |
| 291. | 4-Cl-C₆H₄-CH₂- | C₂H₅ |
| 292. | 3-(CH₃O)-C₆H₄-CH₂- | C₂H₅ |
| 293. | 4-(CH₃O)-C₆H₄-CH₂- | C₂H₅ |
| 294. | 2-(CH₃O)-C₆H₄-CH₂- | C₂H₅ |
| 295. | 3-Cl-C₆H₄-CH₂- | C₂H₅ |
| 296. | 2-Cl-C₆H₄-CH₂- | C₂H₅ |
| 297. | 4-(F₃C)-C₆H₄-CH₂- | C₂H₅ |
| 298. | NC-CH₂- | C₂H₅ |
| 299. | NC-CH₂-CH₂- | C₂H₅ |
| 300. | NC-CH₂-CH(CH₃)- | C₂H₅ |
| 301. | NC-CH₂-C(CH₃)₂- | C₂H₅ |
| 302. | NC-CH₂-CH₂-CH₂- | C₂H₅ |
| 303. | FH₂C-CH₂- | C₂H₅ |
| 304. | CIH₂C-CH₂- | C₂H₅ |
| 305. | BrH₂C-CH₂- | C₂H₅ |
| 306. | FH₂C-CH(CH₃)- | C₂H₅ |
| 307. | CIH₂C-CH(CH₃)- | C₂H₅ |
| 308. | BrH₂C-CH(CH₃)- CH₃ | C₂H₅ |
| 309. | F₂HC-CH₂- | C₂H₅ |
| 310. | F₃C-CH₂- | C₂H₅ |
| 311. | FH₂C-CH₂-CH₂- | C₂H₅ |
| 312. | CIH₂C-CH₂-CH₂- | C₂H₅ |
| 313. | BrH₂C-CH₂-CH₂- | C₂H₅ |
| 314. | F₂HC-CH₂-CH₂- | C₂H₅ |
| 315. | F₃C-CH₂-CH₂- | C₂H₅ |
| 316. | CH₃-O-CH₂-CH₂- | C₂H₅ |
| 317. | CH₃-S-CH₂-CH₂- | C₂H₆ |
| 318. | CH₃-SO₂-CH₂-CH₂- | C₂H₅ |
| 319. | C₂H₅-O-CH₂-CH₂- | C₂H₅ |
| 320. | (CH₃)₂CH-O-CH₂-CH₂- | C₂H₅ |
| 321. | C₂H₅-S-CH₂-CH₂- | C₂H₅ |
| 322. | C₂H₅-SO₂-CH₂-CH₂- | C₂H₅ |
| 323. | (CH₃)₂N-CH₂-CH₂- | C₂H₅ |
| 324. | (C₂H₅)₂N-CH₂-CH₂- | C₂H₅ |
| 325. | [(CH₃)₂CH]₂N-CH₂-CH₂- | C₂H₅ |
| 326. | CH₃-O-CH₂-CH(CH₃)- | C₂H₅ |
| 327. | CH₃-S-CH₂-CH(CH₃)- | C₂H₅ |
| 328. | CH₃-SO₂-CH₂-CH(CH₃)- | C₂H₅ |
| 329. | C₂H₅-O-CH₂-CH(CH₃)- | C₂H₅ |
| 330. | C₂H₅-S-CH₂-CH(CH₃)- | C₂H₅ |
| 331. | C₂H₅-SO₂-CH₂-CH(CH₃)- | C₂H₅ |
| 332. | (CH₃)₂N-CH₂-CH(CH₃)- | C₂H₅ |
| 333. | (C₂H₅)₂N-CH₂-CH(CH₃)- | C₂H₅ |
| 334. | [(CH₃)₂CH]₂N-CH₂-CH(CH₃)- | C₂H₅ |
| 335. | CH₃-O-CH(CH₃)-CH₂- | C₂H₅ |
| 336. | CH₃-S-CH(CH₃)-CH₂- | C₂H₅ |
| 337. | CH₃-SO₂-CH(CH₃)-CH₂- | C₂H₅ |
| 338. | C₂H₅-O-CH(CH₃)-CH₂- | C₂H₅ |
| 339. | C₂H₅S-CH(CH₃)-CH₂- | C₂H₅ |
| 340. | C₂H₅-SO₂-CH(CH₃)-CH₂- | C₂H₅ |
| 341. | (CH₃)₂N-CH(CH₃)-CH₂- | C₂H₅ |
| 342. | (C₂H₅)₂N-CH(CH₃)-CH₂- | C₂H₅ |
| 343. | [(CH₃)₂CH]₂N-CH(CH₃)-CH₂- | C₂H₅ |
| 344. | CH₃-O-CH₂-CH₂-CH₂- | C₂H₅ |
| 345. | CH₃-S-CH₂-CH₂-CH₂- | C₂H₅ |
| 346. | CH₃-SO₂-CH₂-CH₂-CH₂- | C₂H₅ |
| 347. | C₂H₅-O-CH₂-CH₂-CH₂- | C₂H₅ |
| 348. | C₂H₅-S-CH₂-CH₂-CH₂- | C₂H₅ |
| 349. | C₂H₅-SO₂-CH₂-CH₂-CH₂- | C₂H₅ |
| 350. | (CH₃)₂N-CH₂-CH₂-CH₂- | C₂H₅ |
| 351. | (C₂H₅)₂N-CH₂-CH₂-CH₂- | C₂H₅ |
| 352. | CH₃-O-CH₂-C(CH₃)₂- | C₂H₅ |
| 353. | CH₃-S-CH₂-C(CH₃)₂- | C₂H₅ |
| 354. | CH₃-SO₂-CH₂-C(CH₃)₂- | C₂H₅ |
| 355. | C₂H₅-O-CH₂-C(CH₃)₂- | C₂H₅ |
| 356. | C₂H₅-S-CH₂-C(CH₃)₂- | C₂H₅ |
| 357. | C₂H₅-SO₂-CH₂-C(CH₃)₂- | C₂H₅ |
| 358. | (CH₃)₂N-CH₂-C(CH₃)₂- | C₂H₅ |
| 359. | (C₂H₅)₂N-CH₂-C(CH₃)₂- | C₂H₅ |
| 360. | [(CH₃)₂CH]₂N-CH₂-C(CH₃)₂- | C₂H₅ |
| 361. | Cl-CH₂-C≡C-CH₂- | C₂H₅ |
| 362. | CH₃-O-C(O)-CH₂ | C₂H₅ |
| 363. | C₂H₅-O-C(O)-CH₂ | C₂H₅ |
| 364. | CH₃-O-C(O)-CH(CH₃)- | C₂H₅ |
| 365. | C₂H₅-O-C(O)-CH(CH₃)- | C₂H₅ |
| 366. | (CH₃O)₂CH-CH₂- | C₂H₅ |
| 367. | (C₂H₅O)₂CH-CH₂- | C₂H₅ |
| 368. | C(=O)CH₃ | C₂H₅ |
| 369. | C(=O)CH₂-CH₃ | C₂H₅ |
| 370. | C(=O)CF₃ | C₂H₅ |
| 371. | C(=O)CCl₃ | C₂H₅ |
| 372. | C(=O)CH₂-CH₂-CH₃ | C₂H₅ |
| 373. | C(=O)C-(CH₃)₃ | C₂H₅ |
| 374. | C(=O)CH₂-C₆H₅ | C₂H₅ |
| 375. | C(=O)CH₂-CH₂-CH₃ | C₂H₅ |
| 376. | H | C(=O)CH₃ |
| 377. | CH₃ | C(=O)CH₃ |
| 378. | CH₃CH₂- | C(=O)CH₃ |
| 379. | (CH₃)₂CH- | C(=O)CH₃ |
| 380. | CH₃CH₂CH₂- | C(=O)CH₃ |
| 381. | n-C₄H₉ | C(=O)CH₃ |
| 382. | (CH₃)₃C- | C(=O)CH₃ |
| 383. | (CH₃)₂CH-CH₂- | C(=O)CH₃ |
| 384. | n-C₅H₁₁ | C(=O)CH₃ |
| 385. | (CH₃)₂CH-CH₂-CH₂- | C(=O)CH₃ |
| 386. | (C₂H₅)₂-CH- | C(=O)CH₃ |
| 387. | (CH₃)₃C-CH₂- | C(=O)CH₃ |
| 388. | (CH₃)₃C-CH₂-CH₂- | C(=O)CH₃ |
| 389. | C₂H₅CH(CH₃)-CH₂- | C(=O)CH₃ |
| 390. | CH₃-CH₂-C(CH₃)₂- | C(=O)CH₃ |
| 391. | (CH₃)₂CH-CH(CH₃)- | C(=O)CH₃ |
| 392. | (CH₃)₃C-CH(CH₃)- | C(=O)CH₃ |
| 393. | (CH₃)₂CH-CH₂-CH(CH₃)- | C(=O)CH₃ |
| 394. | CH₃-CH₂-C(CH₃)(C₂Hₛ)- | C(=O)CH₃ |
| 395. | CH₃-CH₂-CH₂-C(CH₃)₂- | C(=O)CH₃ |
| 396. | C₂H₅-CH₂-CH(CH₃)-CH₂- | C(=O)CH₃ |
| 397. | cyclopropyl | C(=O)CH₃ |
| 398. | cyclopropyl-CH₂- | C(=O)CH₃ |
| 399. | cyclopropyl-CH(CH₃)- | C(=O)CH₃ |
| 400. | cyclobutyl | C(=O)CH₃ |
| 401. | cyclopentyl | C(=O)CH₃ |
| 402. | cyclohexyl | C(=O)CH₃ |
| 403. | HC≡C-CH₂- | C(=O)CH₃ |
| 404. | HC≡C-CH(CH₃)- | C(=O)CH₃ |
| 405. | HC≡C-C(CH₃)₂- | C(=O)CH₃ |
| 406. | HC≡C-C(CH₃)(C₂H₅)- | C(=O)CH₃ |
| 407. | HC≡C-C(CH₃)(C₃H₇)- | C(=O)CH₃ |
| 408. | CH₂=CH-CH₂- | C(=O)CH₃ |
| 409. | H₂C=CH-CH(CH₃)- | C(=O)CH₃ |
| 410. | H₂C=CH-C(CH₃)₂- | C(=O)CH₃ |
| 411. | H₂C=CH-C(C₂H₅)(CH₃)- | C(=O)CH₃ |
| 412. | C₆H₅-CH₂- | C(=O)CH₃ |
| 413. | 4-(CH₃)₃C-C₆H₄-CH₂- | C(=O)CH₃ |
| 414. | C₆H₅-CH₂- | C(=O)CH₃ |
| 415. | 4-(CH₃)₃C-C₆H₄-CH₂- | C(=O)CH₃ |
| 416. | 4-Cl-C₆H₄-CH₂- | C(=O)CH₃ |
| 417. | 3-(CH₃O)-C₆H₄-CH₂- | C(=O)CH₃ |
| 418. | 4-(CH₃O)-C₆H₄-CH₂- | C(=O)CH₃ |
| 419. | 2-(CH₃O)=C₆H₄-CH₂- | C(=O)CH₃ |
| 420. | 3-Cl-C₆H₄-CH₂- | C(=O)CH₃ |
| 421. | 2-Cl-C₆H₄-CH₂- | C(=O)CH₃ |
| 422. | 4-(F₃C)C₆H₄-CH₂- | C(=O)CH₃ |
| 423. | NC-CH₂- | C(=O)CH₃ |
| 424. | NC-CH₂-CH₂- | C(=O)CH₃ |
| 425. | NC-CH₂-CH(CH₃)- | C(=O)CH₃ |
| 426. | NC-CH₂-C(CH₃)₂- | C(=O)CH₃ |
| 427. | NC-CH₂-CH₂-CH₂- | C(=O)CH₃ |
| 428. | FH₂C-CH₂- | C(=O)CH₃ |
| 429. | CIH₂C-CH₂- | C(=O)CH₃ |
| 430. | BrH₂C-CH₂- | C(=O)CH₃ |
| 431. | FH₂C-CH(CH₃)- | C(=O)CH₃ |
| 432. | CIH₂C-CH(CH₃)- | C(=O)CH₃ |
| 433. | BrH₂C-CH(CH₃)-CH₃ | C(=O)CH₃ |
| 434. | F₂HC-CH₂- | C(=O)CH₃ |
| 435. | F₃C-CH₂- | C(=O)CH₃ |
| 436. | FH₂C-CH₂-CH₂- | C(=O)CH₃ |
| 437. | CIH₂C-CH₂-CH₂- | C(=O)CH₃ |
| 438. | BrH₂C-CH₂CH₂- | C(=O)CH₃ |
| 439. | F₂HC-CH₂-CH₂- | C(=O)CH₃ |
| 440. | F₃C-CH₂CH₂- | C(=O)CH₃ |
| 441. | CH₃-O-CH₂-CH₂- | C(=O)CH₃ |
| 442. | CH₃-S-CH₂-CH₂ | C(=O)CH₃ |
| 443. | CH₃-SO₂-CH₂-CH₂- | C(=O)CH₃ |
| 444. | C₂H₅-O-CH₂CH₂- | C(=O)CH₃ |
| 445. | (CH₃)₂CH-O-CH₂-CH₂- | C(=O)CH₃ |
| 446. | C₂H₅-S-CH₂-CH₂- | C(=O)CH₃ |
| 447. | C₂H₅-SO₂-CH₂CH₂- | C(=O)CH₃ |
| 448. | (CH₃)₂N-CH₂CH₂- | C(=O)CH₃ |
| 449. | (C₂H₅)₂N-CH₂-CH₂- | C(=O)CH₃ |
| 450. | [(CH₃)₂CH]₂N-CH₂-CH₂- | C(=O)CH₃ |
| 451. | CH₃-O-CH₂-CH(CH₃)- | C(=O)CH₃ |
| 452. | CH₃-S-CH₂-CH(CH₃)- | C(=O)CH₃ |
| 453. | CH₃-SO₂-CH₂-CH(CH₃)- | C(=O)CH₃ |
| 454. | C₂H₅-O-CH₂CH(CH₃)- | C(=O)CH₃ |
| 455. | C₂H₅-S-CH₂CH(CH₃)- | C(=O)CH₃ |
| 456. | C₂H₅-SO₂-CH₂-CH(CH₃)- | C(=O)CH₃ |
| 457. | (CH₃)₂N-CH₂-CH(CH₃)- | C(=O)CH₃ |
| 458. | (C₂H₅)₂N-CH₂-CH(CH₃)- | C(=O)CH₃ |
| 459. | [(CH₃)₂CH]₂N-CH₂-CH(CH₃)- | C(=O)CH₃ |
| 460. | CH₃-O-CH(CH₃)-CH₂- | C(=O)CH₃ |
| 461. | CH₃-S-CH(CH₃)-CH₂₋ | C(=O)CH₃ |
| 462. | CH₃-SO₂-CH(CH₃)-CH₂- | C(=O)CH₃ |
| 463. | C₂H₅-O-CH(CH₃)-CH₂- | C(=O)CH₃ |
| 464. | C₂H₅-S-CH(CH₃)-CH₂- | C(=O)CH₃ |
| 465. | C₂H₅-SO₂-CH(CH₃)CH₂- | C(=O)CH₃ |
| 466. | (CH₃)₂N-CH(CH₃)CH₂- | C(=O)CH₃ |
| 467. | (C₂H₅)₂N-CH(CH₃)-CH₂- | C(=O)CH₃ |
| 468. | [(CH₃)₂CH]₂N-CH(CH₃)-CH₂- | C(=O)CH₃ |
| 469. | CH₃-O-CH₂CH₂CH₂- | C(=O)CH₃ |
| 470. | CH₃-S-CH₂-CH₂-CH₂- | C(=O)CH₃ |
| 471. | CH₃-SO₂-CH₂-CH₂-CH₂- | C(=O)CH₃ |
| 472. | C₂H₅-O-CH₂-CH₂-CH₂- | C(=O)CH₃ |
| 473. | C₂H₅-S-CH₂-CH₂-CH₂- | C(=O)CH₃ |
| 474. | C₂H₅-SO₂-CH₂-CH₂-CH₂- | C(=O)CH₃ |
| 475. | (CH₃)₂N-CH₂-CH₂-CH₂- | C(=O)CH₃ |
| 476. | (C₂H₅)N₂-CH₂CH₂-CH₂- | C(=O)CH₃ |
| 477. | CH₃-O-CH₂-C(CH₃)₂- | C(=O)CH₃ |
| 478. | CH₃-S-CH₂C(CH₃)- | C(=O)CH₃ |
| 479. | CH₃-SO₂CH₂C(CH₃)₂- | C(=O)CH₃ |
| 480. | C₂H₅-O-CH₂-C(CH₃)₂- | C(=O)CH₃ |
| 481. | C₂H₅-S-CH₂-C(CH₃)₂- | C(=O)CH₃ |
| 482. | C₂H₅-SO₂CH₂C(CH₃)₂- | C(=O)CH₃ |
| 483. | (CH₃)₂N-CH₂-C(CH₃)₂- | C(=O)CH₃ |
| 484. | (C₂H₅)₂N-CH₂-C(CH₃)₂- | C(=O)CH₃ |
| 485. | [(CH₃)₂CH]₂N-CH₂C(CH₃)₂- | C(=O)CH₃ |
| 486. | Cl-CH₂-C≡C-CH₂- | C(=O)CH₃ |
| 487. | CH₃-O-C(O)CH₂ | C(=O)CH₃ |
| 488. | C₂H₅-O-C(O)-CH₂ | C(=O)CH₃ |
| 489. | CH₃-O-C(O)-CH(CH₃)- | C(=O)CH₃ |
| 490. | C₂H₅-O-C(O)-CH(CH₃)- | C(=O)CH₃ |
| 491. | (CH₃O)CH-CH₂- | C(=O)CH₃ |
| 492. | (C₂H₅O)₂CH-CH₂- | C(=O)CH₃ |
| 493. | C(=O)CH₃ | C(=O)CH₃ |
| 494. | C(=O)CH₂-CH₃ | C(=O)CH₃ |
| 495. | C(=O)CF₃ | C(=O)CH₃ |
| 496. | C(=O)CCl₃ | C(=O)CH₃ |
| 497. | C(=O)CH₂-CH₂-CH₃ | C(=O)CH₃ |
| 498. | C(=O)C-(CH₃)₃ | C(=O)CH₃ |
| 499. | C(=O)CH₂-C₆H₅ | C(=O)CH₃ |
| 500. | C(=O)CH₂-CH₂-CH₃ | C(=O)CH₃ |
| 501. | H | C(=O)C₂H₅ |
| 502. | CH₃ | C(=O)C₂H₅ |
| 503. | CH₃CH₂- | C(=O)C₂H₅. |
| 504. | (CH₃)₂CH- | C(=O)C₂H₅ |
| 505. | CH₃CH₂CH₂- | C(=O)C₂H₅ |
| 506. | n-C₄H₉ | C(=O)C₂H₅ |
| 507. | (CH₃)C- | C(=O)C₂H₅ |
| 508. | (CH₃)₂CH-CH₂- | C(=O)C₂H₅ |
| 509. | n-C₅H₁₁ | C(=O)C₂H₅ |
| 510. | (CH₃)₂CH-CH₂-CH₂- | C(=O)C₂H₅ |
| 511. | (C₂H₅)₂-CH- | C(=O)C₂H₅ |
| 512. | (CH₃)₃C-CH₂- | C(=O)C₂H₅ |
| 513. | (CH₃)₃C-CH₂-CH₂- | C(=O)C₂H₅ |
| 514. | C₂H₅CH(CH₃)-CH₂- | C(=O)C₂H₅ |
| 515. | CH₃-CH₂-C(CH₃)₂- | C(=O)C₂H₅ |
| 516. | (CH₃)₂CH-CH(CH₃)- | C(=O)C₂H₅ |
| 517. | (CH₃)₃C-CH(CH₃)- | C(=C)C₂H₅ |
| 518. | (CH₃)₂CH-CH₂-CH(CH₃)- | C(=O)C₂H₅ |
| 519. | CH₃-CH₂-C(CH₃)(C₂H₅)- | C(=O)C₂H₅ |
| 520. | CH₃-CH₂-CH₂-C(CH₃)₂- | C(=O)C₂H₅ |
| 521. | C₂H₅-CH₂-CH(CH₃)-CH₂- | C(=O)C₂H₅ |
| 522. | cyclopropyl | C(=O)C₂H₅ |
| 523. | cyclopropyl-CH₂- | C(=O)C₂H₅ |
| 524. | cyclopropyl-CH(CH₃)- | C(=O)C₂H₅ |
| 525. | cyclobutyl | C(=O)C₂H₅ |
| 526. | cyclopentyl | C(=O)C₂H₅ |
| 527. | cyclohexyl | C(=O)C₂H₅ |
| 528. | HC≡C-CH₂- | C(=O)C₂H₅ |
| 529. | HC≡C-CH(CH₃)- | C(=O)C₂H₅ |
| 530. | HC≡C-C(CH₃)₂- | C(=O)C₂H₅ |
| 531. | HC≡C-C(CH₃)(C₂H₅)- | C(=O)C₂H₅ |
| 532. | HC≡C-C(CH₃)(C₃H₇)- | C(=O)C₂H₅ |
| 533. | CH₂=CH-CH₂- | C(=O)C₂H₅ |
| 534. | H₂C=CH-CH(CH₃)- | C(=O)C₂H₅ |
| 535. | H₂C=CH-C(CH₃)₂- | C(=O)C₂H₅ |
| 536. | H₂C=CH-C(C₂H₅)(CH₃)- | C(=O)C₂H₅ |
| 537. | C₆H₅-CH₂- | C(=O)C₂H₅ |
| 538. | 4-(CH₃)₃C-C₆H₄-CH₂- | C(=O)C₂H₅ |
| 539. | C₆H₅-CH₂- | C(=O)C₂H₅ |
| 540. | 4-(CH₃)₃C-C₆H₄-CH₂- | C(=O)C₂H₅ |
| 541. | 4-Cl-C₆H₄-CH₂- | C(=O)C₂H₅ |
| 542. | 3-(CH₃O)-C₆H₄-CH₂- | C(=O)C₂H₅ |
| 543. | 4-(CH₃O)-C₆H₄-CH₂- | C(=O)C₂H₅ |
| 544. | 2-(CH₃O)-C₆H₄-CH₂- | C(=O)C₂H₅ |
| 545. | 3-Cl-C₆H₄-CH₂- | C(=O)C₂H₅ |
| 546. | 2-Cl-C₆H₄-CH₂- | C(=O)C₂H₅ |
| 547. | 4-(F₃C)-C₆H₄-CH₂- | C(=O)C₂H₅ |
| 548. | NC-CH₂- | C(=O)C₂H₅ |
| 549. | NC-CH₂-CH₂- | C(=O)C₂H₅ |
| 550. | NC-CH₂CH(CH₃)- | C(=O)C₂H₅ |
| 551. | NC-CH₂-C(CH₃)₂- | C(=O)C₂H₅ |
| 552. | NC-CH₂-CH₂-CH₂- | C(=O)C₂H₅ |
| 553. | FH₂C-CH₂- | C(=O)C₂H₅ |
| 554. | ClH₂C-CH₂- | C(=O)C₂H₅ |
| 555. | BrH₂C-CH₂- | C(=O)C₂H₅ |
| 556. | FH₂C-CH(CH₃)- | C(=O)C₂H₅ |
| 557. | CIH₂C-CH(CH₃)- | C(=O)C₂H₅ |
| 558. | BrH₂C-CH(CH₃)- CH₃ | C(=O)C₂H₅ |
| 559. | F₂HC-CH₂- | C(=O)C₂H₅ |
| 560. | F₃C-CH₂- | C(=O)C₂H₅ |
| 561. | FH₂C-CH₂-CH₂- | C(=O)C₂H₅ |
| 562. | CIH₂C-CH₂-CH₂- | C(=O)C₂H₅ |
| 563. | BrH₂C-CH₂CH₂- | C(=O)C₂H₅ |
| 564. | F₂HC-CH₂CH₂- | C(=O)C₂H₅ |
| 565. | F₃C-CH₂-CH₂- | C(=O)C₂H₅ |
| 566. | CH₃-O-CH₂-CH₂- | C(=O)C₂H₅ |
| 567. | CH₃-S-CH₂-CH₂- | C(=O)C₂H₅ |
| 568. | CH₃-SO₂CH₂CH₂- | C(=O)C₂H₅ |
| 569. | C₂H₅-O-CH₂CH₂- | C(=O)C₂H₅ |
| 570. | (CH₃)₂CH-O-CH₂-CH₂- | C(=O)C₂H₅ |
| 571. | C₂H₅-S-CH₂-CH₂- | C(=O)C₂H₅ |
| 572. | C₂H₅-SO₂-CH₂-CH₂- | C(=O)C₂H₅ |
| 573. | (CH₃)₂N-CH₂-CH₂- | C(=O)C₂H₅ |
| 574. | (C₂H₅)₂N-CH₂-CH₂ | C(=O)C₂H₅ |
| 575. | [(CH₃)₂CH]₂N-CH₂-CH₂- | C(=O)C₂H₅ |
| 576. | CH₃-O-CH₂-CH(CH₃)- | C(=O)C₂H₅ |
| 577. | CH₃-S-CH₂-CH(CH₃)- | C(=O)C₂H₅ |
| 578. | CH₃-SO₂-CH₂-CH(CH₃)- | C(=O)C₂H₅ |
| 579. | C₂H₅-O-CH₂-CH(CH₃)- | C(=O)C₂H₅ |
| 580. | C₂H₅-S-CH₂-CH(CH₃)- | C(=O)C₂H₅ |
| 581. | C₂H₅-SO₂-CH₂CH(CH₃)- | C(=O)C₂H₅ |
| 582. | (CH₃)₂N-CH₂-CH(CH₃)- | C(=O)C₂H₅ |
| 583. | (C₂H₅)₂N-CH₂-CH(CH₃)- | C(=O)C₂H₅ |
| 584. | [(CH₃)₂CH]₂N-CH₂-CH(CH₃)- | C(=O)C₂H₅ |
| 585. | CH₃-O-CH(CH₃)-CH₂- | C(=O)C₂H₅ |
| 586. | CH₃-S-CH(CH₃)-CH₂- | C(=O)C₂H₅ |
| 587. | CH₃-SO₂-CH(CH₃)-CH₂- | C(=O)C₂H₅ |
| 588. | C₂H₅-O-CH(CH₃)-CH₂- | C(=O)C₂H₅ |
| 589. | C₂H₅-S-CH(CH₃)-CH₂- | C(=O)C₂H₅ |
| 590. | C₂H₅-SO₂CH(CH₃)-CH₂- | C(=O)C₂H₅ |
| 591. | (CH₃)₂N-CH(CH₃)-CH₂- | C(=O)C₂H₅ |
| 592. | (C₂H₅)₂N-CH(CH₃)-CH₂- | C(=O)C₂H₅ |
| 593. | [(CH₃)₂CH]₂N-CH(CH₃)-CH₂- | C(=O)C₂H₅ |
| 594. | CH₃-O-CH₂-CH₂-CH₂- | C(=O)C₂H₅ |
| 595. | CH₃-S-CH₂-CH₂-CH₂- | C(=O)C₂H₅ |
| 596. | CH₃-SO₂CH₂-CH₂-CH₂- | C(=O)C₂H₅ |
| 597. | C₂H₅-O-CH₂CH₂CH₂- | C(=O)C₂H₅ |
| 598. | C₂H₅-S-CH₂CH₂CH₂- | C(=O)C₂H₅ |
| 599. | C₂H₅-SO₂-CH₂-CH₂-CH₂- | C(=O)C₂H₅ |
| 600. | (CH₃)₂N-CH₂-CH₂-CH₂- | C(=O)C₂H₅ |
| 601. | (C₂H₅)₂N-CH₂CH₂CH₂- | C(=O)C₂H₅ |
| 602. | CH₃-O-CH₂-C(CH₃)₂- | C(=O)C₂H₅ |
| 603. | CH₃-S-CH₂-C(CH₂- | C(=O)C₂H₅ |
| 604. | CH₃-SO₂CH₂C(CH₃)₂- | C(=O)C₂H₅ |
| 605. | C₂H₅-O-CH₂-C(CH₃)₂- | C(=O)C₂H₅ |
| 606. | C₂H₅-S-CH₂-C(CH₃)₂ | C(=O)C₂H₅ |
| 607. | C₂H₅-SO₂-CH₂-C(CH₃)₂- | C(=O)C₂H₅ |
| 608. | (CH₃)₂N-CH₂-C(CH₃)₂- | C(=O)C₂H₅ |
| 609. | (C₂H₅)N-CH₂C(CH₃)₂- | C(=O)C₂H₅ |
| 610. | [(CH₃)₂CH]₂N-CH₂-C(CH₃)₂- | C(=O)C₂H₅ |
| 611. | Cl-CH₂-C=C-CH₂- | C(=O)C₂H₅ |
| 612. | CH₃-O-C(O)-CH₂ | C(=O)C₂H₅ |
| 613. | C₂H₅-O-C(O)-CH₂ | C(=O)C₂H₅ |
| 614. | CH₃-O-C(O)-CH(CH₃)- | C(=O)C₂H₅ |
| 615. | C₂H₅-O-C(O)-CH(CH₃)- | C(=O)C₂H₅ |
| 616. | (CH₃O)₂CH-CH₂- | C(=O)C₂H₅ |
| 617. | (C₂H₅O)₂CH-CH₂- | C(=O)C₂H₅ |
| 618. | C(=O)CH₃ | C(=O)C₂H₅ |
| 619. | C(=O)CH₂-CH₃ | C(=O)C₂H₅ |
| 620. | C(=O)CF₃ | C(=O)C₂H₅ |
| 621. | C(=O)CCl₃ | C(=O)C₂H₅ |
| 622. | C(=O)CH₂-CH₂-CH₃ | C(=O)C₂H₅ |
| 623. | C(=O)C-(CH₃)₃ | C(=O)C₂H₅ |
| 624. | C(=O)CH₂-C₆H₅ | C(=O)C₂H₅ |
| 625. | C(=O)CH₂-CH₂-CH₃ | C(=O)C₂H₅ |
| 626. | H | C(=O)C(CH₃)₃ |
| 627. | CH₃ | C(=O)C(CH₃)₃ |
| 628. | CH₃CH₂- | C(=O)C(CH₃)₂ |
| 629. | (CH₃)₂CH- | C(=O)C(CH₃)₃ |
| 630. | CH₃CH₂CH₂- | C(=O)C(CH₃)₃ |
| 631. | n-C₄H₉ | C(=O)C(CH₃)₃ |
| 632. | (CH₃)₃C- | C(=O)C(CH₃)₃ |
| 633. | (CH₃)₂CH-CH₂- | C(=O)C(CH₃)₃ |
| 634. | n-C₅H₁₁ | C(=O)C(CH₃)₃ |
| 635. | (CH₃)₂CH-CH₂CH₂- | C(=O)C(CH₃)₃ |
| 636. | (C₂H₅)₂-CH- | C(=O)C(CH₃)₃ |
| 637. | (CH₃)₃C-CH₂- | C(=O)C(CH₃)₃ |
| 638. | (CH₃)₃C-CH₂-CH₂- | C(=O)C(CH₃)₃ |
| 639. | C₂H₅CH(CH₃)-CH₂- | C(=O)C(CH₃)₃ |
| 640. | CH₃-CH₂-C(CH₃)₂- | C(=O)C(CH₃)₃ |
| 641. | (CH₃)₂CH-CH(CH₃)- | C(=O)C(CH₃)₃ |
| 642. | (CH₃)₃C-CH(CH₃)- | C(=O)C(CH₃)₃ |
| 643. | (CH₃)₂CH-CH₂-CH(CH₃)- | C(=O)C(CH₃)₃ |
| 644. | CH₃-CH₂C(CH₃)(C₂H₅)- | C(=O)C(CH₃)₃ |
| 645. | CH₃-CH₂CH₂C(CH₃)₂- | C(=O)C(CH₃)₂ |
| 646. | C₂H₅-CH₂-CH(CH₃)-CH₂- | C(=O)C(CH₃)₃ |
| 647. | cyclopropyl | C(=O)C(CH₃)₃ |
| 648. | cyclopropyl-CH₂- | C(=O)C(CH₃)₃ |
| 649. | cyclopropyl-CH(CH₃)- | C(=O)C(CH₃)₃ |
| 650. | cyclobutyl | C(=O)C(CH₃)₃ |
| 651. | cyclopentyl | C(=O)C(CH₃)₃ |
| 652. | cyclohexyl | C(=O)C(CH₃)₃ |
| 653. | HC≡C-CH₂- | C(=O)C(CH₃)₃ |
| 654. | HC≡C-CH(CH₃)- | C(=O)C(CH₃)₃ |
| 655. | HC≡C-C(CH₃)₂- | C(=O)C(CH₃)₃ |
| 656. | HC≡C-C(CH₃)(C₂H₅)- | C(=O)C(CH₃)₃ |
| 657. | HC≡C-C(CH₃)(C₃H₇)- | C(=O)C(CH₃)₃ |
| 658. | CH₂=CH-CH₂- | C(=O)C(CH₃)₃ |
| 659. | H₂C=CH-CH(CH₃)- | C(=O)C(CH₃)₃ |
| 660. | H₂C=CH-C(CH₃)₂₋ | C(=O)C(CH₃)₃ |
| 661. | H₂C=CH-C(C₂H₅)(CH₃)- | C(=O)C(CH₃)₃ |
| 662. | C₆H₅-CH₂- | C(=O)C(CH₃)₃ |
| 663. | 4-(CH₃)₃C-C₆H₄-CH₂- | C(=O)C(CH₃)₃ |
| 664. | C₆H₅-CH₂- | C(=O)C(CH₃)₃ |
| 665. | 4-(CH₃)₃C-C₆H₄-CH₂- | C(=O)C(CH₃)₃ |
| 666. | 4-Cl-C₆H₄-CH₂- | C(=O)C(CH₃)₃ |
| 667. | 3-(CH₃O)-C₆H₄-CH₂- | C(=O)C(CH₃)₃ |
| 668. | 4-(CH₃O)-C₆H₄-CH₂- | C(=O)C(CH3)₃ |
| 669. | 2-(CH₃O)-C₆H₄-CH₂- | C(=O)C(CH₃)₃ |
| 670. | 3-Cl-C₆H₄-CH₂- | C(=O)C(CH₃)₃ |
| 671. | 2-Cl-C₆H₄-CH₂- | C(=O)C(CH₃)₃ |
| 672. | 4-(F₃C)-C₆H₄-CH₂- | C(=O)C(CH₃)₃ |
| 673. | NC-CH₂- | C(=O)C(CH₃)₃ |
| 674. | NC-CH₂-CH₂- | C(=O)C(CH₃)₃ |
| 675. | NC-CH₂-CH(CH₃)- | C(=O)C(CH₃)₃ |
| 676. | NC-CH₂C(CH₃)₂- | C(=O)C(CH₃)₃ |
| 677. | NC-CH₂-CH₂CH₂- | . C(=O)C(CH₃)₂ |
| 678. | FH₂C-CH₂- | C(=O)C(CH₃)₃ |
| 679. | CIH₂C-CH₂- | C(=O)C(CH₃)₃ |
| 680. | BrH₂C-CH₂- | C(=O)C(CH₃)₃ |
| 681. | FH₂C-CH(CH₃)- | C(=O)C(CH₃)₃ |
| 682. | ClH₂C-CH(CH₃)- | C(=O)C(CH₃)₃ |
| 683. | BrH₂C-CH(CH₃)- CH₃ | C(=O)C(CH₃)₃ |
| 684. | F₂HC-CH₂- | C(=O)C(CH₃)₃ |
| 685. | F₃C-CH₂- | C(=O)C(CH₃)₃ |
| 686. | FH₂C-CH₂-CH₂- | C(=O)C(CH₃)₃ |
| 687. | ClH₂C-CH₂-CH₂- | C(=O)C(CH₃)₃ |
| 688. | BrH₂C-CH₂-CH₂- | C(=O)C(CH₃)₃ |
| 689. | F₂HC-CH₂-CH₂- | C(=O)C(CH₃)₂ |
| 690. | F₃C-CH₂-CH- | C(=O)C(CH₃)₃ |
| 691. | CH₃-O-CH₂-CH₂- | C(=O)C(CH₃)₃ |
| 692. | CH₃-S-CH₂-CH₂- | C(=O)C(CH₃)₃ |
| 693. | CH₃-SO₂-CH₂-CH₂- | C(=O)C(CH₃)₃ |
| 694. | C₂H₅-O-CH₂CH₂- | C(=O)C(CH₃)₃ |
| 695. | (CH₃)₂CH-O-CH₂-CH₂- | C(=O)C(CH₃)₃ |
| 696. | C₂H₅-S-CH₂CH₂- | C(=O)C(CH₃)₃ |
| 697. | C₂H₅-SO₂-CH₂-CH₂- | C(=O)C(CH₃)₃ |
| 698. | (CH₃)₂N-CH₂-CH₂- | C(=O)C(CH₃)₃ |
| 699. | (C₂H₅)₂N-CH₂-CH₂- | C(=O)C(CH₃)₃ |
| 700. | [(CH₃)₂CH]₂N-CH₂CH₂- | C(=O)C(CH₃)₃ |
| 701. | CH₃-O-CH₂-CH(CH₃)- | C(=O)C(CH₃)₃ |
| 702. | CH₃-S-CH₂-CH(CH₃)- | C(=O)C(CH₃)₃ |
| 703. | CH₃-SO₂-CH₂-CH(CH₃)- | C(=O)C(CH₃)₃ |
| 704. | C₂H₅-O-CH₂CH(CH₃)- | C(=O)C(CH₃)₃ |
| 705. | C₂H₅-S-CH₂CH(CH₃)- | C(=O)C(CH₃)₃ |
| 706. | C₂H₅-SO₂-CH₂-CH(CH₃)- | C(=O)C(CH₃)₃ |
| 707. | (CH₃)₂N-CH₂-CH(CH₃)- | C(=O)C(CH₃)₃ |
| 708. | (C₂H₅)₂N-CH₂-CH(CH₃)- | C(=O)C(CH₃)₃ |
| 709. | [(CH₃)₂CH]₂N-CH₂-CH(CH₃)- | C(=O)C(CH₃)₃ |
| 710. | CH₃-O-CH(CH₃)-CH₂- | C(=O)C(CH₃)₃ |
| 711. | CH₃-S-CH(CH₃)-CH₂- | C(=O)C(CH₃)₃ |
| 712. | CH₃-SO₂-CH(CH₃)-CH₂- | C(=O)C(CH₃)₃ |
| 713. | C₂H₅-O-CH(CH₃)-CH₂- | C(=O)C(CH₃)₃ |
| 714. | C₂H₅-S-CH(CH₃)-CH₂- | C(=O)C(CH₃)₃ |
| 715. | C₂H₅-SO₂-CH(CH₃)-CH₂- | C(=O)C(CH₃)₃ |
| 716. | (CH₃)₂N-CH(CH₃)-CH₂- | C(=O)C(CH₃)₃ |
| 717. | (C₂H₅)N-CH(CH₃)-CH₂- | C(=O)C(CH₃)₃ |
| 718. | [(CH₃)₂CH]₂N-CH(CH₃)-CH₂- | C(=O)C(CH₃)₃ |
| 719. | CH₃-O-CH₂-CH₂-CH₂- | C(=O)C(CH₃)₃ |
| 720. | CH₃-S-CH₂-CH₂-CH₂- | C(=O)C(CH₃)₃ |
| 721. | CH₃-SO₂-CH₂-CH₂-CH₂- | C(=O)C(CH₃)₃ |
| 722. | C₂H₅-O-CH₂-CH₂-CH₂- | C(=O)C(CH₃)₃ |
| 723. | C₂H₅-S-CH₂-CH₂CH₂- | C(=O)C(CH₃)₃ |
| 724. | C₂H₅-SO₂-CH₂-CH₂-CH₂- | C(=O)C(CH₃)₃ |
| 725. | (CH₃)₂N-CH₂-CH₂CH₂- | C(=O)C(CH₃)₃ |
| 726. | (C₂H₅)₂N-CH₂-CH₂-CH₂- | C(=O)C(CH₃)₃ |
| 727. | CH₃-O-CH₂C(CH₃)₂- | C(=O)C(CH₃)₃ |
| 728. | CH₃-S-CH₂-C(CH₃)₂- | C(=O)C(CH₃)₃ |
| 729. | CH₃-SO₂-CH₂-C(CH₃)₂- | C(=O)C(CH₃)₃ |
| 730. | C₂H₅-O-CH₂C(CH₃)₂- | C(=O)C(CH₃)₃ |
| 731. | C₂H₅-S-CH₂-C(CH₃)₂- | C(=O)C(CH₃)₃ |
| 732. | C₂H₅-SO₂-CH₂-C(CH₃)₂- | C(=O)C(CH₃)₃ |
| 733. | (CH₃)₂N-CH₂C(CH₃)₂- | C(=O)C(CH₃)₃ |
| 734. | (C₂H₅)₂N-CH₂-C(CH₃)₂- | C(=O)C(CH₃)₃ |
| 735. | [(CH₃)₂CH]N-CH₂C(CH₃)₂- | C(=O)C(CH₃)₃ |
| 736. | Cl-CH₂C=C-CH₂- | C(=O)C(CH₃)₃ |
| 737. | CH₃-O-C(O)-CH₂ | C(=O)C(CH₃)₃ |
| 738. | C₂H₅-O-C(O)CH₂ | C(=O)C(CH₃)₃ |
| 739. | CH₃-O-C(O)-CH(CH₃)- | C(=O)C(CH₃)₃ |
| 740. | C₂H₅-O-C(O)-CH(CH₃)- | C(=O)C(CH₃)₃ |
| 741. | (CH₃O)₂CH-CH₂- | C(=O)C(CH₃)₃ |
| 742. | (C₂H₅O)CH-CH₂- | C(=O)C(CH₃)₃ |
| 743. | C(=O)CH₃ | C(=O)C(CH₃)₃ |
| 744. | C(=O)CH₂-CH₃ | C(=O)C(CH₃)₃ |
| 745. | C(=O)CF₃ | C(=O)C(CH₃)₃ |
| 746. | C(=O)CCl₃ | C(=O)C(CH3)₃ |
| 747. | C(=O)CH₂-CH₂-CH₃ | C(=O)C(CH₃)₃ |
| 748. | C(=O)C-(CH₃)₃ | C(=O)C(CH₃)₃ |
| 749. | C(=O)CH₂-C₂H₅ | C(=O)C(CH₃)₃ |
| 750. | C(=O)CH₂-CH₂-CH₃ | C(=O)C(CH₃)₃ |
| 751. | H | C(=O)CF₃ |
| 752. | CH₃ | C(=O)CF₃ |
| 753. | CH₃C₃CH₂- | C(=O)CF₃ |
| 754. | (CH₃)₂CH- | C(=O)CF₃ |
| 755. | CH₃CH₂CH₂- | C(=O)CF₃ |
| 756. | n-C₄H₉ | C(=O)CF₃ |
| 757. | (CH₃)₃C- | C(=O)CF₃ |
| 758. | (CH₃)₂CH-CH₂- | C(=O)CF₃ |
| 759. | n-C₅H₁₁ | C(=O)CF₃ |
| 760. | (CH₃)₂CH-CH₂-CH₂- | C(=O)CF₃ |
| 761. | (C₂H₅)₂-CH- | C(=O)CF₃ |
| 762. | (CH₃)₃C-CH₂- | C(=O)CF₃ |
| 763. | (CH₃)₃C-CH₂CH₂- | C(=O)CF₃ |
| 764. | C₂H₅CH(CH₃)-CH₃ | C(=O)CF₃ |
| 765. | CH₃-CH₂-C(CH₃)₂- | C(=O)CF₃ |
| 766. | (CH₃)₂CH-CH(CH₃) | C(=O)CF₃ |
| 767. | (CH₃)₃C-CH(CH₃)- | C(=O)CF₃ |
| 768. | (CH₃)₂CH-CH₂-CH(CH₃)- | C(=O)CF₃ |
| 769. | CH₃-CH₂-C(CH₃)(C₂H₅)- | C(=O)CF₃ |
| 770. | CH₃-CH₂-CH₂-C(CH₃)₂- | C(=O)CF₃ |
| 771. | C₂H₅-CH₂-CH(CH₃)-CH₂- | C(=O)CF₃ |
| 772. | cyclopropyl | C(=O)CF₃ |
| 773. | cyclopropyl-CH₂- | C(=O)CF₃ |
| 774. | cyclopropyl-CH(CH₃)- | C(=O)CF₃ |
| 775. | cyclobutyl | C(=O)CF₃ |
| 776. | cyclopentyl | C(=O)CF₃ |
| 777. | cyclohexyl | C(=O)CF₃ |
| 778. | HC≡C-CH₂- | C(=O)CF₃ |
| 779. | HC≡C-CH(CH₃)- | C(=O)CF₃ |
| 780. | HC≡C-C(CH₃)₂- | C(=O)CF₃ |
| 781. | HC≡C-C(CH₃)(C₂H₅)- | C(=O)CF₃ |
| 782. | HC≡C-C(CH₃)(C₃H₇)- | C(=O)CF₃ |
| 783. | CH₂=CH-CH₂- | C(=O)CF₃ |
| 784. | H₂C=CH-CH(CH₃)- | C(=O)CF₃ |
| 785. | H₂C=CH-C(CH₃)₂- | C(=O)CF₃ |
| 786. | H₂C=CH-C(C₂H₅)(CH₃)- | C(=O)CF₃ |
| 787. | C₆H₅-CH₂- | C(=O)CF₃ |
| 788. | 4-(CH₃)₃C-C₆H₄-CH₂- | C(=O)CF₃ |
| 789. | C₆H₅-CH₂- | C(=O)CF₃ |
| 790. | 4-(CH₃)₃C-C₆H₄-CH₂- | C(=O)CF₃ |
| 791. | 4-Cl-C₆H₄-CH₂- | C(=O)CF₃ |
| 792. | 3-(CH₃O)-C₆H₄-CH₂- | C(=O)CF₃ |
| 793. | 4-(CH₃O)-C₆H₄-CH₂₋ | C(=O)CF₃ |
| 794. | 2-(CH₃O)-C₆H₄-CH₂- | C(=O)CF₃ |
| 795. | 3-Cl-C₆H₄-CH₂- | C(=O)CF₃ |
| 796. | 2-Cl-C₆H₄-CH₂- | C(=O)CF₃ |
| 797. | 4-(F₃C)-C₆H₄-CH₂- | C(=O)CF₃ |
| 798. | NC-CH₂- | C(=O)CF₃ |
| 799. | NC-CH₂-CH₂- | C(=O)CF₃ |
| 800.. | NC-CH₂-CH(CH₃)- | C(=O)CF₃ |
| 801. | NC-CH₂-C(CH₃)₂- | C(=O)CF₃ |
| 802. | NC-CH₂-CH₂-CH₂- | C(=O)CF₃ |
| 803. | FH₂C-CH₂- | C(=O)CF₃ |
| 804. | ClH₂C-CH₂₋ | C(=O)CF₃ |
| 805. | BrH₂C-CH₂- | C(=O)CF₃ |
| 806. | FH₂C-CH(CH₃)- | C(=O)CF₃ |
| 807. | ClH₂C-CH(CH₃)- | C(=O)CF₃ |
| 808. | BrH₂C-CH(CH₃)- CH₃ | C(=O)CF₃ |
| 809. | F₂HC-CH₂- | C(=O)CF₃ |
| 810. | F₃C-CH₂- | C(=O)CF₃ |
| 811. | FH₂C-CH₂-CH₂- | C(=O)CF₃ |
| 812. | ClH₂C-CH₂-CH₂- | C(=O)CF₃ |
| 813. | BrH₂C-CH₂-CH₂- | C(=O)CF₃ |
| 814. | F₂HC-CH₂-CH₂- | C(=O)CF₃ |
| 815. | F₃C-CH₂-CH₂- | C(=O)CF₃ |
| 816. | CH₃-O-CH₂-CH₂- | C(=O)CF₃ |
| 817. | CH₃-S-CH₂-CH₂- | C(=O)CF₃ |
| 818. | CH₃-SO₂-CH₂-CH₂- | C(=O)CF₃ |
| 819. | C₂H₅-O-CH₂-CH₂- | C(=O)CF₃ |
| 820. | (CH₃)₂CH-O-CH₂-CH₂- | C(=O)CF₃ |
| 821. | C₂H₅-S-CH₂-CH₂- | C(=O)CF₃ |
| 822. | C₂H₅-SO₂-CH₂-CH₂- | C(=O)CF₃ |
| 823. | (CH₃)₂N-CH₂-CH₂- | C(=O)CF₃ |
| 824. | (C₂H₅)₂N-CH₂-CH₂- | C(=O)CF₃ |
| 825. | [(CH₃)₂CH]₂N-CH₂-CH₂- | C(=O)CF₃ |
| 826. | CH₃-O-CH₂-CH(CH₃)- | C(=O)CF₃ |
| 827. | CH₃-S-CH₂-CH(CH₃)- | C(=O)CF₃ |
| 828. | CH₃-SO₂-CH₂-CH(CH₃)- | C(=O)CF₃ |
| 829. | C₂H₅-O-CH₂-CH(CH₃)- | C(=O)CF₃ |
| 830. | C₂H₅-S-CH₂-CH(CH₃)- | C(=O)CF₃ |
| 831. | C₂H₅-SO₂-CH₂-CH(CH₃) | C(=O)CF₃ |
| 832. | (CH₃)₂N-CH₂-CH(CH₃)- | C(=O)CF₃ |
| 833. | (C₂H₅)₂N-CH₂-CH(CH₃)- | C(=O)CF₃ |
| 834. | [(CH₃)₂CH]₂N-CH₂-CH(CH₃)- | C(=O)CF₃ |
| 835. | CH₃-O-CH(CH₃)-CH₂- | C(=O)CF₃ |
| 836. | CH₃-S-CH(CH₃)-CH₂- | C(=O)CF₃ |
| 837. | CH₃-SO₂-CH(CH₃)-CH₂- | C(=O)CF₃ |
| 838. | C₂H₅-O-CH(CH₃)-CH₂- | C(=O)CF₃ |
| 839. | C₂H₅-S-CH(CH₃₎-CH₂- | C(=O)CF₃ |
| 840. | C₂H₅-SO₂-CH(CH₃)-CH₂- | C(=O)CF₃ |
| 841. | (CH₃)₂N-CH(CH₃yCH₂- | C(=O)CF₃ |
| 842. | (C₂H₅)₂N-CH(CH₃)-CH₂- | C(=O)CF₃ |
| 843. | [(CH₃)₂CH]₂N-CH(CH₃)-CH₂- | C(=O)CF₃ |
| 844. | CH₃-O-CH₂-CH₂-CH₂- | C(=O)CF₃ |
| 845. | CH₃-S-CH₂-CH₂-CH₂- | C(=O)CF₃ |
| 846. | CH₃-SO₂-CH₂-CH₂-CH₂- | C(=O)CF₃ |
| 847. | C₂H₅-O-CH₂-CH₂-CH₂- | C(=O)CF₃ |
| 848. | C₂H₅-S-CH₂-CH₂-CH₂- | C(=O)CF₃ |
| 849. | C₂H₅-SO₂-CH₂-CH₂-CH₂- | C(=O)CF₃ |
| 850. | (CH₃)₂N-CH₂-CH₂-CH₂₋ | C(=O)CF₃ |
| 851. | (C₂H₅)₂N-CH₂-CH₂-CH₂- | C(=O)CF₃ |
| 852. | CH₃-O-CH₂-C(CH₃)₂- | C(=O)CF₃ |
| 853. | CH₃-S-CH₂-C(CH₃)₂- | C(=O)CF₃ |
| 854. | CH₃-SO₂-CH₂-C(CH₃)₂- | C(=O)CF₃ |
| 855. | C₂H₅-O-CH₂-C(CH₃)₂- | C(=O)CF₃ |
| 856. | C₂H₅-S-CH₂-C(CH₃)₂- | C(=O)CF₃ |
| 857. | C₂H₅-SO₂-CH₂-C(CH₃)₂- | C(=O)CF₃ |
| 858. | (CH₃)₂N-CH₂-C(CH₃)₂- | C(=O)CF₃ |
| 859. | (C₂H₅)₂N-CH₂-C(CH₃)₂- | C(=O)CF₃ |
| 860. | [(CH₃)₂CH]₂N-CH₂-C(CH₃)₂- | C(=O)CF₃ |
| 861. | Cl-CH₂-C≡C-CH₂- | C(=O)CF₃ |
| 862. | CH₃-O-C(O)-CH₂ | C(=O)CF₃ |
| 863. | C₂H₅-O-C(O)-CH₂ | C(=O)CF₃ |
| 864. | CH₃-O-C(O)-CH(CH₃)- | C(=O)CF₃ |
| 865. | C₂H₅-O-C(O)-CH(CH₃)- | C(=O)CF₃ |
| 866. | (CH₃O)₂CH-CH₂- | C(=O)CF₃ |
| 867. | (C₂H₅O)₂CH-CH₂- | C(=O)CF₃ |
| 868. | C(=O)CH₃ | C(=O)CF₃ |
| 869. | C(=O)CH₂-CH₃ | C(=O)CF₃ |
| 870. | C(=O)CF₃ | C(=O)CF₃ |
| 871. | C(=O)CCl₃ | C(=O)CF₃ |
| 872. | C(=O)CH₂-CH₂-CH₃ | C(=O)CF₃ |
| 873. | C(=O)C-(CH₃)₃ | C(=O)CF₃ |
| 874. | C(=O)CH₂-C₆H₅ | C(=O)CF₃ |
| 875. | C(=O)CH₂-CH₂-CH₃ | C(=O)CF₃ |
| 876. | H | C(=O)CCl₃ |
| 877. | CH₃ | C(=O)CCl₃ |
| 878. | CH₃CH₂- | C(=O)CCl₃ |
| 879. | (CH₃)₂CH- | C(=O)CCl₃ |
| 880. | CH₃CH₂CH₂- | C(=O)CCl₃ |
| 881. | n-C₄H₉ | C(=O)CCl₃ |
| 882. | (CH₃)₃C- | C(=O)CCl₃ |
| 883. | (CH₃)₂CH-CH₂- | C(=O)CCl₃ |
| 884. | n-C₅H₁₁ | C(=O)CCl₃ |
| 885. | (CH₃)₂CH-CH₂-CH₂- | C(=O)CCl₃ |
| 886. | (C₂H₅)₂-CH- | C(=O)CCl₃ |
| 887. | (CH₃)₃C-CH₂- | C(=O)CCl₃ |
| 888. | (CH₃)₃C-CH₂-CH₂- | C(=O)CCl₃ |
| 889. | C₂H₅CH(CH₃)-CH₂- | C(=O)CCl₃ |
| 890. | CH₃-CH₂-C(CH₃)₂- | C(=O)CCl₃ |
| 891. | (CH₃)₂CH-CH(CH₃)- | C(=O)CCl₃ |
| 892. | (CH₃)₃C-CH(CH₃)- | C(=O)CCl₃ |
| 893. | (CH₃)₂CH-CH₂-CH(CH₃)- | C(=O)CCl₃ |
| 894. | CH₃-CH₂-C(CH₃)(C₂H₅)- | C(=O)CCl₃ |
| 895. | CH₃-CH₂-CH₂-C(CH₃)₂- | C(=O)CCl₃ |
| 896. | C₂H₅-CH₂-CH(CH₃)-CH₂- | C(=O)CCl₃ |
| 897. | cyclopropyl | C(=O)CCl₃ |
| 898. | cyclopropyl-CH₂- | C(=O)CCl₃ |
| 899. | cyclopropyl-CH(CH₃)- | C(=O)CCl₃ |
| 900. | cyclobutyl | C(=O)CCl₃ |
| 901. | cyclopentyl | C(=O)CCl₃ |
| 902. | cyclohexyl | C(=O)CCl₃ |
| 903. | HC≡C-CH₂- | C(=O)CCl₃ |
| 904. | HC≡C-CH(CH₃)- | C(=O)CCl₃ |
| 905. | HC≡C-C(CH₃)₂- | C(=O)CCl₃ |
| 906. | HC≡C-C(CH₃)(C₂H₅)- | C(=O)CCl₃ |
| 907. | HC≡C-C(CH₃)(C₃H₇)- | C(=O)CCl₃ |
| 908. | CH₂=CH-CH₂- | C(=O)CCl₃ |
| 909. | H₂C=CH-CH(CH₃)- | C(=O)CCl₃ |
| 910. | H₂C=CH-C(CH₃)₂- | C(=O)CCl₃ |
| 911. | H₂C=CH-C(C₂H₅)(CH₃)- | C(=O)CCl₃ |
| 912. | C₆H₅-CH₂- | C(=O)CCl₃ |
| 913. | 4-(CH₃)₃C-C₆H₄-CH₂- | C(=O)CCl₃ |
| 914. | C₆H₅-CH₂- | C(=O)CCl₃ |
| 915. | 4-(CH₃)₃C-C₆H₄-CH₂- | C(=O)CCl₃ |
| 916. | 4-Cl-C₆H₄-CH₂- | C(=O)CCl₃ |
| 917. | 3-(CH₃O)-C₆H₄-CH₂- | C(=O)CCl₃ |
| 918. | 4-(CH₃O)-C₆H₄-CH₂- | C(=O)CCl₃ |
| 919. | 2-(CH₃O)C₆H₄-CH₂- | C(=O)CCl₃ |
| 920. | 3-Cl-C₆H₄-CH₂- | C(=O)CCl₃ |
| 921. | 2-Cl-C₆H₄-CH₂- | C(=O)CCl₃ |
| 922. | 4-(F₃C)-C₆H₄-CH₂- | C(=O)CCl₃ |
| 923. | NC-CH₂- | C(=O)CCl₃ |
| 924. | NC-CH₂-CH₂- | C(=O)CCl₃ |
| 925. | NC-CH₂-CH(CH₃)- | C(=O)CCl₃ |
| 926. | NC-CH₂-C(CH₃)₂- | C(=O)CCl₃ |
| 927. | NC-CH₂-CH₂-CH₂- | C(=O)CCl₃ |
| 928. | FH₂C-CH₂- | C(=O)CCl₃ |
| 929. | ClH₂C-CH₂- | C(=O)CCl₃ |
| 930. | BrH₂C-CH₂- | C(=O)CCl₃ |
| 931. | FH₂C-CH(CH₃)- | C(=O)CCl₃ |
| 932. | ClH₂C-CH(CH₃)- | C(=O)CCl₃ |
| 933. | BrH₂C-CH(CH₃)- CH₃ | C(=O)CCl₃ |
| 934. | F₂HC-CH₂- | C(=O)CCl₃. |
| 935. | F₃C-CH₂- | C(=O)CCl₃ |
| 936. | FH₂C-CH₂-CH₂- | C(=O)CCl₃ |
| 937. | ClH₂C-CH₂-CH₂- | C(=O)CCl₃ |
| 938. | BrH₂C-CH₂-CH₂- | C(=O)CCl₃ |
| 939. | F₂HC-CH₂-CH₂- | C(=O)CCl₃ |
| 940. | F₃C-CH₂-CH₂- | C(=O)CCl₃ |
| 941. | CH₃-O-CH₂-CH₂- | C(=O)CCl₃ |
| 942. | CH₃-S-CH₂-CH₂- | C(=O)CCl₃ |
| 943. | CH₃-SO₂-CH₂-CH₂- | C(=O)CCl₃ |
| 944. | C₂H₅-O-CH₂-CH₂- | C(=O)CCl₃ |
| 945. | (CH₃)₂CH-O-CH₂-CH₂- | C(=O)CCl₃ |
| 946. | C₂H₅-S-CH₂-CH₂- | C(=O)CCl₃ |
| 947. | C₂H₅-SO₂-CH₂-CH₂- | C(=O)CCl₃ |
| 948. | (CH₃)₂N-CH₂-CH₂- | C(=O)CCl₃ |
| 949. | (C₂H₅)₂N-CH₂-CH₂- | C(=O)CCl₃ |
| 950. | [(CH₃)₂CH]₂N-CH₂-CH₂- | C(=O)CCl₃ |
| 951. | CH₃-O-CH₂-CH(CH₃)- | C(=O)CCl₃ |
| 952. | CH₃-S-CH₂-CH(CH₃)- | C(=O)CCl₃ |
| 953. | CH₃-SO₂-CH₂-CH(CH₃)- | C(=O)CCl₃ |
| 954. | C₂H₅-O-CH₂-CH(CH₃)- | C(=O)CCl₃ |
| 955. | C₂H₅-S-CH₂-CH(CH₃)- | C(=O)CCl₃ |
| 956. | C₂H₅-SO₂-CH₂-CH(CH₃)- | C(=O)CCl₃ |
| 957. | (CH₃)₂N-CH₂-CH(CH₃)- | C(=O)CCl₃ |
| 958. | (C₂H₅)₂N-CH₂-CH(CH₃)- | C(=O)CCl₃ |
| 959. | [(CH₃)₂CH]₂N-CH₂-CH(CH₃)- | C(=O)CCl₃ |
| 960. | CH₃-O-CH(CH₃)-CH₂- | C(=O)CCl₃ |
| 961. | CH₃-S-CH(CH₃)-CH₂- | C(=O)CCl₃ |
| 962. | CH₃-SO₂-CH(CH₃)-CH₂- | C(=O)CCl₃ |
| 963. | C₂H₅-O-CH(CH₃)-CH₂- | C(=O)CCl₃ |
| 964. | C₂H₅-S-CH(CH₃)-CH₂- | C(=O)CCl₃ |
| 965. | C₂H₅-SO₂-CH(CH₃)-CH₂- | C(=O)CCl₃ |
| 966. | (CH₃)₂N-CH(CH₃)-CH₂- | C(=O)CCl₃ |
| 967. | (C₂H₅)₂N-CH(CH₃)-CH₂- | C(=O)CCl₃ |
| 968. | [(CH₃)₂CH]₂N-CH(CH₃)-CH₂- | C(=O)CCl₃ |
| 969. | CH₃-O-CH₂-CH₂-CH₂- | C(=O)CCl₃ |
| 970. | CH₃-S-CH₂-CH₂-CH₂- | C(=O)CCl₃ |
| 971. | CH₃-SO₂-CH₂-CH₂-CH₂- | C(=O)CCl₃ |
| 972. | C₂H₅-O-CH₂-CH₂-CH₂- | C(=O)CCl₃ |
| 973. | C₂H₅-S-CH₂-CH₂-CH₂- | C(=O)CCl₃ |
| 974. | C₂H₅-SO₂-CH₂-CH₂-CH₂- | C(=O)CCl₃ |
| 975. | (CH₃)₂N-CH₂-CH₂-CH₂- | C(=O)CCl₃ |
| 976. | (C₂H₅)₂N-CH₂-CH₂-CH₂- | C(=O)CCl₃ |
| 977. | CH₃-O-CH₂-C(CH₃)- | C(=O)CCl₃ |
| 978. | CH₃-S-CH₂-C(CH₃)₂- | C(=O)CCl₃ |
| 979. | CH₃-SO₂-CH₂-C(CH₃)₂- | C(=O)CCl₃ |
| 980. | C₂H₅-O-CH₂-C(CH₃)₂- | C(=O)CCl₃ |
| 981. | C₂H₅-S-CH₂-C(CH₃)₂- | C(=O)CCl₃ |
| 982. | C₂H₅-SO₂-CH₂-C(CH₃)₂- | C(=O)CCl₃ |
| 983. | (CH₃)₂N-CH₂-C(CH₃)₂- | C(=O)CCl₃ |
| 984. | (C₂H₅)₂N-CH₂-C(CH₃)₂- | C(=O)CCl₃ |
| 985. | [(CH₃)₂CH]₂N-CH₂-C(CH₃)₂- | C(=O)CCl₃ |
| 986. | Cl-CH₂-C≡C-CH₂- | C(=O)CCl₃ |
| 987. | CH₃-O-C(O)-CH₂ | C(=O)CCl₃ |
| 988. | C₂H₅-O-C(O)-CH₂ | C(=O)CCl₃ |
| 989. | CH₃-O-C(O)-CH(CH₃)- | C(=O)CCl₃ |
| 990. | C₂H₅-O-C(O)-CH(CH₃)- | C(=O)CCl₃ |
| 991. | (CH₃O)₂CH-CH₂- | C(=O)CCl₃ |
| 992. | (C₂H₅O)₂CH-CH₂- | C(=O)CCl₃ |
| 993. | C(=O)CH₃ | C(=O)CCl₃ |
| 994. | C(=O)CH₂-CH₃ | C(=O)CCl₃ |
| 995. | C(=O)CF₃ | C(=O)CCl₃ |
| 996. | C(=O)CCl₃ | C(=O)CCl₃ |
| 997. | C(=O)CH₂-CH₂-CH₃ | C(=O)CCl₃ |
| 998. | C(=O)C-(CH₃)₃ | C(=O)CCl₃ |
| 999. | C(=O)CH₂-C₆H₅ | C(=O)CCl₃ |
| 1000. | C(=O)CH₂-CH₂-CH₃ | C(=O)CCl₃ |
| 1001. | -CH₂CH₂- | |
| 1002. | -CH₂CH₂CH₂- | |
| 1003. | -CH₂CH₂CH₂- | |
| 1004. | -CH₂CH₂CH₂CH₂- | |
| 1005. | -CH₂CH₂CH₂CH₂CH₂- | |
| 1006. | -CH₂CH₂CH₂CH₂CH₂CH₂- | |
| 1007. | -CH₂CH₂OCH₂CH₂- | |
| 1008. | -CH₂CH₂-NH-CH₂CH₂- | |
| 1009. | -CH=CH-CH=CH- | |
| 1010. | -N=CH-CH=CH- | |
| 1011. | -CH=N-CH=CH- | |
| 1012. | -C(CH₃)=CH-CH=CH- | |
| 1013. | -C(CH₃)=CH-CH=C(CH₃)- | |
| 1014. | -CH=C(CH₃)-C(CH₃)=CH- | |
| 1015. | -CH=C(Cl)-C(Cl)=CH- | |
| 1016. | -CH=C(Br)-C(Br)=CH- | |

Cyanopyridinesulfonamide compounds of the formula Ia, i.e. compounds I, where, n = 2, can be prepared, for example, by reacting a 2-cyanopyridine-sulfonylhalide (II) with ammonia or a primary or secondary amine (III) (see Scheme 1).

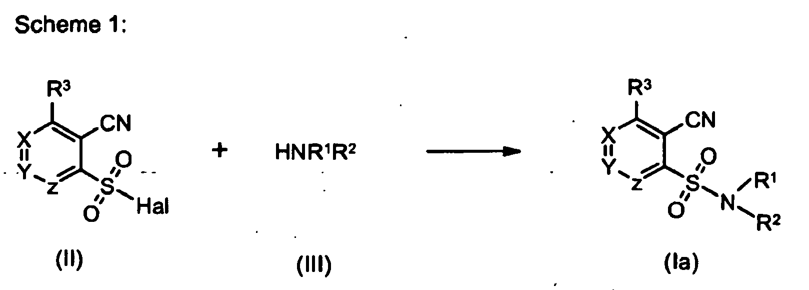

In Scheme 1 the variables X, Y, Z and R¹ to R³ are as defined above and Hal is halogen, especially chlorine or bromine. The reaction of the sulfonylhalide II, especially a sulfonylchloride, with the amine III can be carried out similarly to standard methods of reacting sulfonylhalides with primary or secondary amines as described in J. March, 4th edition 1992, p. 499 and in the literature cited therein.

In general, the amine III is employed in an at least equimolar amount, preferably at least 2-fold molar excess, based on the sulfonyl halide II, to bind the hydrogen halide formed. It may be advantageous to employ the amine III in an up to 6-fold molar excess, based on the sulfonylhalide II**.**

It may be advantageous to carry out the reaction in the presence of an auxiliary base, Suitable auxiliary bases include organic bases, for example tertiary amines, such as aliphatic tertiary amines, such as trimethylamine, triethylamine or diisopropylamine, cycloaliphatic tertiary amines such as N-methylpiperidine or aromatic amines such pyridine, substituted pyridines such as 2,3,5-collidine, 2,4,6-collidine, 2,4-lutidine, 3,5-lutidine or 2,6-lutidine and inorganic bases for example alkali metal hydrides such as sodium hydride and potassium hydride, alkali metal carbonates and alkaline earth metal carbonates such as lithium carbonate, potassium carbonate and sodium carbonate; calcium carbonate and alkaline metal hydrogencarbonates such as sodium hydrogen carbonate. The molar ratio of auxiliary base to sulfonylhalide II is preferably in the range of from 1:1 to 4:1, preferably 1:1 to 2:1. If the reaction is carried out in the presence of an auxiliary base, the molar ratio of amine III to sulfonylhalide II usually is 1:1 to 1.5:1,

The reaction of II and III is usually carried out in the presence of a solvent. Suitable solvents are polar solvents which are inert under the reaction conditions, for example C₁-C₄-alkanols such as methanol, ethanol, n-propanol or isopropanol, dialkyl ethers such as diethyl ether, diisopropyl ether or methyl tert-butyl ether, cyclic ethers such as dioxane or tetrahydrofuran, acetonitrile, carboxamides such as N,N-dimethylformamide, N,N-dimethyl acetamide or N-methylpyrrolidinone, water, (provided the sulfonylhalide II is sufficiently resistent to hydrolysis under the reaction conditions used or a mixture thereof.

The reaction of II and III is usually carried out at a reaction temperature ranging from 0°C to the boiling point of the solvent, preferably from 0 to 30°C.

If not commercially available, the amines III can be prepared by standard methods for preparing primary or secondary amines.

Sulfonylhalides II except for the compound of formula II, where Hal is chlorine, X is CR⁴, Y is CR⁵, Z is N, R⁴ is hydrogen and R³ and R⁵ are each CH₃, are new and can be prepared by the methods described hereinafter.

Sulfonylchloride compounds II wherein Z is nitrogen can be be prepared, for example, according to the reaction sequence shown in Scheme 2 where the variables R³, R⁴ and R⁵ are as defined above: 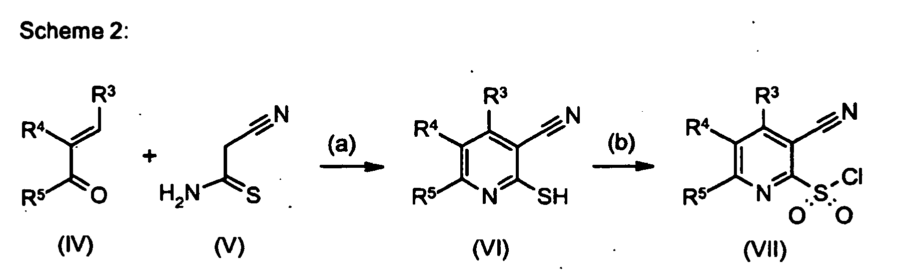
a) Condensation of cyanothioacetamide V with an unsaturated carbonyl compound IV to obtain the thiopyridine VI by analogy to a process described in Liebigs Annalen der Chemie, No. 1, (1986), pp. 210-219 and the Journal of the Chinese Chemical Society (Taiwan), Vol. 49, No. 4, (2002), pp. 561-565.
b) Oxidation of the thiol VI to the sulfonylchloride VII, for example, by reacting the thiol VI with chlorine in water or a water-solvent mixture, e. g. a mixture of water and acetic acid or water and dichloromethane, in analogy to a process described in Jerry March, 3rd edition, 1985, reaction 9-27, p. 1087.

Sulfonylchloride compounds II wherein Y is nitrogen can be prepared, for example, according to the reaction sequence shown in Scheme 3 wherein the variables R³, R⁴ and R⁶ are as defined above and R is alkyl, e.g. n-propyl: 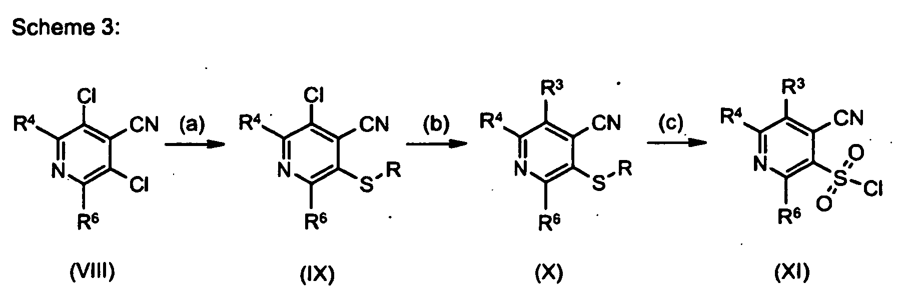
(a) Conversion of a dichloroisonicotinonitrile VIII into a thioether IX can be carried out, for example, by analogy to a process described in Heterocycles Vol. 43, No. 9, (1996), pp. 1893-1899, by reacting VIII with the anion of an alkylmercaptan reagent, formed from alkylmercaptan and a base. Suitable bases comprise, for example, alkali metal hydrides such as sodium hydride and potassium hydride, alkali metal carbonates and alkaline earth metal carbonates such as lithium carbonate, potassium carbonate and sodium carbonate, calcium carbonate, alkaline metal hydrogencarbonates such as sodium hydrogen carbonate, as well as organo alkyl metal reagents like butyl lithium. The reaction is usually performed in an inert organic solvent, for example dialkyl ethers such as diethyl ether, diisopropyl ether or methyl tert-butyl ether, cyclic ethers such as dioxane or tetrahydrofuran, acetonitrile, carboxamides such as N,N-dimethylformamide, N,N-dimethyl acetamide or N-methylpyrrolidinone. The reaction is usually carried out at a reaction temperature ranging from 0 °C to the boiling point of the solvent.
   The molar ratio of the alkaline thiopropylate to dichloronicotononitrile VIII is preferably in the range from 1:1 to 1.5:1.
(b) If R³ is a (halo)alkyl or (halo)alkoxy group, respectively, introduction of said group can be achieved, for example, by analogy to a process described in Heterocycles Vol. 43, No. 9, (1996), pp. 1893-1899, by reacting IX with an alkali metal alkylate or alkali metal alkoxide, e.g. by reacting with sodium methylate in an inert organic solvent. Suitable solvents comprise, for example, dialkyl ethers such as diethyl ether, diisopropyl ether or methyl tert-butyl ether, cyclic ethers such as dioxane or tetrahydrofuran, acetonitrile, carboxamides such as N,N-dimethylformamide, N,N-dimethyl acetamide or N-methylpyrrolidinone. The reaction is usually carried out at a reaction temperature ranging from 0 °C to the boiling point of the solvent. The molar ratio of alkalimetal alkylate or alkoxide to chloronicotononitrile IX is preferably in the range from 1:1 to 5:1, preferably 1.1 to 1.5:1.
(c) Oxidative cleavage of the thioether X to obtain the sulfonylchloride XI can be achieved, for example, by reacting the thioether X with chlorine in water or a water-solvent mixture, e. g. a mixture of water and acetic acid or water and dichloromethane, in analogy to a process described in Canadian Journal of Chemistry, Vol. 74, No. 9, (1996), pp. 1638-1648.

The dichloronicotononitrile VIII can be prepared in analogy to a process described in Heterocycles Vol: 43, No. 9, (1996), pp. 1893-1899; or a process described in Journal of Medicinal Chemistry, Vol. 44, No.6, (2001), pp. 988-1000.

Sulfonylchloride compounds II wherein Y is nitrogen and R³ is alkyl, e.g. CH₃, or haloalkyl, can also be prepared, for example, according to the reaction sequence shown in Scheme 4 where the variables R⁴ and R⁶ are as defined above and R is alkyl, e.g. n-propyl or n-butyl: 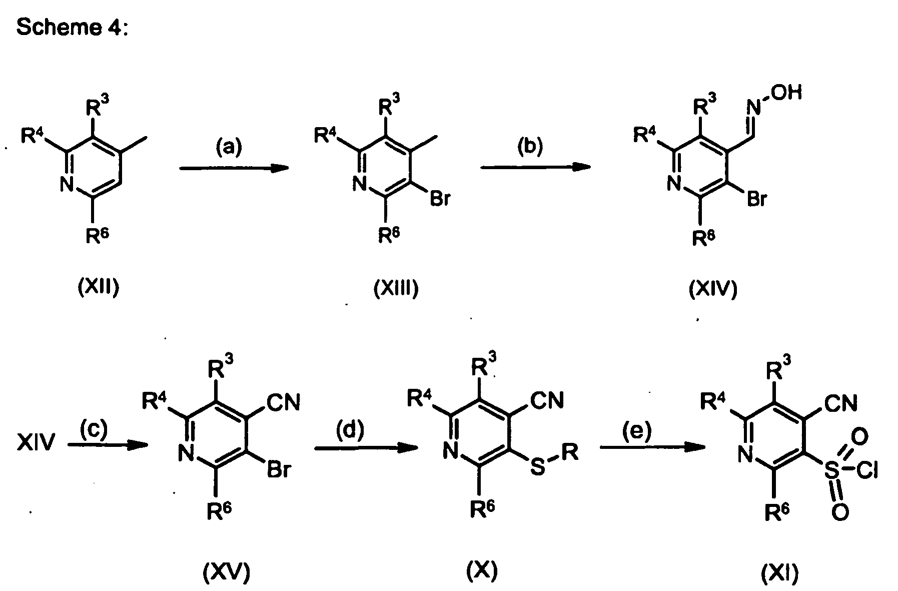
(a) Conversion of a pyridine compound XII into a bromide XIII can be achieved by reaction of XII with a bromination agent such as bromine in a polar solvent such as sulfuric acid. The reaction can be performed by analogy to a process described in Zeitschrift fur Chemie, Vol. 28, No. 2, (1988), pp. 59-60.
(b) Conversion of XIII into the oxime XIV can be achieved by reacting XIII with an alkali amide base such as lithiumdiisopropylamide or an alkali alkoxide base such as potassium tertbutoxide and an nitrosation reagent like alkyl nitrites, for example n-butyl nitrite, by analogy to a process described in Heterocycles, Vol. 6, No. 9-10, (1977), pp. 1616-1621.
(c) Dehydration of oxime XIV to nitrile XV can be achieved by reaction of XIV with refluxing acetic anhydride according to a process described in Heterocycles, Vol. 5, No. 1, (1987), pp. 343-345.
(d) Conversion of the bromonitrile XV into a thioether X can be achieved by reacting X with an alkaline thioalkylate reagent formed from alkylmercaptan and a base in analogy to step a) of scheme 3.
(e) Oxidative cleavage of the thioether X to obtain the sulfonylchloride XI can be achieved, for example, by reacting the thioether X with chlorine in water or a water-solvent mixture as descrived for step c) of scheme 3.

A sulfonamide compound I wherein X is nitrogen can be prepared, for example, according to the reaction sequence shown in Scheme 5 where the variables R¹, R², R³, R⁵ and R⁶ are as defined above. A skilled person will readily appreciate that compounds XIX and XX are within the scope of formula I and thus form also part of the invention: 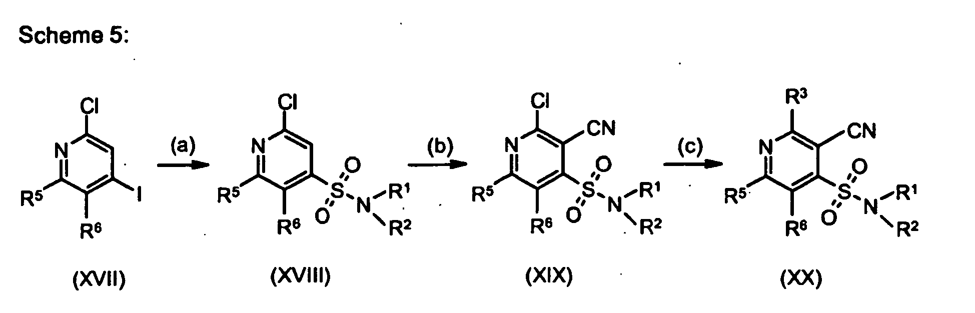
(a) Conversion of a 4-iodopyridine XVII into a sulfonamide XVIII can be performed by analogy to the process described in Journal of Organic Chemistry, Vol. 68, No. 21, (2003), pp. 8274-8276. The iodopyridine XVII is e.g. treated with an alkylmagnesium halide such as isopropylmagnesium chloride to generate the corresponding grignard reagent, followed by treatment with SO₂ and subsequent treatment with sulfuryl chloride. The thus obtained sulfonylchloride may be isolated or not and subsequentially converted into the sulfonamide XVIII by reacting the intermediate sulfonylchloride with a primary or secondary amine similarly to the process described in scheme 1.
(b) Conversion of XVIII into a nitrile XIX can be achieved by reaction of XVIII with a strong organometallic base like a lithiumdialkylamide or an alkyllithium such as lithiumdiisopropylamide or t-butyllithium followed by treatment with a cyanide source like p-toluene sulfonyl cyanide in a inert organic solvent like tetrahydrofuran. The reaction can be performed by analogy to a process described in Journal of Heterocyclic Chemistry 1992, Vol. 29, No. 1, pp. 61-64 and Journal of Organic Chemistry 1987, Vol. 52, No. 6, pp. 1133-1136.
(c) If desired, chlorine can be replaced by a (halo)alkyl or (halo)alkoxy substituent R³, for example, by analogy to the process described in step b) of scheme 3.

The preparation of the sulfonamide compounds I wherein X is nitrogen and n = 2 can also be carried out, for example, according to the reaction sequence shown in Scheme 6 where the variables R¹, R², R³, R⁵ and R⁶ are as defined above: 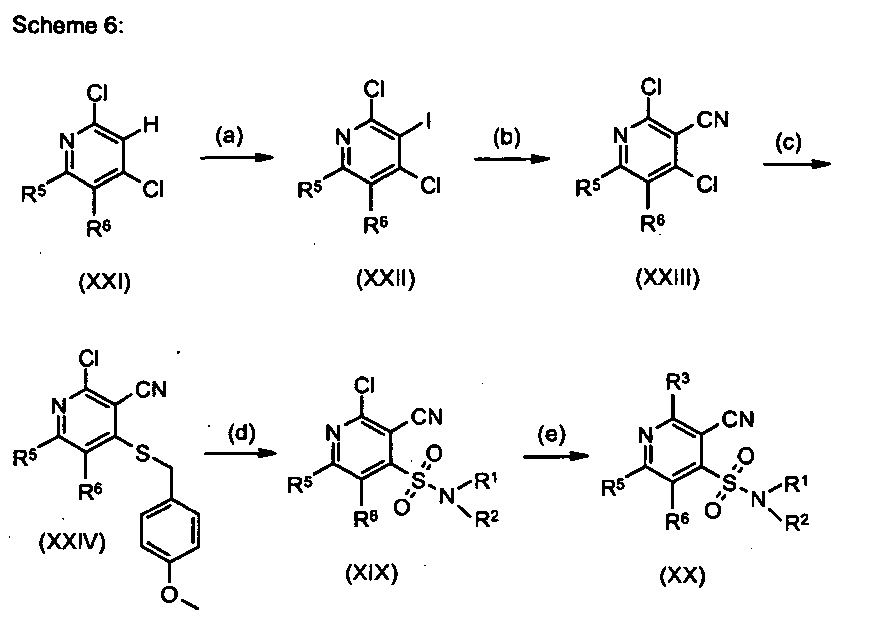
(a) Conversion of XXI into a iodide XXII can be performed by reaction of XXI with a strong organometallic base like a lithiumdialkylamide or an alkyllithium such as lithiumdiisopropylamide or t-butyllithium followed by treatment with iodide in an inert organic solvent such as tetrahydrofuran by analogy to a process described in European Journal of Organic Chemistry, Vol. 7, (2001), pp. 1371-1376. The starting XXI can be prepared according to a procedure described in Synthetic Communications, Vol. 22, No.19, (1992), pp. 2829-2837.
(b) Conversion of iodide XXII into a nitrile XXII is achieved by reaction of XXII with copper(I)cyanide in refluxing N,N-dimethylformamide (perferably under an inter gas atmosphere) by analogy to a process described in Journal of Medicinal Chemistry Vol. 47, No. 14, (2004), pp. 3658-3664.
(c) In step c the chloride XXIII is converted into a p-methoxybenzylthioether XXIV by reaction of XXIII with an alkaline 4-methoxybenzylthiolate reagent formed from 4-methoxybenzylmercaptane and a base by analogy to a process described in Bioorganic & Medicinal Chemistry Letters, Vo. 11, No. 14, (2001), pp. 1951-1954.
   Suitable base are e.g. alkali metal hydrides, alkali metal carbonates and alkaline earth metal carbonates, or an organo alkyl metal reagent such as butyl lithium. The reaction can be performed in an inert organic solvent, for example dialkyl ethers cyclic ethers such as tetrahydrofuran, carboxamides such as N,N-dimethyl formamide, N,N-dimethyl acetamide or N-methylpyrrolidinone. The reaction is usually carried out at a reaction temperature ranging from 0 °C to the boiling point of the solvent. The molar ratio of the alkaline thiopropylate to dichloronicotononitrile XXIII is preferably in the range from 1:1 to 1.5:1.
(d) Oxidative cleavage of the thioether XXIV to obtain the sulfonamide XXV is achieved, for example, by reacting XXIV with chlorine in water or a water-solvent mixture in analogy to step c) of scheme 3, directly followed by reaction of the intermediate sulfonyl chloride with amines by analogy to the method described scheme 1.
(e) Replacement of chlorine and Introduction of an (halo)alkyl or (halo)alkoxide substituent can be achieved, for example, by analogy to the process described in step b) of scheme 3.

The cyanopyridine sulfinylamide compounds of the formula XXV, i. e. compounds I, where n = 1 can be prepared, for example, by reacting a 2-cyanopyridine-sulfinylhalide (XXVI) (in particular.a 2-cyanopyridine-sulfinylchloride XXVI) with ammonia or a primary or secondary amine (III) by analogy to a process described in Journal of Organic Chemistry 1983, Vol. 48 pp. 4803-4807 (see Scheme 7).

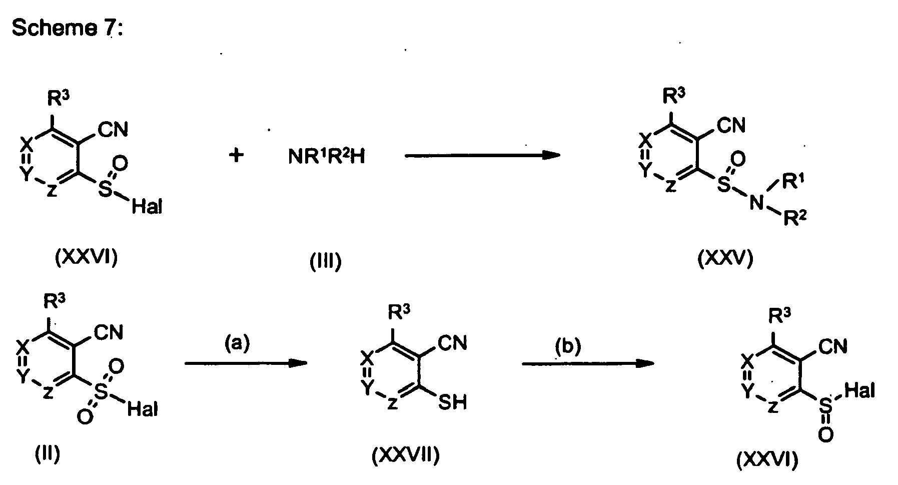

In Scheme 7 the variables X, Y, Z and R¹ to R³ are as defined above. Hal is halogen, in particular chlorine. In general, the amine III is employed in an at least equimolar amount, preferably at least 2-fold molar excess, based on the sulfinylhalide XXVI, to bind the hydrogen halide formed. It may be advantageous to employ the primary amine III in an up to 6-fold molar excess, based on the sulfinylhalide XXVI. The reaction of III with XXVI is usually carried out at a reaction temperature ranging from 0 °C to the boiling point of the solvent, preferably from 0 to 30 °C.

The reaction of a sulfinylhalide XXVI with an amine III is usually carried out in the presence of a solvent. Suitable solvents are polar solvents which are inert under the reaction conditions, for example C₁-C₄-alkanols such as methanol, ethanol, n-propanol or isopropanol, dialkyl ethers such as diethyl ether, diisopropyl ether or methyl tert-butyl ether, cyclic ethers such as dioxane or tetrahydrofuran, acetonitrile, carboxamides such as N,N-dimethylformamide, N,N-dimethyl acetamide or N-methylpyrrolidinone, water, (provided the sulfinylchloride XXI is sufficiently resistent to hydrolysis under the reaction conditions used) or a mixture thereof.

The sulfinylchloride compounds XXI may be prepared, for example by the process as described hereinafter (see scheme 7) comprising steps (a) and (b):
(a) Reduction of a sulfonylhalide II to the corresponding thiol XXVII using a suitable reducing agent. The sulfonylhalide starting compounds II can be prepared as described above in the schemes 2-6. Suitable reducing agent include (1) tris(2-carboxyethyl)phosphine in a mixture of dioxane and water as described in Synthetic Communications 2003, Vol. 33, No. 20, pp. 3503-3511; or (2) triphenylphosphine with or without the addition of iodine in an aromatic solvent like benzene or toluene as described in Bulletin of the Chemical Society of Japan 1983, Vol. 56, No. 12, pp. 3802-3812; or (3) zinc in combination with dichlorodimethylsilane, dimethylacetamide in a chlorinated hydrocarbon like dichloroethane as described in Tetrahedron Letters 1999, Vol. 40, pp. 3179-3182.
(b) Conversion of the thiol XXVII into the sulfinylchloride XXVI by oxidative chlorination using sulfuryl chloride in acetic acid as described in Synthesis 1987, No. 1, pp. 72-73.

The sulfenylamide compounds of the formula XXVIII, i. e. compounds I, where n = 0 can be prepared, for example, by reacting a 2-cyanopyridine-sulfenychloride (XXIX) with ammonia or a primary or secondary amine (III) by analogy to a process described in Journal of Organic Chemistry 1977, V0. 42, No. 4, pp. 597-600 or in Journal of Medicinal Chemistry 2001, Vol. 44, No. 13, pp. 2253-2258 (see Scheme 8):

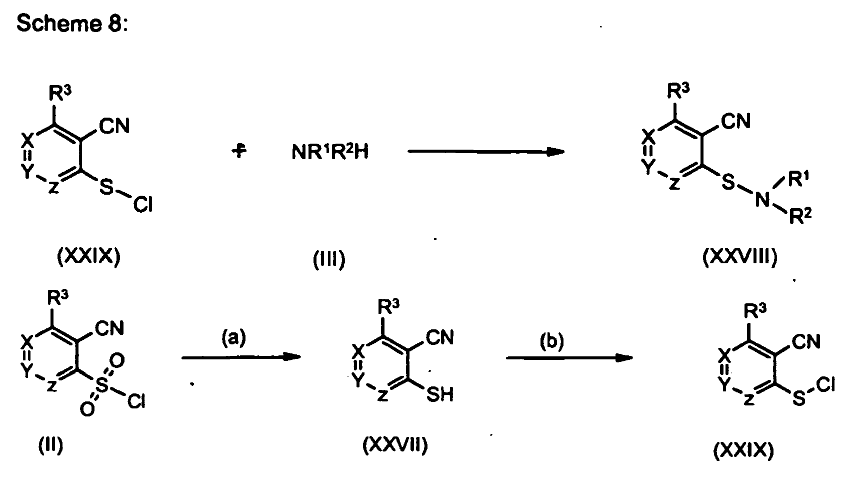

In Scheme 8 the variables X, Y, Z and R¹ to R³ are as defined above The reaction of a sulfenylchloride XXIX with an amine III is usually carried out at a reaction temperature ranging from 0 °C to the boiling point of the solvent, preferably from 0 to 30 °C.

In general, the amine III is employed in an at least equimolar amount, preferably at least 2-fold molar excess, based on the sulfenylchloride XXIX, to bind the hydrogen chloride formed, It may be advantageous to employ the amine III in an up to 6-fold molar excess, based on the sulfenylchloride XXIX.

The reaction is usually carried out in the presence of a solvent. Suitable solvents are polar solvents which are inert under the reaction conditions, for example C₁-C₄-alkanols such as methanol, ethanol, n-propanol or isopropanol, dialkyl ethers such as diethyl ether, diisopropyl ether or methyl tert-butyl ether, cyclic ethers such as dioxane or tetrahydrofuran, acetonitrile, carboxamides such as N,N-dimethylformamide, N,N-dimethyl acetamide or N-methylpyrrolidinone, water, (provided the sulfenylchloride XXIX is sufficiently resistent to hydrolysis under the reaction conditions used) or a mixture thereof.

The sulfenylchloride compounds XXIX may be prepared, for example by the process as described hereinafter comprising steps a) and b) (see scheme 8).
a) Reduction of a sulfonylhalide II to the corresponding thiol XXVII using a suitable reducing agent as described for step a) in scheme 7; and
b) Direct conversion of the thiol XXVII to the sulfenylchloride XXV by oxidative chlorination using N-chlorosuccinimide in a chlorinated hydrocarbon such as dichloromethane as described in the Journal of Organic Chemistry 1985, Vol. 50, No. 19 pp. 3592-3595.

If individual compounds cannot be prepared via the above-described routes, they can be prepared by derivatization of other compounds I or by customary modifications of the synthesis routes described.

The reaction mixtures are worked up in the customary manner, for example by mixing with water, separating the phases, and, if appropriate, purifying the crude products by chromatography, for example on alumina or silica gel may be employed. Some of the intermediates and end products may be obtained in the form of colorless or pale brown viscous oils which are freed or purified from volatile components under reduced pressure and at moderately elevated temperature. If the intermediates and end products are obtained as solids, they may be purified by recrystallization or digestion.

The compounds of the formula I and their salts are in particular suitable for efficiently controlling arthropodal pests such as arachnids, myriapods and insects as well as nematodes.

In particular, they are suitable for controlling insect pests, such as insects from the order of the

lepidopterans (Lepidoptera): for example *Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, E*/*asmopalpus lignosellus, Eupoecilia ambiguella, Evetria bouliana, Feltia subterranea, Galleria mellonella, Grapholitha funebrana, Grapholitha molesta, Heliothis armigera, Heliothis vinascens, Heliothis zea, Hellula undalis, Hibemia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Mala*cosoma *neustria, Mamestra brassicae, Orgyia pseudotsugata, Ostrinia nubilalis, Panolis flammea, Pectinophora gossypiella, Peridroma saucia, Phalera bucephala, Phthori*maea *operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera eridania, Spodoptera frugiperda, Spodoptera littoralis, Spodoptera litura, Thaumatopoea pityocampa, Tortrix viridana, Trichoplusia ni* and *Zeiraphera canadensis,*
beetles (Coleoptera), for example *Agrilus sinuatus, Agriotes lineatus, Agriotes obscurus, Amphimallus solstitialis, Anisandrus dispar, Anthonomus grandis, Anthonomus pomorum, Aphthona euphoridae, Athous haemorrhoidalis, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Cetonia aurata, Ceuthorrhynchus assimilis, Ceuthorrhynchus napi, Chaetocnema tibialis, Conoderus vespertinus, Crioceris asparagi, Ctenicera ssp., Diabrotica longicomis, Diabrotica semipunctata , Diabrotica 12-punctata, Diabrotica virgifera, Diabrotica speciosa, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius califomicus, Lissorhoptrus oryzophilus, Melanotus communis, Meligethes aeneus, Melolontha hippocastani, Melolontha mololontha, Oulema oryzae, Ortiorrhynchus sulcatus, Otiorrhynchus ovatus, Phaedon cochleariae, Phyllobius pyri, Phyllotreta chrysocephala, Phyllophaga sp., Phyllopertha horticola, Phyllotreta nemorum, Phyllotreta striolata, Popillia japonica, Sitona lineatus* and *Sitophilus granaria,*
flies, mosquitoes (Diptera), for example *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Ceratitis capitata, Chrysomya bezziana, Chrysomya hominivorax, Chrysomya macellaria, Chrysops discalis, Chrysops silacea, Chrysops atlanticus, Cochliomyia hominivorax, Contarinia sorghicola, Cordylobia anthropophaga, Culex pipiens, Culex nigripalpus, Culex quinquefasciatus, Culex tarsalis, Culiseta inornata, Culiseta melanura, Dacus cucurbitae, Dacus oleae, Dasineura brassicae, Delia antique, Delia coarctata, Delia platura, Delia radicum, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hylemyia platura, Hypoderma lineata, Leptoconops torrens, Liriomyza sativae, Liriomyza trifolii, Lucilia caprina, Lucilia cuprina, Lucilia sericata, Lycoria pectoralis, Mansonia tilillanus, Mayetiola destructor, Musca domestica, Muscina stabulans, Oestrus ovis, Opomyza florum, Oscinella frit, Pegomya hysocyami, Phorbia antiqua, Phobia brassicae, Phorbia coarctata, Phlebotomus argentipes, Psorophora columbiae, Psila rosae, Psorophora discolor, Prosimulium mixtum, Rhagoletis cerasi, Rhagoletis pomonella, Sarcophagi haemorrhoidalis, Sarcophaga sp., Simulium vittatum, Stomoxys calcitrans, Tabanus bovinus, Tabanus atratus, Tabanus lineola, and Tabanus similis, Tipula oleracea* and *Tipula paludosa,*
thrips (Thysanoptera), e.g. *Dichromothrips corbetti, Dichromothrips spp., Frankliniella fusca, Frankliniella occidentalis, Frankliniella tritici, Scirtothrips citri, Thrips oryzae, Thrips palmi* and *Thrips tabaci,* cockroaches (Blattaria - Blattodea), e.g. *Blattelia germanica, Blattelia asahinae, Blattella orientalis, Peri-planeta americana, Periplaneta japonica, Periplaneta brunnea, Periplaneta fuligginosa, and Periplaneta australasiae,*
true bugs *(Hemiptera),* e.g. *Acrostemum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis* and *Thyanta perditor,*
Homoptera e.g. *Acyrthosiphon onobrychis, Adelges laricis, Aphidula nasturtii, Aphis craccivora, Aphis fabae, Aphis forbesi, Aphis pomi, Aphis gossypii, Aphis grossulariae, Aphis schneideri, Aphis spiraecola, Aphis sambuci, Acyrthosiphon pisum, Au*/*acorthum solani, Bemisia tabaci, Bemisia argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzus persicae, Myzus ascalonicus, Myzus cerasi, Myzus varians, Nasonovia ribisnigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand, Viteus vitifolii, Cimex lectularius, Cimex hemipterus, Reduvius senilis, Triatoma spp., and Arilus critatus*
termites (Isoptera ), e.g. *Calotermes flavicollis, Leucotermes flavipes, Heterotermes aureus, Reticulitermes flavipes, Reticulitermes lucifugus, Reticulitermes virginicus,* and *Termes natalensis,*
ants, bees, wasps, sawflies (Hymenoptera), e.g. *Athalia rosae*, *Atta cephalotes, Atta capiguara, Atta cephalotes, Atta laevigata, Atta robusta, Atta sexdens, Atta texana, Crematogaster spp., Hoplocampa minuta, Hoplocampa testudinea, Monomorium pharaonis, Solenopsis geminata, Solenopsis invicta, Solenopsis richteri, Solenopsis xyloni, Pogonomyrmex barbatus, Pogonomyrmex californicus, Pheidole megacephala, Dasymutilla occidentalis, Bombus spp. Vespula squamosa, Paravespula vulgaris, Paravespula pennsylvanica, Paravespula germanica, Dolichovespula maculata, Vespa crabro, Polistes rubiginosa, Camponotus floridanus, and Linepithema humile,*
crickets, grasshoppers, locusts (Orthoptera), e.g *Acheta domestica, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca gregaria, Schistocerca peregrina, Stauronotus maroccanus, Dociostaurus maroccanus, Tachycines asynamorus, Oedaleus senegalensis, Zonozerus variegatus, Hieroglyphus daganensis, Kraussaria angulifera, Calliptamus italicus, Chortoicetes terminifera, and Locustana pardalina.*
fleas (Siphonaptera), e.g. *Ctenocephalides felis, Ctenocephalides canis, Xenopsylla cheopis, Pulex irritants, Tunga penetrans, and Nosopsyllus fasciatus,*
silverfish, firebrat (Thysanura), e.g. *Lepisma saccharina and Thermobia domestica,*
centipedes (Chilopoda), e.g. *Scutigera coleoptrata,*
milipedes (Diplopoda), e.g. *Narceus spp.,*
earwigs (Dermaptera), e.g. *forficula auricularia,* and
lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus and Solenopotes capillatus.*
Collembola (springtails), *e.g. Onychiurus ssp..*

They are also suitable for controlling Nematodes : plant parasitic nematodes such as root knot nematodes, *Meloidogyne hapia, Meloidogyne incognita, Meloidogyne javani*ca, and other Meloidogyne species; cyst-forming nematodes, *Globodera rostochiensis* and other Globodera species; *Heterodera avenae, Heterodera glycines, Heterodera schachtii, Heterodera trifolii,* and other Heterodera species; Seed gall nematodes, Anguina species; Stem and foliar nematodes, Aphelenchoides species; Sting nematodes, *Belonolaimus longicaudatus* and other Belonolaimus species; Pine nematodes, *Bursaphelenchus xylophilus* and other Bursaphelenchus species; Ring nematodes, Criconema species, Criconemella species, Criconemoides species, Mesocriconema species; Stem and bulb nematodes, *Ditylenchus destructor, Ditylenchus dipsaci* and other Ditylenchus species; Awl nematodes, Dolichodorus species; Spiral nematodes, *Heliocotylenchus multicinctus* and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, *Longidorus elongatus* and other Longidorus species; Lesion nematodes, *Pratylenchus neglectus, Pratylenchus penetrans, Pratylenchus curvitatus, Pratylenchus goodeyi* and other Pratylenchus species; Burrowing nematodes, *Radopholus similis* and other Radopholus species; Reniform nematodes, *Rotylenchus robustus* and other Rotylenchus species; Scutellonema species; Stubby root nematodes, *Trichodorus primitivus* and other Trichodorus species, Paratrichodorus species; Stunt nematodes, *Tylenchorhynchus claytoni,* Tylenchorhynchus dubius and other Tylenchorhynchus species; Citrus nematodes, Tylenchulus species; Dagger nematodes, Xiphinema species; and other plant parasitic nematode species.

The compounds of the formula I and their salts are also useful for controlling Arachnoidea, such as arachnids *(Acarina),* e.g. of the families *Argasidae, Ixodidae* and *Sarcoptidae,* such as *Amblyomma americanum, Amblyomma variegatum, Ambryomma maculatum, Argas persicus, Boophilus annulatus, Boophilus decoloratus, Boophilus microplus, Dermacentor silvarum, Dermacentor andersoni, Dermacentor variabilis, Hyalomma truncatum, Ixodes ricinus, Ixodes rubicundus, Ixodes scapularis, Ixodes holocydus, Ixodes pacficus, Omithodorus moubata, Omithodorus hermsi, Omithodorus turicata, Omithonyssus bacoti, Otobius megnini, Dermanyssus gallinae, Psoroptes ovis, Rhipicephalus appendiculatus, Rhipicephalus evertsi, Rhipicephalus sanguineus, Sarcoptes scabiei,* and Eriophyidae spp. such as *Aculus schlechtendali, Phyllocoptrata oleivora* and *Eriophyes sheldoni;* Tarsonemidae spp. such as *Phytonemus pallidus* and *Polyphagotarsonemus latus;* Tenuipalpidae spp. such as *Brevipalpus phoenicis;* Tetranychidae spp. such as *Tetranychus cinnabarinus, Tetranychus kanzawai, Tetranychus pacificus, Tetranychus telarius* and *Tetranychus urticae, Panonychus ulmi, Panonychus citri, and Oligonychus pratensis,* Araneida, e.g. Latrodectus mactans, and Loxosceles reclusa.

Compounds of the formula I are particularly useful for controlling insects, preferably sucking or piercing insects such as insects from the genera Thysanoptera, Hymenoptera, Orthoptera and Homptera, in particular the following species:
Compounds of the formula I are particularly useful for controlling insects of the orders Homoptera and Thysanoptera and more preferably for controlling aphids.

For use in a method according to the present invention, the compounds I can be converted into the customary formulations, e.g. solutions, emulsions, suspensions, dusts, powders, pastes, granules and directly sprayable solutions. The use form depends on the particular purpose and application method. Formulations and application methods are chosen to ensure in each case a fine and uniform distribution of the compound of the formula I according to the present invention.

The formulations are prepared in a known manner, e.g. by extending the active ingredient with solvents and/or carriers, if desired using surfactants, i.e. emulsifiers and dispersants and other formulation auxiliaries.

Solvents/carriers, which are suitable, are e.g.:
- solvents such as water, aromatic solvents (for example Solvesso products, xylene and the like), paraffins (for example mineral fractions), alcohols (for example methanol, butanol, pentanol, benzyl alcohol), ketones (for example cyclohexanone, gamma-butyrolactone), pyrrolidones (NMP, NOP), acetates (glycol diacetate), alkyl lactates, lactones such as g-butyrolactone, glycols, fatty acid dimethylamides, fatty acids and fatty acid esters, triglycerides, oils of vegetable or animal origin and modified oils such as alkylated plant oils. In principle, solvent mixtures may also be used.
- carriers such as ground natural minerals and ground synthetic minerals, such as silica gels, finely divided silicic acid, silicates, talc, kaolin, attaclay, limestone, lime, chalk, bole, loess, clay, dolomite, diatomaceous earth, calcium sulfate and magnesium sulfate, magnesium oxide, ground synthetic materials, fertilizers, such as, for example, ammonium sulfate, ammonium phosphate, ammonium nitrate, ureas and products of vegetable origin, such as cereal meal, tree bark meal, wood meal and nutshell meal, cellulose powders and other solid carriers.

Suitable surfactants are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenyl ether, ethoxylated isooctylphenol, octylphenol, nonylphenol, alkylphenyl polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignin-sulfite waste liquors and methylcellulose.

Suitable auxiliaries comprise stabilizers, buffers, antioxidants, biocides, antifoams, thickeners, antifreeze and the like.

Suitable thickeners are compounds which confer a pseudoplastic flow behavior to the formulation, i.e. high viscosity at rest and low viscosity in the agitated stage. Mention may be made, in this connection, for example, of cammercial thickeners based on polysaccharides, such as Xanthan Gum^{®} (Kelzan^{®} from Kelco), Rhodopol^{®} 23 (Rhone Poulenc) or Veegum^{®} (from R.T. Vanderbilt), or organic phyllosilicates, such as Attaclay^{®} (from Engelhardt). Antifoam agents suitable for the dispersions according to the invention are, for example, silicone emulsions (such as, for example, Silikon^{®} SRE, Wacker or Rhodorsil^{®} from Rhodia), long-chain alcohols, fatty acids, organofluorine compounds and mixtures thereof. Biocides can be added to stabilize the compositions according to the invention against attack by microorganisms. Suitable biocides are, for example, based on isothiazolones such as the compounds marketed under the trademarks Proxel^{®} from Avecia (or Arch) or Acticide^{®} RS from Thor Chemie and Kathon^{®} MK from Rohm & Haas. Suitable antifreeze agents are organic polyols, for example ethylene glycol, propylene glycol or glycerol. These are usually employed in amounts of not more than 10% by weight, based on the total weight of the active compound composition. If appropriate, the active compound compositions according to the invention may comprise 1 to 5% by weight of buffer, based on the total amount of the formulation prepared, to regulate the pH, the amount and type of the buffer used depending on the chemical properties of the active compound or the active compounds. Examples of buffers are alkali metal salts of weak inorganic or organic acids, such as, for example, phosphoric acid, boronic acid, acetic acid, propionic acid, citric acid, fumaric acid, tartaric acid, oxalic acid and succinic acid.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Powders, materials for spreading and dusts can be prepared by mixing or concomitantly grinding the active substances with a solid carrier.

Granules, for example coated granules, impregnated granules and homogeneous granules, can be prepared by binding the active ingredients to solid carriers. Examples of solid carriers are mineral earths such as silica gels, silicates, talc, kaolin, attaclay, liniestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the active ingredient. The active ingredients are employed in a purity of from 90% to 100%, preferably 95% to 100% (according to NMR spectrum).

The following are examples of formulations:

### 1. Products for dilution with water

| | |
|---|---|
| A | Soluble concentrates (SL) 10 parts by weight of a compound according to the invention are dissolved in water or in a water-soluble solvent. As an alternative, wetters or other auxiliaries are added. The active ingredient dissolves upon dilution with water. |
| | |
| B | Dispersible concentrates (DC) 20 parts by weight of a compound according to the invention are dissolved in cyclohexanone with addition of a dispersant, for example polyvinylpyrrolidone. Dilution with water gives a dispersion. |
| | |
| C | Emulsifiable concentrates (EC) 15 parts by weight of a compound according to the invention are dissolved in xylene with addition of calcium dodecylbenzenesulfonate and castor oil ethoxylate (in each case 5% strength). Dilution with water gives an emulsion. |
| | |
| D | Emulsions (EW, EO) 40 parts by weight of a compound according to the invention are dissolved in xylene with addition of calcium dodecylbenzenesulfonate and castor oil ethoxylate (in each case 5% strength). This mixture is introduced into water by means of an emulsifier (Ultraturrax) and made into a homogeneous emulsion. Dilution with water gives an emulsion. |
| | |
| E | Suspensions (SC, OD) In an agitated ball mill, 20 parts by weight of a compound according to the invention are milled with addition of dispersant, wetters and water or an organic solvent to give a fine active ingredient suspension. Dilution with water gives a stable suspension of the active ingredient. |
| | |
| F | Water-dispersible granules and water-soluble granules (WG, SG) 50 parts by weight of a compound according to the invention are ground finely with addition of dispersants and wetters and made into water-dispersible or water-soluble granules by means of technical appliances (for example extrusion, spray tower, fluidized bed). Dilution with water gives a stable dispersion or solution of the active ingredient. |
| | |
| G | Water-dispersible powders and water-soluble powders (WP, SP) 75 parts by weight of a compound according to the invention are ground in a rotor-stator mill with addition of dispersant, wetters and silica gel. Dilution with water gives a stable dispersion or solution with the active ingredient. |

### 2. Products to be applied undiluted

| | |
|---|---|
| H | Dustable powders (DP) 5 parts by weight of a compound according to the invention are ground finely and mixed intimately with 95% of finely divided kaolin. This gives a dustable product. |
| | |
| I | Granules (GR, FG, GG, MG) 0.5 parts by weight of a compound according to the invention is ground finely and associated with 95.5% carriers. Current methods are extrusion, spray drying or the fluidized bed. This gives granules to be applied undiluted. |
| | |
| J | ULV solutions (UL) 10 parts by weight of a compound according to the invention are dissolved in an organic solvent, for example xylene. This gives a product to be applied undiluted. |

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active ingredient concentrations in the ready-to-use products can be varied within relatively wide ranges. In general, they are from 0.0001 to 10%, preferably from 0.01 to 1%.

The active ingredients may also be used successfully in the ultra-low-volume process (ULV), it being possible to apply formulations comprising over 95% by weight of active ingredient, or even to apply the active ingredient without additives.

In the method of this invention compounds I may be applied with other active ingredients, for example with other pesticides, insecticides, herbicides, fertilizers such as ammonium nitrate, urea, potash, and superphosphate, phytotoxicants and plant growth regulators, safeners and nematicides. These additional ingredients may be used sequentially or in combination with the above-described compositions, if appropriate also added only immediately prior to use (tank mix). For example, the plant(s) may be sprayed with a composition of this invention either before or after being treated with other active ingredients.

The following list of pesticides together with which the compounds according to the invention can be used, is intended to illustrate the possible combinations, but not to impose any limitation:
Organo(thio)phosphates: Acephate, Azamethiphos, Azinphos-methyl, Chlorpyrifos, Chlorpyrifos-methyl, Chlorfenvinphos, Diazinon, Dichlorvos, Dicrotophos, Dimethoate, Disulfoton, Ethion, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Sulprophos, Tetrachlorvinphos, Terbufos, Triazophos, Trichlorfon;
Carbamates: Alanycarb, Bendiocarb, Benfuracarb, Carbaryl, Carbofuran, Carbosulfan, Fenoxycarb, Furathiocarb, Indoxacarb, Methiocarb, Methomyl, Oxamyl, Pirimicarb, Propoxur, Thiodicarb, Triazamate;
Pyrethroids: Allethrin, Bifenthrin, Cyfluthrin, Cyhalothrin, Cyphenothrin, Cypermethrin, alpha-Cypermethrin, beta-Cypermethrin, zeta-Cypermethrin, Deltamethrin, Esfenvalerate, Ethofenprox, Fenpropathrin, Fenvalerate, Imiprothrin, Lambda-Cyhalothrin, Permethrin, Prallethrin, Pyrethrin I and II, Silafluofen, Tau-Fluvalinate, Tefluthrin, Tetramethrin, Tralomethrin, Transfluthrin;
Growth regulators: a) chitin synthesis inhibitors: benzoylureas: Chlorfluazuron, Cyromazine, Diflubenzuron, Flucydoxuron, Flufenoxuron, Hexaflumuron, Lufenuron, Novaluron, Teflubenzuron, Triflumuron; Buprofezin, Diofenolan, Hexythiazox, Etoxazole, Clofentazine; b) ecdysone antagonists: Halofenozide, Methoxyfenozide, Tebufenozide; c) juvenoids: Pyriproxyfen, Methoprene, Fenoxycarb; d) lipid biosynthesis inhibitors: Spirodiclofen, Spiromesifen,
Neonicotinoids: Clothianidine, Dinotefuran, Imidacloprid, Thiamethoxam, Nitenpyram, Nithiazine, Acetamiprid, Thiacloprid;
Pyrazole pesticides: Acetoprole, Ethiprole, Fipronil, Tebufenpyrad, Tolfenpyrad, Vaniliprole;
Various: Abamectin, Acequinocyl, Amidoflumet, Amidrazone, Amitraz, Azadirachtin, Benclothiaz, Bifenazate, Bistrifluron, Cartap, Chlorfenapyr, Chlordimeform, Cyflumetofen, Cyromazine, Diafenthiuron, Dimefluthrin, Diofenolan, Emamectin, Endosulfan, Fenazaquin, Flonicamid, Fluacyprim, Flubendiamide, Flufenerim, Flupyrazofos, Formetanate, Formetanate hydrochloride, Hydramethylnon, Indoxacarb, Lepimectin, Metaflumizone, Milbemectin, Piperonylbutoxide, Profluthrin, Pyridaben, Pyridalyl, Pymetrozine, Pyrafluprole, Pyriprole, Spinosad, Sulfur, Tebufenpyrad, Thiocyclam, Tolfenpyrad; the pesticide of the following formula Γ¹, as described in WO 98/05638 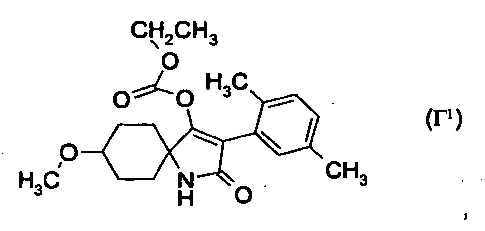 aminoisothiazole compound of formula Γ², 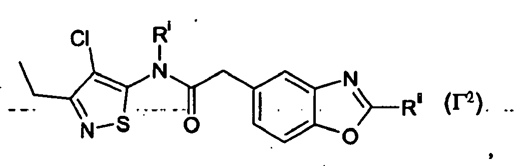 wherein R¹ is -CH₂OCH₃ or H and R^{B} is -CF₂CF₂CF₃, anthranilamide compounds of formula Γ³ 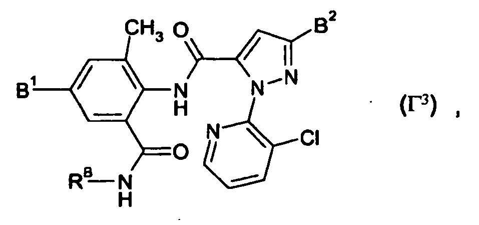 wherein B¹ is hydrogen or a chlorine atom, B² is a bromine atom or CF₃, and R^{B} is C₁-C₆-alkyl, and
compounds of the formulae 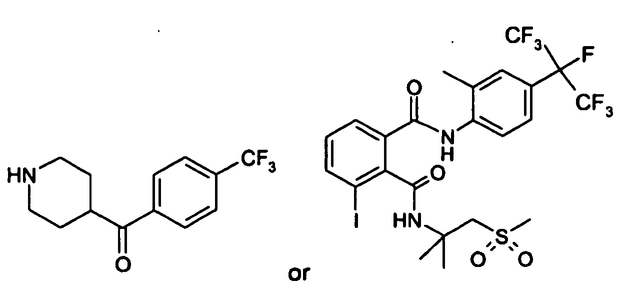 and malononitrile compounds as described in JP 2002 284608, WO 02/89579, WO 02/90320, WO 02/90321, WO 04/06677, WO 04/20399, or JP 2004 99597.

In the methods according to the invention the pests are controlled by contacting the target parasite/pest, its food supply, habitat, breeding ground or its locus with a pesticidally effective amount of compounds of formula I or with a salt thereof or with a composition, containing a pesticidally effective amount of a compound of formula I or a salt thereof.

"Locus" means a habitat, breeding ground, plant, seed, soil, area, material or environment in which a pest or parasite is growing or may grow.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

The compounds of the invention can also be applied preventively to places at which occurrence of the pests is expected.

The compounds of formula I may be also used to protect growing plants from attack or infestation by pests by contacting the plant with a pesticidally effective amount of compounds of formula I. As such, "contacting" includes both direct contact (applying the compounds/compositions directly on the pest and/or plant - typically to the foliage, stem or roots of the plant) and indirect contact (applying the compounds/compositions to the locus of the pest and/or plant).

The aforementioned compositions are particularly useful for protecting crop plants against infestation of said pests or for combating these pests in infested plants.

For use in treating crop plants, the rate of application of the active ingredients of this invention may be in the range of 0.1 g to 4000 g per hectare, desirably from 25 g to 600 g per hectare, more desirably from 50 g to 500 g per hectare.

In the case of soil treatment or of application to the pests dwelling place or nest, the quantity of active ingredient ranges from 0.0001 to 500 g per 100 m², preferably from 0.001 to 20 g per 100 m².

The compounds of formula I are also suitable for the treatment of seeds in order to protect the seed from insect pest, in particular from soil-living insect pests and the resulting plant's roots and shoots against soil pests and foliar insects.

The compounds of formula I are particularly useful for the protection of the seed from soil pests and the resulting plant's roots and shoots against soil pests and foliar insects. The protection of the resulting plant's roots and shoots is preferred. More preferred is the protection of resulting plant's shoots from piercing and sucking insects, wherein the protection from aphids is most preferred.

The present invention therefore comprises a method for the protection of seeds from insects, in particular from soil insects and of the seedlings' roots and shoots from insects, in particular from soil and foliar insects, said method comprising contacting the seeds before sowing and/or after pregermination with a compound of the general formula I or a salt thereof. Particularly preferred is a method, wherein the plant's roots and shoots are protected, more preferably a method, wherein the plants shoots are protected form piercing and sucking insects, most preferably aa method, wherein the plants shoots are protected from aphids.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

Compositions which are useful for seed treatment are e.g.:
A Soluble concentrates (SL, LS)
D Emulsions (EW, EO, ES)
E Suspensions (SC, OD, FS)
F Water-dispersible granules and water-soluble granules (WG, SG)
G Water-dispersible powders and water-soluble powders (WP, SP, WS)
H Dustable powders (DP, DS)

Preferred FS formulations of compounds of formulae I for seed treatment usually comprise from 0.1 to 80% by weight (1 to 800 g/L) of the active ingredient, from 0.1 to 20 % by weight (1 to 200 g/L) of at least one surfactant, e.g. 0.05 to 5 % by weight of a wetter and from 0.5 to 15 % by weight of a dispersing agent, up to 20 % by weight, e.g. , from 5 to 20 % of an anti-freeze agent, from 0 to 15 % by weight, e.g. 1 to 15 % by weight of a pigment and/or a dye, from 0 to 40 % by weight, e.g. 1 to 40 % by weight of a binder (sticker /adhesion agent), optionally up to 5 % by weight, e.g. from 0.1 to 5 % by weight of a thickener, optionally from.0.1 to 2 % of an anti-foam agent, and optionally a preservative such as a biocide, antioxidant or the like, e.g. in an amount from 0.01 to 1 % by weight and a filler/vehicle up to 100 % by weight.

Suitable pigments or dyes for seed treatment formulations are pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Binders, which are also referred to as stickers / adhesion agents are added to improve the adhesion of the active materials on the seeds after treatment. Suitable adhesives are block copolymers EO/PO surfactants but also polyvinylalcohols, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers and copolymers derived from these polymers.

In the treatment of seed, the application rates of the compounds I are generally from 0.1 g to 10 kg per 100 kg of seed, preferably from 1 g to 5 kg per 100 kg of seed, in particular from 1 g to 1000 g per 100 kg of seed.

The invention therefore also relates to seed comprising a compound of the formula I or an agriculturally useful salt of I, as defined herein. The amount of the compound I or the agriculturally useful salt thereof will in general vary from 0.1 g to 10 kg per 100 kg of seed, preferably from 1 g to 5 kg per 100 kg of seed, in particular from 1 g to 1000 g per 100 kg of seed.

The compounds of the invention may also be applied against non-crop insect pests, such as ants, termites, wasps, flies, mosquitos, crickets, or cockroaches. For use against said non-crop pests, compounds of formula I are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics.

The bait employed in the composition is a product which is sufficiently attractive to incite insects such as ants, termites, wasps, flies, mosquitos, crickets etc. or cock-roaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

For use in bait compositions, the typical content of active ingredient is from 0.001 weight % to 15 weight %, desirably from 0.001 weight % to 5% weight % of active compound.

Formulations of compounds of formula I as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250 °C, dimethylformamide, N-methylpyrrolidone, dimethyl sulfoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of formula I and its respective compositions can also be used in mosquito and fumigating coils, smoke cartridges, vaporizer plates or long-term vaporizers and also in moth papers, moth pads or other heat-independent vaporizer systems.
The present invention is now illustrated in further details by the following examples.

### I. Synthesis Examples

### Example 1: 3-Cyano-4-methylpyridine-2-N,N-dimethylsulfonamide

1.1 2-Thio-3-cyano-4-methylpyridine: 2.00 g (19.6 mmol) of cyanothioacetamide and 1.39 g (19.6 mmol) of crotonaldehyde were dissolved in 100 ml of dry ethanol. At room temperature, 160 mg (1.96 mmol) of sodium ethoxide were added. The resulting solution was refluxed for 7 h under a nitrogen atmosphere. After being cooled to room temperature, the reaction mixture was stirred at 0 °C for 90 min. The precipitate was collected by filtration and dried to afford to afford 0.60 g (3.79 mmol) of 2-thio-3-cyano-4-methylpyridine.
1.2 3-Cyano-4-methyl-pyridine-2-sulfonylchloride: 1.00 of 2-thio-3-cyano-4-methylpyridine were dissolved in 15 ml of a 1 N aqueous solution of HCl. At 0-5°C chlorine gas was passed through the reaction mixture for 1 h, after which the reaction mixture was stirred at 5 °C for 30 minutes. The obtained precipitate was filtered off; it washed with ice water and dried to afford 2.0 g (∼6.6 mmol) of 3-cyano-4-methyl-pyridine-2-sulfonylchloride which was used in the following reaction without further purification.
1.3 3-Cyano-4-methyl-pyridin-2-*N,N*-dimethylsulfonamide: A solution of 2.0 g (6.6 mmol) of 3-cyano-4-methylpyridinesulfonylchloride in 10 ml of tetrahydrofuran was added to a solution of 1.73 g (15.3 mmol) of dimethylamine in 20 ml of tetrahydrofuran at -5 °C. The reaction mixture was stirred at 0 °C for 2 hours. Then water was added. Tetrahydrofuran was removed from the reaction mixture by evaporation. The resulting aqueous mixture was stirred at 0 °C for 1 h. The precipitate was collected by filtration and dried. The aqueous phase was extracted three times with dichloromethane. The combined organic extracts were dried over sodium sulfate and filtered. The filtrate was concentrated in vacuo and united with the collected solid to afford all in all 1.00 g (4.22 mmol) of the title compound.

The compounds nos. 2 to 11 of the formula I with Z = N, n = 2 listed in the following table 1 were prepared by analogy to the method described in example 1.

**Table 1:**

| | | | | | | |
|---|---|---|---|---|---|---|
| 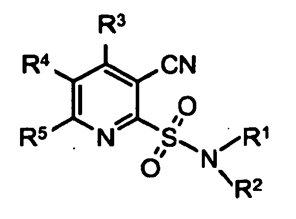 | | | | | | |

| Example no. | R¹ | R² | R³ | R⁴ | R⁵ | Physical property [m.p. [°C] RT (HPLC/MS) or chemical shift (¹H-NMR)] |
|---|---|---|---|---|---|---|
| 1. | CH₃ | CH₃ | CH₃ | H | H | 108-111 |
| 2. | CH₂-CH₃ | H | CH₃ | H | H | ¹H-NMR |
| 3. | CH₃ | H | CH₃ | H | CH₃ | 129-132 |
| 4. | CH₂-CH₃ | H | CH₃ | H | CH₃ | 134-136 |
| 5. | CH₃ | CH₃ | CH₃ | H | CH₃ | 103-108 |
| 6. | CH₂-C₆H₅ | H | CH₃ | H | CH₃ | 148-151 |
| 7. | CH₂-C₆H₅ | H | CF₃ | H | CH₃ | 127-130 |
| 8. | CH₂-CH₃ | H | CF₃ | H | CH₃ | 68-72 |
| 9 | CH₃ | CH₃ | CF₃ | H | CH₃ | 106-108 |
| 10. | CH₃ | CH₃ | CF₃ | H | C₈H₅ | 156-158 |
| 11. | CH₂CH₃ | H | CF₃ | H | C₆H₅ | ¹H-NMR |

### Example 12: 4-Cyano-5-methyl-pyridine-3-sulfonic acid dimethylamide

12.1. 5-Bromo-3,4-dimethylpyridine: 50 ml of concentrated sulfuric acid were cooled to 0 °C. 10.0 g (91.5 mmol) of 3,4-lutidin where added dropwise. The mixture was heated to 60 °C, and bromine was added slowly over a period of 30 min. After completion of the addition, the mixture was stirred at 60 °C for 90 min. After being cooled to room temperature, the reaction mixture was poured on crushed ice. The aqueous solution was washed with dichloromethane. Then the aqueous solution was cooled to 0 °C, basified with a 50 % aqueous sodium hydroxide solution and extracted three times with dichloromethane. The combined organic layers were washed with water, dried over Na₂SO₄ and evaporated under reduced pressure. The crude product was purified by recrystallization to yield 11.4 g 52.1 mmol) of the title compound.
12.2. 3-Bromo-5-methyl-pyridine-4-carbaldehyde-O-methyloxime: 2.00 g (10.75 mmol) 5-bromo-3,4-dimethylpyridine and 1.52 g (14 mmol) *n*-butyl nitrite were dissolved in 10 ml of dimethyl formamide (DMF) and cooled to -78 °C. 2.83 g (24.7 mmol) of potassium *tert*-butoxide dissolved in 10 ml of DMF were added dropwise over 10 minutes. The reaction mixture was stirred at -50 °C for 30 minutes; Then a mixture of 3 ml of glacial acetic acid and 5 ml water was added at -50 °C. The reaction mixture was warmed to room temperature and 50 ml of water were added. The precipitate was collected and dried to give 1.60 g (7.3 mmol) of the title compound.
12.3. 3-Bromo-5-methyl-isonicotinonitrile: 0.70 g (3.26 mmol) of 3-bromo-5-methylpyridine-4-carbaldehyde-O-methyloxime were dissolved in 3 ml of acetic anhydride and refluxed for 4 hours. After completion the solvent was evaporated under reduced pressure. The solid residue was dissolved in dichloromethane, washed with water and dried over Na₂SO₄. The solvent was evaporated under reduced pressure to give 0.56 g (2.8 mmol) of the title compound as a yellow solid.
12.4. 3-Methyl-5-propylsulfanyl-isonicotinonitrile: 1.00 g (5.1 mmol) of 3-bromo-5-methyl-isonicotinonitrile and 0.60 g (7.6 mmol) of propane-1-thiol were dissolved in 5 ml of DMF. At 0 °C, a solution of 0.67 g (10.2 mmol) potassium hydroxide in 2 ml of water was added drop wise. The reaction mixture was stirred at 0 °C for 2 hours before 20 ml of water was added. The resulting precipitate was collected. The solid was washed with water and dried to give 0.95 g (4.7 mmol) of the title compound as a colorless solid having a melting point of 37-39 °C.
12.5. 4-Cyano-5-methyl-pyridin-2-sulfonylchloride: 0.9 g (4.7 mmol) of 2-thio-3-cyano-4-methylpyridine were dissolved in 15 ml of a 1 N aqueous solution of HCl. At 0-5 °C chlorine gas was passed through the reaction mixture for 1 h. Then the reaction mixture was stirred at 5 °C for 30 minutes. The emerging precipitate was filtered off; washed with ice water and dried to afford 1.0 g (-4.6 mmol) of the title compound, which was used in the following reaction without further purification.
12.6 4-Cyano-5-methylpyridine-3-sulfonic acid dimethylamide: A solution of 1.0 g (4.6 mmol) of 4-cyano-5-methylpyridin-2-sulfonylchloride in 10 ml of tetrahydrofuran was added to a solution of 1.35 g (12 mmol) of dimethylamine in 20 ml of tetrahydrofuran at -5 °C. The-reaction mixture was stirred at 0 °C for 2 hours after which water was added. Tetrahydrofuran was removed from the reaction mixture by evaporation. The remaining aqueous mixture was extracted three times with dichloromethane. The combined organic extracts were dried over Na₂SO₄ and filtered. The filtrate was concentrated in vacuo. The residue was purified by flash chromatography to yield 0.23 g (0.97 mmol) of the title compound having a melting point of 128-131 °C.

### Example 38: 4-Cyano-5-difluormethoxypyridine-3-sulfonic acid dimethylamide

38.1. 3,5-Dichloro-isonicotinonitrile: 30.0 g (170.5 mmol) 3,5-dichloro-4-pyridine carbaldehyde were dissolved in 200 ml of formic acid. 15.4 g (221.6 mmol) of hydro xylamine hydrochloride and 2 drops of concentrate sulfuric acid were added and the resulting mixture was refluxed for 3.5 hours. The reaction mixture was cooled to room temperature, the solvent was evaporated under reduced pressure and the residue was redissolved in methyl *tert*-butyl ether. The resulting solution was washed with water, aqueous saturated solution of NaHCO₃ and again with water. The organic phase was dried over Na₂SO₄ and the solvent was evaporated under reduced pressure to give 28.3 g (163.6 mmol) of the title compound which was used in the following reaction without further purification.
38.2. 3-Chloro-5-propylsulfanylisonicotinonitrile: At -78 °C, a solution of 6.47 g (85 mmol) of propane-1-thiol in 100 ml of dry tetrahydrofuran was treated with 53.1 ml (85 mmol) of a 1.6 molar solution of n-butyl lithium in hexane. The resulting milky white suspension was allowed to warm to room temperature and stirred for additional 15 minutes. In a separate flask, 14.0 g (80.9 mmol) of 3,5-dichloroisonicotinonitrile were dissolved in 100 ml of dry tetrahydrofuran. At room temperature, the lithium thiopropylate solution was slowly added over a period of 30 minutes. After the addition was complete, the resulting solution was stirred for further 1 hour, after which the reaction was quenched by addition of 200 ml of saturated aqueous NH₄Cl solution. After addition of ethyl acetate, the phases were separated, and the aqueous layer was extracted three times with ethyl acetate. The combined organic layers were washed with saturated brine, dried over Na₂SO₄ and evaporated. The residue slowly crystallized to give 16.95 g (79.7 mmol) of the title compound without further purification.
38.3. 3-Methoxy-5-propylsulfanyl-isonicotinonitrile: 15.2 g (84.3 mmol) of a 30 % by wheight solution of sodium methylate in methanol were dissolved in 50 ml of dry tetrahydrofuran. 17.9 g (84.3 mmol) of 3-chloro-5-propylsulfanyl-isonicotinonitrile dissolved in 150 ml of dry tetrahydrofuran were added at room temperature. The resulting mixture was refluxed for 2.5 hours. Then the mixture was cooled to room temperature and water was added. The aqueous mixture was extracted three times with ethyl acetate. The combined organic layers were washed with saturated brine, dried over Na₂SO₄ and evaporated. The residue was purified by flash chromatography to give 7.0 g (33.6 mmol) of the title compound as a colourless solid.
38.4. 3-Hydroxy-5-propylsulfanyl-isonicotinonitrile: 9.4 g (45.1 mmol) of 3-methoxy-5-propylsulfanyl-isonicotinonitrile and 90.0 g of pyridinium chloride were placed in a flask. The mixture was heated until reflux. After having refluxed the mixture for 10 minutes, it was cooled to room temperature and 1 l of water was added. The aqueous mixture was extracted three times with dichloromethane. The combined organic layers were washed with water, dried over Na₂SO₄ and evaporated to give 6.2 g (31.9 mmol of the title compound, which was used in the following reaction without further purification.
38.5. 3-Difluormethoxy-5-propylsulfanyl-isonicotinonitrile: 1.0 g (5.2 mmol) of the crude 3-hydroxy-5-propylsulfanyl-isonicotinonitrile were dissolved in 20 ml of DMF. 3.56 g (25.7 mmol) of K₂CO₃ were added. 0.81 g (6.2 mmol) of gaseous bromodifluoromethane were added and the resulting mixture was stirred at room temperature for 5 h. Additional 0.81 g (6.2 mmol) of gaseous bromodifluoromethane were added and stirring was continued overnight. The mixture was poured into water and extracted three times with methyl telt-butyl ether. The combined organic layers were washed with brine, dried over Na₂SO₄ and evaporated. The residue was purified by flash chromatography to give 0.46 g (1.89 mmol) of the title compound.
38.6 4-Cyano-5-difluormethoxypyridine-3-sulfonylchloride: 1.80 g (7.4 mmol) of 3-difluormethoxy-5-propylsulfanylisonicotinonitrile were dissolved in 30 ml of dichloromethane and 3 ml of water. At 0 °C chlorine gas was passed through the reaction mixture for 1 h, whereupon the reaction mixture was stirred at 5 °C for 30 minutes. The reaction was quenched by addition of water. After phase separation, the aqueous layer was extracted three times with dichloromethane. The combined organic layers were washed with water and dried over Na₂SO₄ to afford 2.0 g (-6.6 mmol) of the title compound, which was used in the followin reaction without further purification.
38.7. 4-Cyano-5-difluormethoxypyridine-3-sulfonic acid dimethylamide: A solution of 1.10 g (4.1 mmol) of 4-cyano-5-difluormethoxy-pyridin-3-sulfonylchloride in 10 ml of tetrahydrofuran was added to a solution of 1.15 g (10.2 mmol) of dimethylamine in 20 ml of tetrahydrofuran at 0 °C. The reaction mixture was stirred at 0 °C for 2 hours whereupon water was added. Tetrahydrofuran was removed from the reaction mixture by evaporation. The resulting aqueous mixture was extracted three times with dichloromethane. The combined organic extracts were dried over Na₂SO₄ and filtered. The filtrate was concentrated in vacuo. The residue was purified by flash chromatography to give 0.13 g (0.47 mmol) of the title compound.

The compounds nos. 12 to 41 of the formula I with Y = N and n = 2 listed in the following table 2 were prepared by analogy to the methods described in examples 12 and 38.

**Table 2:**

| | | | | | | |
|---|---|---|---|---|---|---|
| 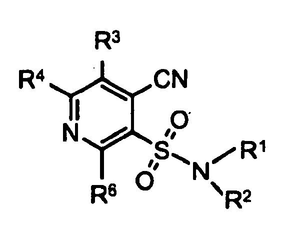 | | | | | | |

| Example no. | R¹ | R² | R³ | R⁴ | R⁶ | m.p. [ °C] or ¹H-NMR |
|---|---|---|---|---|---|---|
| 12. | CH₃ | CH₃ | CH₃ | H | H | 128-131 |
| 13. | CH₂-CH₃ | H | CH₃ | H | H | 96-100 |
| 14. | CH₃ | CH₃ | Cl | H | H | 97-100 |
| 15. | CH₂-CH₃ | CH₂-CH₃ | Cl | H | H | 94-97 |
| 16. | CH₃ | CH₂-CH₃ | Cl | H | H | ¹H-NMR |
| 17. | CH-(CH₃)₂ | H | Cl | H | H | ¹H-NMR |
| 18. | CH₂-CH₂-O-CH₃ | H | Cl | H | H | 127-129 |
| 19. | H | H | Cl | H | H | ¹H-NMR |
| 20. | CH₃ | CH₃ | OCH₃ | H | H | 134-138 |
| 21. | CH₃ | CH₂-C≡CH | OCH₃ | H | H | ¹H-NMR |
| 22. | CH₃ | CH₂-CH₃ | OCH₃ | H | H | ¹H-NMR |
| 23. | CH₃ | OCH₃ | OCH₃ | H | H | 143-152 |
| 24. | CH₃ | C(=O)-CH₃ | OCH₃ | H | H | 142-147 |
| 25. | CH₂-CH₃ | CH₂-CH₃ | OCH₃ | H | H | 93-95 |
| 26. | CH₂-CH₃ | C(=O)-CH₃ | OCH₃ | H | H | ¹H-NMR |
| 27. | CH₂-CH₃ | H | OCH₃ | H | H | 182-185 |
| 28. | CH-(CH₃)₂ | H | OCH₃ | H | H | 182-186 |
| 29. | CH-(CH₃)₂ | C(=O)-CH₃ | OCH₃ | H | H | ¹H-NMR |
| 30. | CH₂-C≡CH | H | OCH₃ | H | H | 164-165 |
| 31. | CH₂-C₆H₅ | H | OCH₃ | H | H | 139-142 |
| 32. | CH₂C₆H₅ | C(=O)-CH₃ | OCH₃ | H | H | ¹H-NMR |
| 33. | CH₂-C=CH₂ | H | OCH₃ | H | H | 137-144 |
| 34. | CH₂-C=CH₂ | C(=O)-CH₃ | OCH₃ | H | H | 110-115 |
| 35. | cyclohexylamine# | --- | OCH₃ | H | H | 142-148 |
| 36. | morpholine# | --- | OCH₃ | H | H | 186-192 |
| 37. | CH₃ | CH₃ | OH | H | H | 177-178 |
| 38. | CH₃ | CH₃ | OCHF₂ | H | H | ¹H-NMR |
| 39. | CH₃ | CH₂-CH₃ | OCHF₂ | H | H | ¹H-NMR |
| 40. | CH₃ | CH₂-C=CH | OCHF₂ | H | H | ¹H-NMR |
| 41. | CH₃ | CH-(CH₃)₂ | OCHF₂ | H | H | ¹H-NMR |

| | | | | | | |
|---|---|---|---|---|---|---|
| # R¹ and R² together with the nitrogen atom to which they are attached | | | | | | |

Some compounds were characterized by ¹H-NMR. The signals are characterized by chemical shift (ppm) vs, tetramethylsilane, by their multiplicity and by their integral (relative number of hydrogen atoms given). The following abbreviations are used to characterize the multiplicity of the signals: M = multiplett, q = quartett, to triplett, d = doublet and s = singulett.

| | |
|---|---|
| Example 2: | 1.20 (t, 3 H), 2.70 (s, 3 H), 5.1 (br. s, 1 H), 7.45 (d, 1H), 8.65 (d, 1 H), CDCl₃ |
| Example 11: | 1.25 (t, 3 H), 3.35 (m, 2 H), 5.5 (br. s, 1H), 7.5-7.65 (m, 5 H), 8.05 (s, 1 H), CDCl₃ |
| Example 16: | 1.25 (m, 3 H), 2.95 (s, 3 H), 3.40 (q, 2 H), 9.00 (s, 1H), 9.10 (s, 1 H), CDCl₃ |
| Example 17: | 1.20 (d, 6 H), 3.65 (m, 1 H), 5.75 (br. s, 1 H), 9.0 (s, 1 H), 9.20 (s, 1 H), CDCl₃ |
| Example 19: | 8.95 (s, 1H), 9.1 (s, 1 H), CD₃OD |
| Example 21; | 2.15 (s, 1 H). 3.0 (s, 3 H), 4.15 (s, 3 H), 4.20 (s, 2 H), 8.70 (s, 1 H), 8.85 (s, 1H), CDCl₃ |
| Example 22: | 1.20 (t, 3 H), 2.95 (s, 3 H), 3.35 (q, 2 H), 4.15 (s, 3H), 8.65 (s, 1 H), 8.85 (s, 1 H), CDCl₃ |
| Example 26: | 1.40 (m, 3 H), 2.30 (s, 3 H), 3.10 (q, 2 H), 4.15 (s, 3 H), 8.70 (s, 1 H), 9.0 (s, 1 H), CDCl₃ |
| Example 29: | 1.45 (d, 6 H), 2.45 (s, 3 H), 4.15 (s, 3 H), 4.35 (m, 1 H), 8.75 (s, 1 H), 8.95 (s, 1 H), CDCl₃ |
| Example 32: | 2.15 (s, 3 H), 4.1 (s, 3 H), 5.20 (s, 2 H), 7.2-7.45 (m, 5 H), 8.65 (s, 1 H), 9.0 (s, 1 H), CDCl₃ |
| Example 38: | 2.95 (s, 6 H), 6.85 (t, 1 H), 8.95 (s, 1 H), 9.05 (s, 1 H), CDCl₃ |
| Example 39: | 1.2 (t, 3 H), 2.95 (s, 3 H), 3.35 (m, 2 H), 6.8 (t, 1 H), 8.95 (s, 1 H), 9.15 (s, 1 H), CDCl₃ |
| Example 40: | 2.15 (s, 1 H), 3.05 (s, 3 H), 4.20 (s, 2 H), 6.85 (t, 1 H), 8.95 (s, 1 H), 9.15 (s, 1 H), CDCl₃ |
| Example 41: | 1.15 (d, 6 H), 2.90 (s, 3 H), 4.25 (m, 1 H), 6.75 /t, 1 H), 8.95 (s, 1 H), 9.15 (s, 1H), CDCl₃ |

### II. Examples of action against pests

The active conmpounds were formulated in a mixture of 50 vol.-% acetone:50 vol.-% water. A nonionic surfactant (Kinetic®) was included in the solution at a volume of 0.01% v/v.

In the following tests, the formulated solutions of the active compounds were diluted to an active ingredient concentration of 300 ppm and the diluted solutions were applied in the below mentioned tests.

The action of the compounds of the formula I against pests was demonstrated by the following experiments:

### II.1 Cotton aphid (aphis gossypii), mixed life stages

Cotton plants at the cotyledon stage were infested prior to treatment by placing a heavily infested leaf from the main aphid colony on top of each cotyledon. The aphids were allowed to transfer overnight and the host leaf was removed. The infested cotyledons were then dipped and agitated in the test solution for 3 seconds and allowed to dry in a fume hood. Test plants were maintained under fluorescent lighting in a 24-hr photoperiod at 25°C and 20-40% relative humidity. Aphid mortality on the treated plants, relative to mortality on untreated check plants, was determined after 5 days.

In this test compounds each of examples 12 to 36, 38, 39, 40 and 41 at 300 ppm provided at least 90 % mortality of cotton aphid (*Aphis gossypii,* mixed life stages) in comparison with untreated controls.

### 11.2 Green Peach Aphid (Myzus persicae), mixed life stages

Bell pepper plants at the first true-leaf stage were infested prior to treatment by placing heavily infested leaves from the main aphid colony on top of the treatment plants. The aphids were allowed to transfer overnight to accomplish an infestation of 30-40 aphids per plant and the host leaves were removed. The infested leaves of the test plants were then dipped and agitated in the test solution for 3 seconds and allowed to dry in a fume hood. Test plants were maintained under fluorescent lighting in a 24-hr photoperiod at 25°C and 20-40% relative humidity. Aphid mortality on the treated plants, relative to mortality on untreated check plants, was determined after 5 days.

In this test compounds each of examples 12 to 36, 38, 39, 40 and 41 at 300 ppm provided at least 90 % mortality of green peach aphidin comparison with untreated controls.

### II.3 Bean Aphid (Aphis fabae)

Nasturtium plants grown in Metro mix in the 1^{st} leaf-pair stage (variety 'Mixed Jewel') were infested with approximately 2-30 laboratory-reared aphids by placing infested cut plants on top of the test plants. The cut plants were removed after 24 hr. Each plant was dipped into the test solution to provide complete coverage of the foliage, stem, protruding seed surface and surrounding cube surface and allowed to dry in the fume hood. The treated plants were kept at about 25°C with continuous fluorescent light. Aphid mortality is determined after 3 days.

In this test compounds each of examples 12 to 36, 38, 39, 40 and 41 at 300 ppm provided at least 90 % mortality of bean aphid in comparison with untreated controls.

## Claims

1. A cyanopyridine compound of the formula I 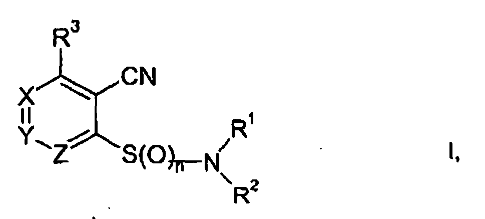 where
n is 0, 1 or 2;
X is N, N-O or C-R⁴;
Y is N, N-O or C-R⁵;
Z is N, N-O or C-R⁶;
with the proviso that one of the variables X, Y and Z is N or NO and the other two variables are optionally substituted carbon atoms;
R¹, R² are, independently of one another, selected from the group consisting of hydrogen, C(=O)-R⁷, C₁-C₁₀-alkyl, C₂-C₆-alkenyl, C₂-C₁₀-alkinyl, C₁-C₁₀-alkoxy or C₃-C₁₀-cycloalkyl, wherein the five last-mentioned radicals may be unsubstituted, partially or fully halogenated and/or may carry 1, 2 or 3 radicals, independently of one another each selected from the group consisting of cyano, nitro, amino, C₁-C₁₀-alkoxy, C₁-C₁₀-alkylthio, C₁-C₁₀-alkylsulfinyl, C₁-C₁₀-alkylsulfonyl, C₁-C₁₀-haloalkoxy, C₁-C₁₀-haloalkylthio, C₁-C₁₀-alkoxycarbonyl, (C₁-C₁₀-alkyl)amino, di-(C₁-C₁₀-alkyl)amino, C₃-C₁₀-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy;
or
R¹ and R² together with the adjacent nitrogen form a 3 to 10-membered ring, optionally substituted by 1, 2 or 3 radicals selected from C₁-C₅-alkyl and halogen, wherein the ring may contain, in addition to the nitrogen and carbon ring members, 1, 2 or 3 heteroatoms as ring members selected from the group consisting of nitrogen, oxygen, sulfur, a group SO, SO₂ or N-R⁶;
R³ is hydrogen, nitro, cyano, azido, amino, halogen, sulfonylamino, sulfenylamino, sulfinylamino, C(=O)R⁸,
C₁-C₆-alkyl, C₂-C₆-alkenyl, C₂-C₆-alkinyl, C₃-C₈-cycloalkyl, C₁-C₆-alkoxy, C₁-C₆-alkylthio, (C₁-C₆-alkyl)amino, di(C₁-C₆-alkyl)amino, C₁-C₆-alkylsulfinyl; or C₁-C₆-alkylsulfonyl, wherein the ten last-mentioned radicals may be unsubstituted, partially or fully halogenated and/or may carry 1, 2 or 3 radicals, selected from the group consisting of cyano, nitro, amino, C₁-C₄-alkoxy, C₁-C₄-alkylthio, C₁-C₄-alkylsulfinyl, C₁-C₄-alkyfsulfonyl, C₁-C₄-haloalkoxy, C₁-C₄-haloalkylthio, (C₁-C₄-alkoxy)carbonyl, (C₁-C₄-alkyl)amino, di(C₁-C₄-alkyl)amino, C₃-C₈-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy;
R⁴, R⁵ and R⁶ are independently of one another selected from the group consisting of hydrogen, halogen, cyano, azido, nitro, C₁-C₆-alkyl, C₃-C₈-cycloalkyl, C₁-C₄-haloalkyl, C₁-C₄-alkoxy, C₁-C₄-alkylthio, C₁-C₄-alkylsulfinyl, C₁-C₄-alkylsulfonyl, C₁-C₄-haloalkoxy, C₁-C₄-haloalkylthio, C₂-C₆-alkenyl, C₂-C₆-alkinyl, (C₁-C₄-alkoxy)carbonyl, amino, (C₁-C₄-alkyl)amino, di(C₁-C₄-alkyl)amino, aminocarbonyl, (C₁-C₄-alkyl)aminocarbonyl, di(C₁-C₄-alkyl)aminocarbonyl, hydroxysulfonyl, sulfonylamino, sulfenylamino, sulfinylamino and C(=O)-R¹⁰;
R⁷ is C₁-C₆-alkyl, C₁-haloalkyl, aryl, aryl-C₁-C₄-alkyl,
3- to 7-membered heteroaryl or heteroaryl-C₁-C₄-alkyl, wherein the heteroaryl ring contains as ring members 1,2 or 3 heteroatoms, selected from the group consisting of nitrogen, oxygen, sulfur, a group SO, SO₂ or N-R¹¹, wherein R¹¹ is hydrogen or C₁-C₄-alkyl;
3- to 7-membered heterocyclyl or heterocyclyl-C₁-C₄-alkyl, wherein the heterocyclic ring contains as ring members 1, 2 or 3 heteroatoms, selected from the group consisting of nitrogen, oxygen, sulfur, a group SO, SO₂ or N-R¹², wherein R¹² is hydrogen or C₁-C₄-alkyl;
and wherein the carbon atoms of the heterocyclic rings may by unsubstituted or substituted by 1 or 2 C₁-C₄-alkyl groups;
R⁸ is hydrogen or C₁-C₄-alkyl;
R⁹ and R¹⁰, independently of one another, are hydrogen, hydroxy, C₁-C₆-alkoxy, amino, C₁-C₄-alkyl, aryl, aryl-C₁-C₄-alkyl,
3- to 7-membered heteroaryl or heteroaryl-C₁-C₄-alkyl, wherein the heteroaryl ring contains as ring members 1, 2 or 3 heteroatoms, selected from the group consisting of nitrogen, oxygen, sulfur, a group SO, SO₂ or N-R¹³, wherein R¹³ is hydrogen or C₁-C₄-alkyl;
3- to 7-membered heterocyclyl or heterocyclyl C₁-C₄-alkyl, wherein the heterocyclic ring contains 1, 2 or 3 heteroatoms, selected from the group consisting of nitrogen, oxygen, sulfur, a group SO, SO₂ or N-R¹⁴, wherein R¹⁴ is hydrogen or C₁-C₄-alkyl;
and wherein the carbon atoms of the heterocyclic rings may by unsubstituted or substituted by 1 or 2 C₁-C₄-alkyl groups;
and/or the agriculturally acceptable salts thereof,
except for
the compounds of formula I, where n is 0; X is CR⁴; Y is CR⁵; Z is N; R¹, R² and R⁴ are each hydrogen; R⁵ is CH₃; and R³ is hydrogen, CH₃ or C(=O)OC₂H₅; and
the compound of formula I, where n is 2; X is CR⁴; Y is CR⁵; Z is N; R¹, R² and R⁴ are each hydrogen; and R³ and R⁵ each are CH₃.

2. A compound of the formula I as defined in claim 1, wherein n is 2.

3. A compound of the formula I as defined in claim 1 or 2, wherein X is C-R⁴, Y is N or NO and Z is C-R⁶.

4. A compound according to claim 3, wherein either R⁴ or R⁶ is hydrogen and the other radical R⁴ or R⁶ is selected from halogen, C₁-C₄-alkyl, or C₁-C₄-haloalkyl.

5. A compound according to claim 3, wherein both R⁴ and R⁶ are hydrogen.

6. A compound of the formula I as defined in claim 1 or 2, wherein X is C-R⁴, Y is C-R⁵ and Z is N or NO.

7. A compound of the formula I as defined in claim 1, wherein R⁹ is halogen, C₁-C₄-alkyl, C₁-C₄-haloalkyl, C₁-C₄-alkoxy or C₁-C₄-heloalkoxy.

8. A compound of the formula I as defined in claim 1, wherein R¹ and R² are, independently of one another, selected from the group consisting of hydrogen, C₁-C₆-alkyl, C₁-C₆-haloalkyl, C₂-C₈-alkenyl, C₂-C₆-haloalkenyl, C₂-C₆-alkinyl, C₂-C₆-haloalklnyl, C₃-C₆-cycloalkyl, C₁-C₄alkoxy-C₁-C₄alkyl and C₁-C₄-alkylthio-C₁-C₄-alkyl, wherein R² may also form a radical C(=O)-R⁷, wherein R⁷ is as defined in claim 1.

9. An agricultural compositions comprising such an amount of at least one compound of the formula I and/or at least one agriculturally useful salt of I. as defined in claim 1, and at least one inert liquid and/or solid agronomically acceptable carrier, wherein a method of treatment of humans is excluded that has a pesticidal action and, if desired, at least one surfactant.

10. The use of a cyanopyridine compound of the formula 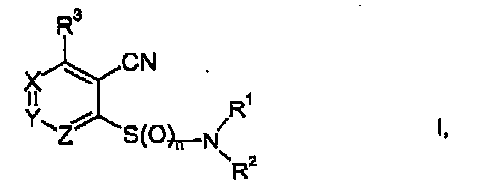 where the index n and the substituents R¹, R², R³, X, Y and Z are as defined In claim 1 or a salt thereof for combating animal pests.

11. A method of combating animal pests which comprises contacting the animal posts, their habit, breeding ground, food supply, plant, seed, soil, area, material or environment in which the animal pests are growing or may grow, or the materials, plants, seeds, soils, surfaces or spaces to be protected from animal attack or infestation with a pesticidally effective amount of at least one compound of the formula I and/or at least one agriculturally acceptable salt thereof, as defined in claim 10, wherein a method of treatment of humans is excluded.

12. A method for protecting crops from attack or infestation by animal pests, which comprises contacting a crop with a pesticidally effective amount of at least one compound of the formula I and/or at least one salt thereof, as defined in claim 10.

13. A method for the protection of seeds from soil insects and of the seedlings' roots and shoots from insects comprising contacting the seeds before sowing and/or after pregermination with a compound of the formula I as defined in claim 10 and/or at least one agriculturally acceptable salt thereof, as defined in claim 10, in pesticidally effective amounts.

14. Seed comprising a compound of the formula I or an agriculturally useful salt of I, as defined in claim 10, in an amount of from 0.1 g to 10 kg per 100 kg of seed.

15. A cyanopyridine sulfonyl halide of the formula II 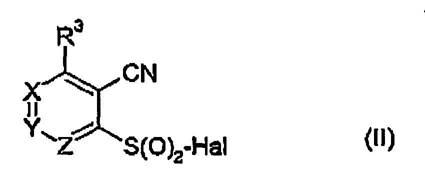 wherein X, Y, Z and R³ are as defined in claim 1 and Hal is halogen,
except for
the compound of formula II, where Hal is chlorine, X is CR⁴, Y is CR⁵, Z is N, R⁴ is hydrogen and R³ and R⁵ are each CH₃.

## Patentansprüche

1. Cyanopyridin-Verbindung der Formel I 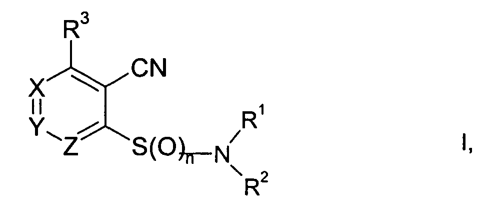 worin
n für 0, 1 oder 2 steht;
X für N, N-O oder C-R⁴ steht;
Y für N, N-O oder C-R⁵ steht;
Z für N, N-O oder C-R⁶ steht;
mit der Maßgabe, dass eine der Variablen X, Y und Z für N oder NO steht und die anderen zwei Variablen für gegebenenfalls substituierte Kohlenstoffatome stehen;
R¹, R² unabhängig voneinander ausgewählt sind unter Wasserstoff, C(=O)-R⁷, C₁-C₁₀-Alkyl, C₂-C₆-Alkenyl, C₂-C₁₀-Alkinyl, C₁-C₁₀-Alkoxy oder C₃-C₁₀-Cycloalkyl, wobei die fünf letztgenannten Reste unsubstituiert, partiell oder vollständig halogeniert sein können und/oder 1, 2 oder 3 Reste aufweisen können, die unabhängig voneinander ausgewählt sind unter Cyano, Nitro, Amino, C₁-C₁₀-Alkoxy, C₁-C₁₀-Alkylthio, C₁-C₁₀-Alkylsulfinyl, C₁-C₁₀-Alkylsulfonyl, C₁-C₁₀-Halogenalkoxy, C₁-C₁₀-Halogenalkylthio, C₁-C₁₀-Alkoxycarbonyl, (C₁-C₁₀-Alkyl)amino, di-(C₁-C₁₀-Alkyl)amino, C₃-C₁₀-Cycloalkyl und Phenyl, wobei Phenyl unsubstituiert, partiell oder vollständig halogeniert sein kann und/oder 1, 2 oder 3 Substituenten aufweisen kann, die unabhängig voneinander unter C₁-C₄-Alkyl, C₁-C₄-Halogenalkyl, C₁-C₄-Alkoxy und C₁-C₄-Halogenalkoxy ausgewählt sind;
oder
R¹ und R² zusammen mit dem benachbarten Stickstoff einen 3- bis 10-gliedrigen Ring bilden, der gegebenenfalls mit 1, 2 oder 3 unter C₁-C₅-Alkyl und Halogen ausgewählten Resten substituiert ist, wobei der Ring neben dem Stickstoff und den Kohlenstoffringgliedern 1, 2 oder 3, unter Stickstoff, Sauerstoff, Schwefel, einer Gruppe SO, SO₂ oder N-R⁸ ausgewählte Heteroatome als Ringglieder enthalten kann;
R³ für Wasserstoff, Nitro, Cyano, Azido, Amino, Halogen, Sulfonylamino, Sulfenylamino, Sulfinylamino, C(=O)R⁹,
C₁-C₆-Alkyl, C₂-C₆-Alkenyl, C₂-C₆-Alkinyl, C₃-C₈-Cycloalkyl, C₁-C₆-Alkoxy, C₁-C₆-Alkylthio, (C₁-C₆-Alkyl)amino, di(C₁-C₆-Alkyl)amino, C₁-C₆-Alkylsulfinyl oder C₁-C₆-Alkylsulfonyl steht, wobei die zehn letztgenannten Reste unsubstituiert, partiell oder vollständig halogeniert sein können und/oder 1, 2 oder 3, unter Cyano, Nitro, Amino, C₁-C₄-Alkoxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, (C₁-C₄-Alkoxy)carbonyl, (C₁-C₄-Alkyl)amino, di(C₁-C₄-Alkyl)amino, C₃-C₈-Cycloalkyl und Phenyl ausgewählte Reste tragen können, wobei Phenyl unsubstituiert, partiell oder vollständig halogeniert sein kann und/oder 1, 2 oder 3, unabhängig voneinander unter C₁-C₄-Alkyl, C₁-C₄-Halogenalkyl, C₁-C₄-Alkoxy und C₁-C₄-Halogenalkoxy ausgewählte Substituenten aufweisen kann;
R⁴, R⁵ und R⁶ unabhängig voneinander unter Wasserstoff, Halogen, Cyano, Azido, Nitro, C₁-C₆-Alkyl, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, C₂-C₆-Alkenyl, C₂-C₆-Alkinyl, (C₁-C₄-Alkoxy)carbonyl, Amino, (C₁-C₄-Alkyl)amino, di(C₁-C₄-Alkyl)amino, Aminocarbonyl, (C₁-C₄-Alkyl)aminocarbonyl, di(C₁-C₄-Alkyl)aminocarbonyl, Hydroxysulfonyl, Sulfonylamino, Sulfenylamino, Sulfinylamino und C(=O)-R¹⁰ ausgewählt sind;
R⁷ für C₁-C₆-Alkyl, C₁-Halogenalkyl, Aryl, Aryl-C₁-C₄-alkyl,
3- bis 7-gliedriges Heteroaryl oder Heteroaryl-C₁-C₄-alkyl, worin der Heteroarylring 1, 2 oder 3, unter Stickstoff, Sauerstoff, Schwefel, einer Gruppe SO, SO₂ oder N-R¹¹ ausgewählte Heteroatome als Ringglieder enthält, wobei R¹¹ für Wasserstoff oder C₁-C₄-Alkyl steht;
3- bis 7-gliedriges Heterocyclyl oder Heterocyclyl-C₁-C₄-alkyl steht, worin der heterocyclische Ring 1, 2 oder 3, unter Stickstoff, Sauerstoff, Schwefel, einer Gruppe SO, SO₂ oder N-R¹² ausgewählte Heteroatome als Ringglieder enthält, worin R¹² für Wasserstoff oder C₁-C₄-Alkyl steht;
und worin die Kohlenstoffatome des heterocyclischen Rings unsubstituiert oder mit 1 oder 2 C₁-C₄-Alkylgruppen substituiert sein können;
R⁸ für Wasserstoff oder C₁-C₄-Alkyl steht;
R⁹ und R¹⁰, unabhängig voneinander für Wasserstoff, Hydroxy, C₁-C₆-Alkoxy, Amino, C₁-C₄-Alkyl, Aryl, Aryl-C₁-C₄-alkyl,
3- bis 7-gliedriges Heteroaryl oder Heteroaryl-C₁-C₄-alkyl, wobei der Heteroarylring 1, 2 oder 3, unter Stickstoff, Sauerstoff, Schwefel, einer Gruppe SO, SO₂ oder N-R¹³ ausgewählte Heteroatome enthält, wobei R¹³ für Wasserstoff oder C₁-C₄-Alkyl steht;
3- bis 7-gliedriges Heterocyclyl oder Heterocyclyl-C₁-C₄-alkyl stehen, wobei der heterocyclische Ring 1, 2 oder 3, unter Stickstoff, Sauerstoff, Schwefel, einer Gruppe SO, SO₂ oder N-R¹⁴ ausgewählte Heteroatome enthält, worin R¹⁴ für Wasserstoff oder C₁-C₄-Alkyl steht;
und worin die Kohlenstoffatome des heterocyclischen Rings unsubstituiert oder mit 1 oder 2 C₁-C₄-Alkylgruppen substituiert sein können;
und/oder die landwirtschaftlich verträglichen Salze davon,
ausgenommen
die Verbindungen der Formel I, worin n für 0 steht; X für CR⁴ steht; Y für CR⁵ steht; Z für N steht; R¹, R² und R⁴ jeweils für Wasserstoff stehen; R⁵ für CH₃ steht; und R³ für Wasserstoff, CH₃ oder C(=O)OC₂H₅ stehen; und
die Verbindungen der Formel I, worin n für 2 steht; X für CR⁴ steht; Y für CR⁵ steht; Z für N steht; R¹, R² und R⁴ jeweils für Wasserstoff stehen; und R³ und R⁵ jeweils für CH₃ stehen.

2. Verbindung der Formel I gemäß Anspruch 1, worin n für 2 steht.

3. Verbindung der Formel I gemäß Anspruch 1 oder 2, worin X für C-R⁴ steht, Y für N oder NO steht und Z für C-R⁶ steht.

4. Verbindung gemäß Anspruch 3, worin entweder R⁴ oder R⁶ für Wasserstoff steht und der andere Rest R⁴ oder R⁶ unter Halogen, C₁-C₄-Alkyl oder C₁-C₄-Halogenalkyl ausgewählt ist.

5. Verbindung gemäß Anspruch 3, worin sowohl R⁴ als auch R⁶ für Wasserstoff stehen.

6. Verbindung der Formel I gemäß Anspruch 1 oder 2, worin X für C-R⁴ steht, Y für C-R⁵ steht und Z für N oder NO steht.

7. Verbindung der Formel I gemäß Anspruch 1, worin R³ für Halogen, C₁-C₄-Alkyl, C₁-C₄-Halogenalkyl, C₁-C₄-Alkoxy oder C₁-C₄-Halogenalkoxy steht.

8. Verbindung der Formel I gemäß Anspruch 1, worin R¹ und R² unabhängig voneinander unter Wasserstoff, C₁-C₆-Alkyl, C₁-C₆-Halogenalkyl, C₂-C₆-Alkenyl, C₂-C₆-Halogenalkenyl, C₂-C₆-Alkinyl, C₂-C₆-Halogenalkinyl, C₃-C₆-Cycloalkyl, C₁-C₄-Alkoxy-C₁-C₄-alkyl und C₁-C₄-Alkylthio-C₁-C₄-alkyl ausgewählt sind, worin R² auch für einen Rest C(=O)-R⁷ stehen kann, worin R⁷ die in Anspruch 1 genannten Bedeutungen aufweist.

9. Landwirtschaftliche Zusammensetzung, enthaltend eine solche Menge wenigstens einer Verbindung der Formel I und/oder wenigstens eines landwirtschaftlich brauchbaren Salzes von I gemäß Anspruch 1 und wenigstens einen inerten flüssigen und/oder festen landwirtschaftlich verträglichen Trägerstoff, dass sie eine pestizide Wirkung zeigt, und gewünschtenfalls, wenigstens einen oberflächenaktiven Stoff.

10. Verwendung einer Cyanopyridin-Verbindung der Formel I 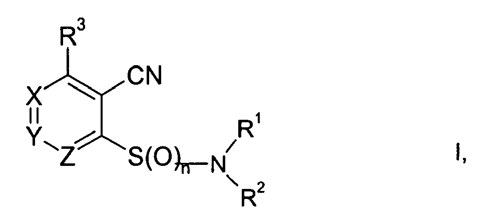 worin der Index n und die Substituenten R¹, R², R³, X, Y und Z die in Anspruch 1 angegebenen Bedeutungen aufweisen, oder ein Salz davon zur Bekämpfung tierischer Schädlinge.

11. Verfahren zur Bekämpfung tierischer Schädlinge, wobei man die tierischen Schädlinge, deren Lebensraum, Brutgebiet, Nahrungsangebot, die Pflanzen, das Saatgut, den Boden, die Flächen, Materialien oder Umgebung, worin die tierischen Schädlinge sich entwickeln oder entwickeln können, oder die vor tierischem Angriff oder Befall zu schützenden Materialien, Pflanzen, Saatgüter, Böden, Flächen oder Räume mit einer pestizid wirksamen Menge wenigstens einer Verbindung der Formel I und/oder wenigstens eines landwirtschaftlich verträglichen Salzes davon gemäß Anspruch 10 in Kontakt bringt, wobei ein Verfahren zur Behandlung von Menschen ausgenommen ist.

12. Verfahren zum Schutz von Ernten vor dem Angriff von oder Befall mit tierischen Schädlingen, wobei man die Ernte mit einer pestizid wirksamen Menge wenigstens einer Verbindung der Formel I und/oder wenigstens eines Salzes davon gemäß Anspruch 10 in Kontakt bringt.

13. Verfahren zum Schutz von Saatgut vor Bodeninsekten und von Wurzeln und Sprossen von Setzlingen gegen Insekten, wobei man das Saatgut vor dem Säen und/oder nach dem Vorkeimen mit einer Verbindung der Formel I gemäß Anspruch 10 und/oder wenigstens eines landwirtschaftlich verträglichen Salzes davon gemäß Anspruch 10 in einer pestizid wirksamen Menge in Kontakt bringt.

14. Saatgut, enthaltend eine Verbindung der Formel I oder ein landwirtschaftlich brauchbares Salz von I gemäß Anspruch 10 in einer Menge von 0,1 g bis 10 kg pro 100 kg Saatgut.

15. Cyanopyridinsulfonylhalogenid der Formel II 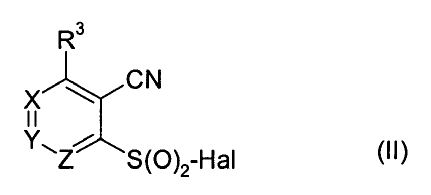 worin X, Y, Z und R³ die in Anspruch 1 genannten Bedeutungen aufweisen und Hal für Halogen steht,
ausgenommen
die Verbindung der Formel II, worin Hal für Chlor steht, X für CR⁴ steht, Y für CR⁵ steht, Z für N steht, R⁴ für Wasserstoff steht und R³ und R⁵ jeweils für CH₃ stehen.

## Revendications

1. Composé de cyanopyridine de formule I 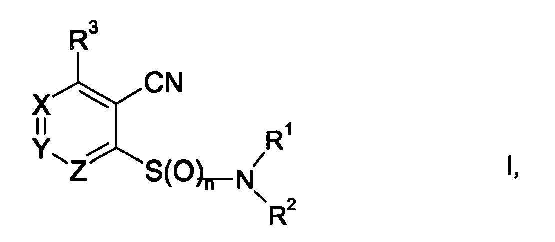 dans laquelle
n vaut 0, 1 ou 2 ;
X est N, N-O ou C-R⁴ ;
Y est N, N-O ou C-R⁵;
Z est N, N-O ou C-R⁶ ;
à condition que l'une des variables X, Y et Z soit N ou NO et que les deux autres variables soient des atomes de carbone éventuellement substitués ;
R¹, R² sont, indépendamment l'un de l'autre, choisis parmi le groupe constitué par hydrogène, C(=O)-R⁷, C₁-C₁₀-alkyle, C₂-C₆-alcényle, C₂-C₁₀-alcynyle, C₁-C₁₀-alcoxy ou C₃-C₁₀-cycloalkyle, où les cinq radicaux mentionnés en dernier peuvent être non substitués, partiellement ou totalement halogénés et/ou peuvent porter 1, 2 ou 3 radicaux, choisis chacun indépendamment l'un de l'autre parmi le groupe constitué par cyano, nitro, amino, C₁-C₁₀-alcoxy, C₁-C₁₀-alkylthio, C₁-C₁₀-alkylsulfinyle, C₁-C₁₀ alkylsulfonyle, C₁-C₁₀-halogénoalcoxy, C₁-C₁₀-halogénoalkylthio, C₁-C₁₀-alcoxycarbonyle, (C₁-C₁₀-alkyl) amino, di- (C₁-C₁₀-alkyl) amino, C₃-C₁₀-cycloalkyle et phényle, ledit phényle pouvant être non substitué, partiellement ou totalement halogéné et/ou porter 1, 2 ou 3 substituants, choisis indépendamment l'un de l'autre parmi le groupe constitué par C₁-C₄-alkyle, C₁-C₄-halogénoalkyle, C₁-C₄-alcoxy et C₁-C₄-halogénoalcoxy ;
ou R¹ et R², conjointement avec l'azote adjacent, forment un cycle de 3 à 10 chaînons, éventuellement substitué par 1, 2 ou 3 radicaux choisis parmi C₁-C₅-alkyle et halogène, où le cycle peut contenir, outre les chaînons de cycle azotés et carbonés, 1, 2 ou 3 hétéroatomes comme chaînons de cycle choisis parmi le groupe constitué par azote, oxygène, soufre, un groupement SO, SO₂ ou N-R⁸ ;
R³ est hydrogène, nitro, cyano, azido, amino, halogène, sulfonylamino, sulfénylamino, sulfinylamino, C(=O)R⁹, C₁-C₆-alkyle, C₂-C₆-alcényle, C₂-C₆-alcynyle, C₃-C₈-cycloalkyle, C₁-C₆-alcoxy, C₁-C₆-alkylthio, (C₁-C₆-alkyl)amino, di(C₁-C₆-alkyl)amino, C₁-C₆-alkylsulfinyle, ou C₁-C₆-alkylsulfonyle, où les dix radicaux mentionnés en dernier peuvent être non substitués, partiellement ou totalement halogénés et/ou peuvent porter 1, 2 ou 3 radicaux, choisis parmi le groupe constitué par cyano, nitro, amino, C₁-C₄-alcoxy, C₁-C₄-alkylthio, C₁-C₄-alkylsulfinyle, C₁-C₄-alkylsulfonyle, C₁-C₄-halogénoalcoxy, C₁-C₄-halogénoalkylthio, (C₁-C₄-alcoxy)carbonyle, (C₁-C₄-alkyl)amino, di (C₁-C₄-alkyl)amino, C₃-C₈-cycloalkyle et phényle, ledit phényle pouvant être non substitué, partiellement ou totalement halogéné et/ou porter 1, 2 ou 3 substituants, choisis indépendamment l'un de l'autre parmi le groupe constitué par C₁-C₄-alkyle, C₁-C₄-halogénoalkyle, C₁-C₄-alcoxy et C₁-C₄-halogénoalcoxy ;
R⁴, R⁵ et R⁶ sont choisis indépendamment l' un de l'autre parmi le groupe constitué par hydrogène, halogène, cyano, azido, nitro, C₁-C₆-alkyle, C₃-C₈-cycloalkyle, C₁-C₄-halogénoalkyle, C₁-C₄-alcoxy, C₁-C₄-alkylthio, C₁-C₄-alkylsulfinyle, C₁-C₄-alkylsulfonyle, C₁-C₄-halogénoalcoxy, C₁-C₄-halogénoalkylthio, C₂-C₆-alcényle, C₂-C₆-alcynyle, (C₁-C₄-alcoxy)carbonyle, amino, (C₁-C₄-alkyl)amino, di(C₁-C₄-alkyl)amino, aminocarbonyle, (C₁-C₄-alkyl)aminocarbonyle, di(C₁-C₄-alkyl)aminocarbonyle, hydroxysulfonyle, sulfonylamino, sulfénylamino, sulfinylamino et C (=O) -R¹⁰ ;
R⁷ est C₁-C₆-alkyle, C₁-halogénoalkyle, aryle, aryl-C₁-C₄-alkyle, hétéroaryle ou hétéroaryl-C₁-C₄-alkyle de 3 à 7 chaînons, où le cycle hétéroaryle contient comme chaînons de cycle 1, 2 ou 3 hétéroatomes, choisis parmi le groupe constitué par azote, oxygène, soufre, un groupement SO, SO₂ ou N-R¹¹, où R¹¹ est hydrogène ou C₁-C₄-alkyle ;
hétérocyclyle ou hétérocyclyl-C₁-C₄-alkyle de 3 à 7 chaînons, où le cycle hétérocyclique contient comme chaînons de cycle 1, 2 ou 3 hétéroatomes, choisis parmi le groupe constitué par azote, oxygène, soufre, un groupement SO, SO₂ ou N-R¹², où R¹² est hydrogène ou C₁-C₄-alkyle ;
et où les atomes de carbone des cycles hétérocycliques peuvent être non substitués ou substitués par 1 ou 2 groupements C₁-C₄-alkyle ;
R⁸ est hydrogène ou C₁-C₄-alkyle ;
R⁹ et R¹⁰, indépendamment l'un de l'autre, sont hydrogène, hydroxy, C₁-C₆-alcoxy, amino, C₁-C₄-alkyle, aryle, aryl-C₁-C₄-alkyle,
hétéroaryle ou hétéroaryl-C₁-C₄-alkyle de 3 à 7 chaînons, où le cycle hétéroaryle contient comme chaînons de cycle 1, 2 ou 3 hétéroatomes, choisis parmi le groupe constitué par azote, oxygène, soufre, un groupement SO, SO₂ ou N-R¹³, où R¹³ est hydrogène ou C₁-C₄-alkyle ;
hétérocyclyle ou hétérocyclyle C₁-C₄-alkyle de 3 à 7 chaînons, où le cycle hétérocyclique contient 1, 2 ou 3 hétéroatomes, choisis parmi le groupe constitué par azote, oxygène, soufre, un groupement SO, SO₂ ou N-R¹⁴, où R¹⁴ est hydrogène ou C₁-C₄-alkyle ;
et où les atomes de carbone des cycles hétérocycliques peuvent être non substitués ou substitués par 1 ou 2 groupements C₁-C₄-alkyle ;
et/ou les sels de celui-ci acceptables sur le plan agricole,
sauf
les composés de formule I, dans laquelle n vaut O ; X est CR⁴ ; Y est CR⁵ ; Z est N ; R¹, R² et R⁴ sont chacun hydrogène ; R⁵ est CH₃ ; et R³ est hydrogène, CH₃ ou c (=O) OC₂H₅ ; et
le composé de formule I, dans laquelle n vaut 2 ; X est CR⁴ ; Y est CR⁵ ; Z est N ; R¹, R² et R⁴ sont chacun hydrogène ; et R³ et R⁵ sont chacun CH₃.

2. Composé de formule I telle que définie selon la revendication 1, dans laquelle n vaut 2.

3. Composé de formule I telle que définie selon la revendication 1 ou 2, dans laquelle X est CR⁴, Y est N ou NO et Z est CR⁶.

4. Composé selon la revendication 3, dans lequel R⁴ ou bien R⁶ est hydrogène et l'autre radical R⁴ ou R⁶ est choisi parmi halogène, C₁-C₄-alkyle, ou C₁-C₄-halogénoalkyle.

5. Composé selon la revendication 3, dans lequel R⁴ ainsi que R⁶ sont hydrogène.

6. Composé de formule I telle que définie selon la revendication 1 ou 2, dans laquelle X est CR⁴, Y est CR⁵ et Z est N ou NO.

7. Composé de formule I telle que définie selon la revendication 1, dans laquelle R³ est halogène, C₁-C₄-alkyle, C₁-C₄-halogénoalkyle, C₁-C₄-alcoxy ou C₁-C₄-halogénoalcoxy.

8. Composé de formule I telle que définie selon la revendication 1, dans laquelle R¹ et R² sont, indépendamment l'un de l'autre, choisis parmi le groupe constitué par hydrogène, C₁-C₆-alkyle, C₁-C₆-halogénoalkyle, C₂-C₆-alcényle, C₂-C₆-halogénoalcényle, C₂-C₆-alcynyle, C₂-C₆-halogénoalcynyle, C₃-C₆-cycloalkyle, C₁-C₄-alcoxy-C₁-C₄-alkyle et C₁-C₄-alkylthio-C₁-C₄-alkyle, où R² peut également former un radical C(=O)-R⁷, où R⁷ est tel que défini selon la revendication 1.

9. Composition agricole comprenant une telle quantité d'au moins un composé de formule I et/ou d'au moins un sel de I utile sur le plan agricole, tel que défini selon la revendication 1, et au moins un véhicule liquide et/ou solide inerte et acceptable sur le plan agronomique qu'elle a une action pesticide et, si on le souhaite, au moins un agent tensioactif.

10. Utilisation d'un composé de cyanopyridine de formule I 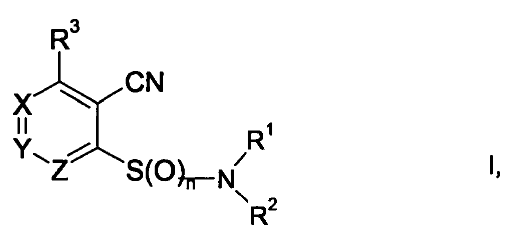 dans laquelle l' indice n et les substituants R¹, R², R³, X, Y et Z sont tels que définis selon la revendication 1 ou un sel de celui-ci, pour lutter contre des animaux nuisibles.

11. Méthode de lutte contre des animaux nuisibles, comprenant la mise en contact des animaux nuisibles, de leur habitat, de leur lieu de reproduction, de leurs sources d'alimentation, des plantes, semences, sol, zone, matériau ou environnement dans lesquels les animaux nuisibles se développent ou sont susceptibles de se développer, ou les matériaux, plantes, semences, sols, surfaces ou espaces à protéger d'une attaque ou d'une infestation par des animaux, avec une quantité efficace sur le plan pesticide d'au moins un composé de formule I et/ou d'au moins un sel de celui-ci acceptable sur le plan agricole, tel que défini selon la revendication 10, où on exclut une méthode de traitement de l'être humain.

12. Méthode de protection de cultures vis-à-vis d'une attaque ou d'une infestation par des animaux nuisibles, comprenant la mise en contact d'une culture avec une quantité efficace sur le plan pesticide d'au moins un composé de formule I et/ou d'au moins un sel de celui-ci, tel que défini selon la revendication 10.

13. Méthode de protection de semences vis-à-vis d'insectes du sol et des racines et pousses des jeunes plants vis-à-vis d'insectes, comprenant la mise en contact des semences avant semis et/ou après prégermination avec un composé de formule I, tel que défini selon la revendication 10, et/ou d'au moins un sel de celui-ci acceptable sur le plan agricole, tel que défini selon la revendication 10, en des quantités efficaces sur le plan pesticide.

14. Semence comprenant un composé de formule I ou un sel de I utile sur le plan agricole, tel que défini selon la revendication 10, 1 en une quantité allant de 0,1 g à 10 kg par 100 kg de semences.

15. Halogénure de cyanopyridinesulfonyle de formule II 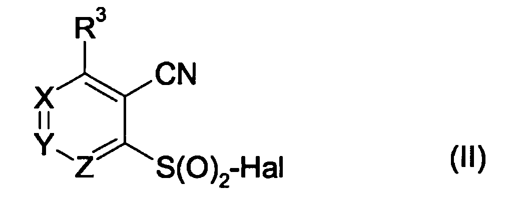 dans laquelle X, Y, Z et R³ sont tels que définis selon la revendication 1 et Hal est halogène,
sauf
le composé de formule II dans laquelle Hal est chlore, X est CR⁴, Y est CR⁵, Z est N, R⁴ est hydrogène et R³ et R⁵ sont chacun CH₃.
